# EUROPEAN PATENT SPECIFICATION

(11) **EP 1 292 621 B1**
(45) Date of publication and mention of the grant of the patent: **20.09.2006**
(21) Application number: 01939953.4
(22) Date of filing: 04.06.2001
(51) Int. Cl.: C07K 19/00, A61K 39/00, A61P 37/06, C07K 14/47, C07K 16/00

(54) **COMPOSITIONS FOR TREATING AUTOIMMUNE DISEASE**
ZUSAMMENSETZUNGEN ZUR BEHANDLUNG VON AUTOIMMUNKRANKHEITEN
COMPOSITIONS POUR LE TRAITEMENT DES MALADIES AUTO-IMMUNES

(43) Date of publication of application: 19.03.2003
(73) Proprietor: University of Tennessee Corporation, Knoxville, TN 37996-0344 (US)
(72) Inventor: ZAGHOUANI, Habib, Columbia, MO 65203 (US)
(74) Representative: Stuart, Ian Alexander
(86) International application number: PCT/US2001/040834
(87) International publication number: WO 2002/026833

(56) References cited:
- WO-A-96/19584
- WO-A-98/30706
- C. LIU ET AL.: "FcgammaRI-targeted fusion proteins result in efficient presentation by human monocytes and antagonist T cell epitopes." JOURNAL OF CLINICAL INVESTIGATION, vol. 98, no. 9, 1 November 1996 (1996-11-01), pages 2001-2007, XP002131533 New York, NY, USA
- E. GOSSELIN ET AL.: "Enhanced antigen presentation using human Fcgamma receptor (monocyte/macrophage)-specific immunogens." THE JOURNAL OF IMMUNOLOGY, vol. 149, no. 11, 1 December 1992 (1992-12-01), pages 3477-3481, XP002184982 Baltimore, MD, USA
- B. MIN ET AL.: "Neonatal exposure to a self-peptide-immunoglobulin chimera circumvents the use of adjuvant and confers resistance to autoimmune disease by a novel mechanism involving interleukin 4 lymph node deviation and interferon gamma-mediated splenic anergy." THE JOURNAL OF EXPERIMENTAL MEDICINE, vol. 188, no. 11, 7 December 1998 (1998-12-07), pages 2007-2017, XP002131535 New York, NY, USA
- C. CULLEN ET AL.: "A divalent major histocompatibility complex/IgG1 fusion protein induces antigen-specific T cell activation in vitro and in vivo." CELLULAR IMMUNOLOGY, vol. 192, no. 1, 25 February 1999 (1999-02-25), pages 54-62, XP001041848 New York, NY, USA
- K. LEGGE ET AL.: "Presentation of a T-cell receptor antagonist peptide by immunoglobulins ablates activation of T cells by a synthetic peptide or protein requiring endocytic processing." THE JOURNAL OF EXPERIMENTAL MEDICINE, vol. 185, no. 6, 17 March 1997 (1997-03-17), pages 1043-1053, XP002131534 New York, NY, USA

## Description

### Field of the Invention

The present invention generally relates to compositions for the effective endocytic presentation of immunosuppressive factors. More particularly, the present invention is directed to compositions comprising immunosuppressive factors that are useful for the treatment of various disorders including, but not limited to, autoimmune disorders. In preferred embodiments the immunosuppressive factors may be T cell receptor antagonists or agonists. Other embodiments of the invention provide for the induction of tolerance in neonates or infants. According to the present invention there is provided a composition comprising, an aggregated immunoglobulin or portion thereof linked to an antigen, wherein the immunoglobulin or portion thereof is capable of crosslinking Fc receptors present on the cell surfaces of antigen presenting cells. Embodiments relate to methods of increasing the levels of IL-10 in an individual in need thereof. Still further embodiments are useful in methods of stimulating peripheral tolerance and/or bystander suppression in an individual in need thereof. Other embodiments relate to compositions for reducing the level of IFNγ in an individual in need thereof. In further embodiments, the present invention provides compositions which facilitate the presentation of an autoantigen by antigen presenting cells which lack or have a reduced level of costimulatory molecules.

### Background of the Invention

Vertebrates possess the ability to mount an immune response as a defense against pathogens from the environment as well as against aberrant cells, such as tumor cells, which develop internally. The immune response is the result of complex interactions between a variety of cells and factors, but generally comprises two main facets. One is a cellular component, in which specialized cells directly attack an offending agent (bearing an antigen) while the other is a humoral component, in which antibody molecules bind specifically to the antigen and aid in its elimination. Acting in concert, the individual elements are quite effective in limiting the initial onslaught of invading pathogens and eliminating them from the host.

The primary cells involved in providing an immune response are lymphocytes which generally comprise two principal classes. The first of these, designated B cells or B lymphocytes, are typically generated in bone marrow and are, among other duties, responsible for producing and secreting antibodies. B cell antibody products tend to react directly with foreign antigens and neutralize them or activate other components of the immune systems which then eliminate them. In particular, opsonizing antibodies bind to extracellular foreign agents thereby rendering them susceptible to phagocytosis and subsequent intracellular killing. On the other hand, T cells or T lymphocytes, which generally develop or mature in the thymus, are responsible for mediating the cellular immune response. These cells do not recognize whole antigens but, instead, respond to short peptide fragments thereof bound to specialized proteins which appear on the surface of the surface of a target cell. More particularly, it appears that proteins produced within the cell, or taken up by the cell from the extracellular milieu, are continually degraded to peptides by normal metabolic pathways. The resulting short fragments associate with intracellular major histocompatibility complex (MHC) molecules and the MHC-peptide complexes are transported to the surface of the cell for recognition by T cells. Thus, the cellular immune system is constantly monitoring a full spectrum of proteins produced or ingested by the cells and is posed to eliminate any cells presenting foreign antigens or tumor antigens; i.e. virus infected cells or cancer cells.

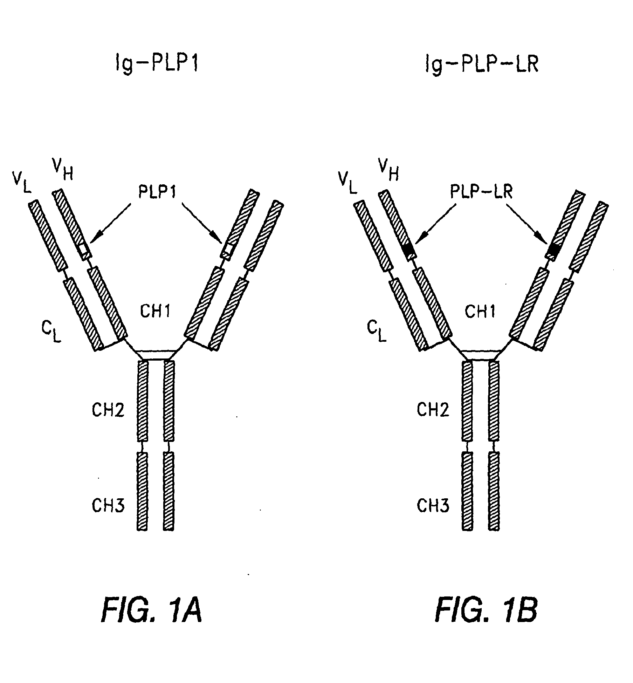

The general structure of immunoglobulin G (lgG), the most common of mammalian antibodies, is shown schematically in Figure 1. As illustrated, IgG is a tetrameric protein complex comprising two identical heavy (H) chains and two identical immunoglobulin light (L) chains. These chains are joined together by disulfide bonds to form the Y-shaped antibody complex. In solution however, the molecule takes on a more globular shape and readily bind to foreign antigens present in biological fluids.

Amino acid sequence analysis of immunoglobulins has led to the definition of specific regions with various functional activities within the chains. Each light chain and each heavy chain has a variable region (V_{L} and V_{H} respectively) defined within the first 110 amino terminal residues. Three dimensional pairing of the V_{L} and V_{H} regions constitute the antigen-recognition portion or "antigen combining site" ("ACS") of immunoglobulin molecule. Because of the tetrameric nature of immunoglobulins, there are two identical antigen combining sites per molecule. The variable domains of these chains are highly heterogeneous in sequence and provide the diversity for antigen combining sites to be highly specific for a large variety of antigenic structures. The heterogeneity of the variable domains is not evenly distributed throughout the variable regions, but is located in three segments, called complementarity determining regions ("CDRs") designated CDR 1, CDR 2 and CDR 3. For further information regarding these structures see Watson et al., *Molecular Biology of the Gene,* Fourth Edition, Benjamin/Cummings Publishing Co., Inc., Menlo Park, CA, 1987.

Each of the heavy chains also includes a constant region defining a particular isotype and assigns the immunoglobulin to one of the immunoglobulin classes and subclasses. The constant region contains units called domains (i.e. C_{H1}, C_{H2}, etc.) which do not vary significantly among antibodies of a single class. The constant region does not participate in antigen binding, but can be associated with a number of biological activities known as "effector functions", such as binding to Fc receptors on cell surfaces of antigen presenting cells (APC's) as well as binding to complement proteins. Antigen presenting cells such as dendritic cells and macrophages are, among other features, generally distinguished by the presence of a Fc receptor. Consequently, if an antibody is bound to a pathogen, it can then link to a phagocyte via the Fc portion. This allows the pathogen to be ingested and destroyed by the phagocyte, a process known as opsonization. Moreover, as will be discussed in more detail below, various pathogenic antigens may be processed and displayed by the APC to further stimulate an immune response.

Unlike the heavy chains, the light chains have a single constant domain (C_{L}). A light chain pairs with a heavy chain through a disulfide bond which attaches heavy constant region C_{H1} to C_{L}. In addition, the heavy chains have a hinge region separating constant regions C_{H1} and C_{H2} from the remainder of the molecule. It is this hinge region that is largely responsible for the flexibility of the tetramer. The two heavy chains of the molecule pair together through disulfide bonds at the junction between the hinge region and C_{H2}.

In order to provide such an extensive repertoire, immunoglobulin genes have evolved so as permit the production of vast numbers of different immunoglobulin proteins from a finite number of genes i.e. inherent polymorphism. Due to inherent polymorphism, mammals are, able to produce antibodies to a seemingly infinite variety of antigens. For a review of immunoglobulin genetics and protein structure see Lewin, *Genes III,* John Wiley and Sons, New York, 1987; and Benjamini et al., *Immunology,* Alan R. Liss, Inc., New York, 1988.

In the past few years antibodies have become extremely important in diagnostic and therapeutic applications due to their diversity and specificity. Increasingly, molecular biology techniques have been used to expand the variety and availability of antibodies for scientific applications. For instance, a single antibody producing B cell can be immortalized by fusion with a tumor cell and expanded to provide an *in vitro* source of antibodies of a single specificity known as a "monoclonal antibody" (mAb). Such an immortal B cell line is termed a "hybridoma."

Until recently, the source of most mAb has been murine (mouse) hybridomas cultured *in vitro.* That is, a mouse was typically injected with a selected antigen or immunogen. Subsequently, the animal was sacrificed and cells removed from its spleen were fused with immortal myeloma cells. Although they have been used extensively in diagnostic procedures, murine mAb have not proven to be well suited for therapeutic applications in most mammals including humans. In part, this is due to the fact that murine antibodies are recognized as foreign by other mammalian species and elicit an immune response which may itself cause illness or undesirable side effects.

To overcome at least some of the problems of immune responses generated by foreign mAb and the lack of suitable human mAb, genetic engineering has been used to construct humanized chimeric immunoglobulin molecules which contain the antigen binding complementarity determining regions of the murine antibodies but in which the remainder of the molecule is composed of human antibody sequences which are not recognized as foreign. Such antibodies have been used to treat tumors as the mouse variable region recognizes the tumor antigen and the humanized portion of the molecule is able to mediate an immune response without being rapidly eliminated by the body. See, for example, Jones et al., *Nature,* 321:522-525, 1986.

Other uses of such antibodies are detailed in co-pending U.S. Application No. 08/363,276 and PCT Publication No. WO 94/14847. In these cases epitopes of foreign antigens such as viral or bacterial epitopes are grafted onto the hypervariable region of an immunoglobulin to induce a response. That is, the engineered antibodies are used as a vaccine to provoke an immune response and confer long term immunogenic memory thereby allowing the subject to fight off subsequent infections.

These and more traditional vaccines are effective in that they stimulate both prongs of the immune system. Despite the intricacies associated with the humoral component of the immune response, it would not, in and of itself, be capable of effectively protecting an animal from the myriad pathogenic assaults to which it is subject each day. Rather, it is only the presence of a highly evolved cellular response that allows higher organisms to survive and proliferate.

As indicated above, T lymphocytes or T cells, which arise from precursors in the bone marrow, are central players in the immune response against invading viruses and other microbes. The progenitor stem cells migrate to the thymus where, as so-called thymocytes, they become specialized. In particular, they begin to display the receptor molecules that later enable mature T cells to detect infection. To be beneficial, T cells must be able to attach through their receptors to microbial antigens (protein markers signaling an invader's presence). At the same time, they should be blind to substances made by the body as self-reactive T cells can destroy normal tissues. Typically, only those thymocytes that make useful receptors will mature fully and enter the bloodstream to patrol the body. Others that would be ineffectual or would attack the body's own tissue are, in healthy individuals, eliminated through apoptosis prior to leaving the thymus.

Mature T cells that finally enter the circulation, either as cytolytic T lymphocytes or T helper cells, remain at rest unless they encounter antigens that their receptors can recognize. Upon encountering the specific antigens for which the lymphocytes have affinity, they proliferate and perform effector functions, the result of which is elimination of the foreign antigens.

T cells have been classified into several subpopulations based on the different tasks they perform. These subpopulations include helper T cells (Tₕ), which are required for promoting or enhancing T and B cell responses; cytotoxic (or cytolytic) T lymphocytes (CTL), which directly kill their target cells by cell lysis; and suppressor T cells (Tₛ) which down-regulate the immune response. In each case the T cells recognize antigens, but only when presented on the surface of a cell by a specialized protein complex attached to the surface of antigen presenting cells. More particularly, T cells use a specific receptor, termed the T cell antigen receptor (TCR), which is a transmembrane protein complex capable of recognizing an antigen in association with the group of proteins collectively termed the major histocompatibility complex (MHC). Thousands of identical TCR's are expressed on each cell. The TCR is related, both in function and structure, to the surface antibody (non-secreted) which B cells use as their antigen receptors. Further, different subpopulations of T cells also express a variety of cell surface proteins, some of which are termed "marker proteins" because they are characteristic of particular subpopulations. For example, most Tₕ cells express the cell surface CD4 protein, whereas most CTL and Tₛ cells express the cell surface CD8 protein. These surface proteins are important in the initiation and maintenance of immune responses which depend on the recognition of, and interactions between, particular proteins or protein complexes on the surface of APCs.

For some time it has been known that the major histocompatibility complex or MHC actually comprises a series of glycosylated proteins comprising distinct quaternary structures. Generally, the structures are of two types: class I MHC which displays peptides from proteins made inside the cell (such as proteins produced subsequent to viral replication), and class II MHC, which generally displays peptides from proteins that have entered the cell from the outside (soluble antigens such as bacterial toxins). Recognition of various antigens is assured by inherited polymorphism which continuously provides a diverse pool of MHC molecules capable of binding any microbial peptides that may arise. Essentially, all nucleated cells produce and express class I MHC which may exhibit naturally occurring peptides, tumor associated peptides or peptides produced by a viral invader. Conversely, only a few specialized lymphoid cells, those generally known as antigen presenting cells, produce and express class II MHC proteins. Regardless of the cell type, both classes of MHC carry peptides to the cell surface and present them to resting T lymphocytes. Ordinarily Tₕ cells recognize class II MHC-antigen complexes while CTL's tend to recognize class I MHC-antigen complexes.

When a resting T cell bearing the appropriate TCR encounters the APC displaying the peptide on its surface, the TCR binds to the peptide-MHC complex. More particularly, hundreds of TCR's bind to numerous peptide-MHC complexes. When enough TCRs are contacted, the cumulative effect activates the T cell. Receptors on T cells that are responsible for the specific recognition of, and response to, the MHC-antigen complex are composed of a complex of several integral plasma membrane proteins. As with the MHC complex previously discussed, a diverse pool of TCR's is assured by inherent polymorphism leading to somatic rearrangement. It should be emphasized that, while the pool of TCR's may be diverse, each individual T cell only expresses a single specific TCR. However, each T cell typically exhibits thousands of copies of this receptor, specific for only one peptide, on the surface of each cell. In addition, several other types of membrane associated proteins are involved with T cell binding and activation.

Activation of the T cell entails the generation of a series of chemical signals (primarily cytokines) that result in the cell taking direct action or stimulating other cells of the immune system to act. In the case of class I MHC-antigen activation, CTL's proliferate and act to destroy infected cells presenting the same antigen. Kitting an infected cell deprives a virus of life support and makes it accessible to antibodies, which finally eliminate it. In contrast, activation of Tₕ cells by class II MHC-antigen complexes does not destroy the antigen presenting cell (which is part of the host's defense system) but rather stimulates the Tₕ cell to proliferate and generate signals (again primarily cytokines) that affect various cells. Among other consequences, the signaling leads to B cell stimulation, macrophage activation, CTL differentiation and promotion of inflammation. This concerted response is relatively specific and is directed to foreign elements bearing the peptide presented by the class II MHC system.

When operating properly the immune response is surprisingly effective at eliminating microscopic pathogens and, to a lesser extent, neoplastic cells. In general, the complicated mechanisms for self-recognition are very efficient and allow a strong response to be directed exclusively at foreign antigens. Unfortunately, the immune system occasionally malfunctions and turns against the cells of the host thereby provoking an autoimmune response. Typically, autoimmunity is held to occur when the antigen receptors on immune cells recognize specific antigens on healthy cells and cause the cells bearing those particular substances to die. In many cases, autoimmune reactions are self-limited in that they disappear when the antigens that set them off are cleared away. However, in some instances the autoreactive lymphocytes survive longer than they should and continue to induce apoptosis or otherwise eliminate normal cells. Some evidence in animals and humans indicates that extended survival of autoreactive cells is implicated in at least two chronic autoimmune disorders, systemic lupus erythematosus and rheumatoid arthritis.

Other mechanisms of action are also thought to contribute to the development of various autoimmune disorders. For example, over the last few years it has become clear that the avidity of T cell-APC interactions dictates thymic learning and tolerance to self antigens. Accordingly, high avidity interactions lead to elimination of the T cell whereas low avidity interactions allow for maturation and exit from the thymus. Although this mechanism is effective in purging the immune system of autoreactivity, T cell precursors endowed with self reactivity could still be generated and migrate to the periphery if the autoantigen is sequestered and does not achieve effective levels of thymic presentation, is subjected to thymic crypticity, or is poorly presented. Moreover, superantigens capable of reacting with particular T cell receptors and events that could stimulate antigen mimicry, epitope spreading or peripheral loosening in peptide crypticity may trigger activation of those self-reactive T cells and cause antigen exposure. In any case, continuous supply of autoantigen and abundant generation of T cell receptor ligands (peptide-MHC complexes) are a likely mechanism of T cell aggressivity. Examples of such a spontaneous break in self-tolerance include multiple sclerosis (MS), rheumatoid arthritis (possibly more than one mechanism) and type I diabetes all of which are thought to be T cell mediated autoimmune diseases.

Regardless of which mechanism is responsible for the corruption of the immune system, the results can be devastating to the individual. For example, multiple sclerosis is a chronic, inflammatory disorder that affects approximately 250,000 individuals in the United States. The inflammatory process occurs primarily within the white matter of the central nervous system and is mediated by T cells, B cells and macrophages which are responsible for the demyelination of the axons. Although the clinical course can be quite variable, the most common form is manifested by relapsing neurological deficits including paralysis, sensory deficits and visual problems.

Once immune cells have spread to the white matter of the central nervous system, the immune response is targeted to several different antigens on myelin. For example, there is a critical antibody response directed to myelin that activates the complement cascade with membrane attack complexes appearing in the spinal fluid. Further, T cells are targeted to certain key portions of various myelin antigens such as those presented on myelin basic protein (MBP) and proteolipid protein (PLP). The T cells in turn produce cytokines which then influence macrophages to attack the myelin and phagocytose large chunks of the myelin sheath. The concerted attack leads to areas of demyelination impairing salutary conduction along the axon and producing and the pathophysiologic defect. Multiple immune responses to several components of a supramolecular structure, like the myelin sheath in multiple sclerosis or the pyruvate dehydrogenase complex in primary biliary cirrhosis, are common in individuals with autoimmune diseases involving discrete organs.

Treatments for autoimmune diseases have met with varying levels of success. For example, it is often possible to correct organ-specific autoimmune disease through metabolic control. Where function is lost and cannot be restored, mechanical substitutes or tissue grafts may be appropriate. However, no effective treatments exist for several of the most disabling disorders including MS. While a number of compounds, including corticosterioids and modified beta interferon, can reduce some symptoms of MS, they have proven to have serious side effects or otherwise been shown to be less than desirable for long term use. Other avenues of treatment have shown promise but have yet to be shown effective.

In this respect, one promising treatment for MS is described in WO 96/16086, which discloses the use of peptide analogs of myelin basic protein (MBP). Compositions comprising these analogs are reportedly able to ameliorate symptoms of MS without excessive side effects. Moreover, use of peptide analogs to myelin constitutive proteins were also shown to be effective in treating the symptoms of experimental allergic encephalomyelitis (EAE), an organ specific immune disorder often used in mice as a model for MS. Specifically, reversal of EAE was achieved with a peptide analog derived from proteolipid (PLP) peptide (Kuchroo et al., *J. Immunol.* 153:3326-3336, 1994. It was shown that when the major TCR contacting residues within the naturally occurring PLP peptide were mutated, the resulting peptide analog, like the natural peptide, bound MHC yet does not activate PLP specific T cells. Instead, the PLP analog inhibits *in vitro* activation of the T cells.

While peptide analogs represent an attractive approach to modulate the effector functions of aggressive T calls and ameliorate autoimmune diseases, several problems limit their effectiveness. For instance, only a few MHC-peptide complexes are available on the surface of a typical APC meaning a single complex may be required to serially trigger about 200 TCRs to activate the T cell. Where the autoantigen is continuously available for normal processing and presentation by the MHC system, it appears that very few surface MHC complexes would be available to bind the peptide analog. Further, as free peptides typically have very short half-rives, they are not readily incorporated and processed by the MHC-antigen presenting system, little will be naturally expressed on the APC. Due to the inefficient presentation, direct engagement of the thousands of TCR's on each T cell likely require prohibitively high intracellular levels of free peptide. The turnover of cell surface MHC molecules also contributes to the short stay of complexes formed at the extracellular milieu (i.e. MHC class II molecules have been in the cell surface for some time before binding the extracellular peptide) while complexes formed in the endocytic compartment will reside for a normal period of time because they have just been translocated to the cell surface. Finally, as previously alluded to, administration of such synthetic epitopes or analogs is extremely problematic in view of the short half-life of peptides in the mammalian body. Between the short half-lives of the MHC complexes and the administered peptides, effective exposure is too brief to permit the induction of a satisfactory immune response further necessitating higher doses.

Accordingly, it is a general object of the present invention to provide compositions for effectively modifying the immune system of a vertebrate to treat an immune disorder.

Compositions embodying the present invention may be useful :
(i) for the effective presentation of T cell receptor antagonists or agonists to modulate the cellular immune response in a subject in need thereof;
(ii) for the treatment and amelioration of various immune disorders;
(iii) for the induction of T cell tolerance in neonates or infants; or
(iv) for the relief of pathological symptoms associated with autoimmune disorders including multiple sclerosis.

### Summary of the Invention

In a broad aspect, the invention provides for an Fc receptor mediated, endocytic delivery system. In selected embodiments the invention provides for the effective presentation of immunosuppressive factors which, in preferred embodiments, may comprise T cell receptor antagonists or agonists. Other preferred embodiments incorporate immunosuppressive factors comprising one or more autoantigenic polypeptides or fragments thereof. That is, the present invention generally provides compositions to present immunosuppressive factors for the selective modification of an immune response in a vertebrate. In particularly preferred embodiments, the invention provides for Fc receptor mediated endocytic presentation of one or more selected T cell receptor antagonists or agonists to modulate an immune response mounted against a specific antigen. As will be appreciated by those skilled in the art, the disclosed methods and compositions may be used to treat any physiological disorder related to the immune response of a vertebrate. For example, this ability to suppress selected components of the immune system may allow, among other things, for the treatment of autoimmune diseases, facilitation of tissue or organ transplants and the mitigation of symptoms produced by allergens. Moreover, the present invention further provides for the induction of tolerance in neonates and infants with regard to autoantigens.

According to the invention, the endocytic presentation of the selected immunosuppressive factor is facilitated through the use of an immunomodulating agent that is able to bind to the Fc receptor (FcR) of antigen presenting cells. Typically, the immunomodulating agent will comprise at least one immunosuppressive factor associated with at least one ligand capable of binding to a Fc receptor. Upon binding to the antigen presenting cell (APC) the immunomodulating agent will be internalized and processed by the APC's natural endocytic pathway. Preferably, the internalized immunosuppressive factor, which can be part or all of an autoantigenic polypeptide or a T cell receptor antagonist or agonist, will then be associated with the newly synthesized endogenous MHC class II structures and presented at the surface of the APC. Those skilled in the art will appreciate that the immunosuppressive factors (especially antagonists), while complexing with T cell receptors when bound to MHC class II structures, will not promote activation of the T cell. Similarly, it will be appreciated that presentation of autoantigenic polypeptide derived or directly administered TCR agonists by APCs in the absence of costimulatory molecules will lead to the induction of tolerance. Accordingly, efficient FcR mediated presentation of appropriate TCR antagonists or agonists (wherein the agonists may be derived from autoantigenic polypeptides or fragments thereof) can prevent a previously primed T cell (i.e. one sensitized to a particular autoantigen) from activating and triggering an immune response despite normal presentation of the natura9y occurring autoantigen.

In a broad sense, the immunomodulating agents of the present invention may comprise any ligand (FcR ligand) that is capable of binding to, and being internalized by, the Fc receptor of an antigen presenting cell That is, the FcR ligand may be any protein, protein fragment, peptide or molecule that effectively binds to a Fc receptor on the surface of any antigen presenting cell. Preferably, the FcR ligand will comprise or mimic at least some portion of a constant region of an immunoglobulin molecule and will not provoke an antigenic response in the subject. In selected aspects of the invention, the FcR ligand will comprise part or all of a constant region from an IgG molecule. Particularly preferred embodiments will employ FcR ligands comprising the entire constant region of a selected immunoglobulin molecule from the species to be treated. Of course, it will also be appreciated that binding to the Fc receptor may also be effected by ligands that comprise small fragments of a single constant region domains or non amino acid based molecular entities. In any case, the FcR ligand may be derived using modern pharmaceutical techniques such as directed evolution, combinatorial chemistry or rational drug design.

As previously alluded to, the compounds of the present invention further comprise an immunosuppressive factor associated with the FcR ligand to provide an immunomodulating agent For the purposes of the instant invention the immunosuppressive factor can be any molecular entity that is capable of being processed by an APC and presented in association with class II MHC molecules on the cell surface. As indicated above, selected embodiments of the invention comprise associating at least one T cell receptor antagonist or agonist with an FcR ligand for efficient presentation via Fc mediated uptake. In particularly preferred embodiments the immunosuppressive factor may comprise one or more selected autoantigenic polypeptides, or fragments thereof, which can be processed (i.e. digested or proteolyzed) to provide the desired TCR agonists. Preferably the autoantigenic polypeptide(s), or fragments thereof, will provide more than one peptide agonist (i.e. peptides comprising more than one amino acid sequence) upon proteolysis during endocytic processing. Presentation of the antagonists or agonists by APCs in the absence of appropriate costimulatory molecules will then result in the down-regulation of the immune response to the relevant autoantigen.

With regard to particularly preferred embodiments the present invention employs immunosuppressive factors comprising all or part of a T cell antagonist. For the purposes of this disclosure the term "antagonist" shall, in accordance with its normal meaning, comprise any substance that interferes with the physiological action of another by combining with, and blocking, its receptor. More particularly, TCR antagonists are molecular entities that, in combination with class II MHC molecules, are capable of non-reactively associating with a T cell receptor and inducing negative signaling via the T cell receptor. Preferably, the TCR antagonist comprises a peptide or protein fragment that is an analog of the normal activating antigen agonist. In particularly preferred embodiments the TCR antagonist is an analog of a T cell epitope.

In other preferred embodiments the immunosuppressive factor may comprise a T cell agonist that is presented but which does not activate primed TCRs upon binding. With respect to this aspect of the invention it has been suprisingly found that when autoantigen agonists are efficiently presented using an FcR ligand they may lead to the induction of tolerance rather than stimulating the immune system. That is, it is believed that efficient FcR uptake of autoantigenic agonists leads to presentation of the agonists by nonprofessional and/or non-activated professional APCs generally lacking costimulatory molecules. Contrary to conventional prior art thinking, this type of presentation ultimately induces inactivation of autoreactive T cells, down-regulation of the immune system and amelioration of any associated autoimmune disease. In order to avoid the activation of APCs or the production of costimulatory molecules (i.e. B-7.1, B-7.2, CD40, etc.), the administration of TCR agonist constructs or agonist producing autoantigenic polypeptide constructs will preferably take place using a carrier lacking an adjuvant (such as saline).

For the purposes of the present disclosure, the term "agonist" shall be used in accordance with its commonly accepted biochemical meaning. In this regard it will be appreciated that, while the T cell agonist may be any molecule that provides the desired immunogenic result, the selected agonist will preferably comprise a peptide or protein fragment. Moreover, those skilled in the art will appreciate that immunomodulating agents comprising one or more T cell receptor agonists may be combined with immunomodulating agents comprising one or more T cell receptor antagonists to provide pharmaceutical formulations that may be used to selectively attenuate a patient's immune response.

With regard to this aspect of the invention the ultimately presented TCR agonists may be derived from an immunosuppressive factor that includes all or part of one or more autoantigenic polypeptides. That is, the constructs of the present invention may preferably comprise an FcR ligand associated with one or more autoantigenic polypeptides, or fragments thereof. Typically the incorporated autoantigenic polypeptide(s) or fragments will comprise the wild type amino acid sequence and, when processed (i.e. proteolyzed) following FcR mediated uptake, will provide one or more TCR agonists for presentation by professional or non professional APCs. The administration of such constructs in accordance with the present invention is particularly advantageous as it may be used to overcome difficulties with epitope spreading. More particularly, when autoimmunity involves multiple epitopes on an autoantigen, the presentation of each of the corresponding agonists (derived from an immunosuppressive factor comprising the entire autoantigen) will lead to tolerance with respect to all the epitopes of interest. Similarly, the presentation of multiple agonists is likely to prove efficient when administered to populations where different individuals develop autoreactivity to different epitopes of a particular autoantigen.

By way of example, a construct of the present invention could take the form of a fusion or chimeric protein comprising the entire Fc region of an IgG molecule covalently linked to natural myelin basic protein (MBP) or natural proteolipid protein (PLP). In other embodiments fusion proteins compatible with the present invention may comprise the Fc region of IgG covalently linked to an immunosuppressive factor comprising the covalentty attached natural forms of PLP and MBP (i.e. IgGFc-MBP-PLP). Such constructs will be internalized via the FcR and endocytically processed (proteolyzed with the resulting agonist fragments associated with MHC complexes) and presented on the surface of the APCs. It must be emphasized that, due to the relatively high levels of presented agonists based on the efficient uptake of the constructs and the lack of costimulatory molecules, the administration of the FcR ligand *I* autoantigen constructs induces anergy rather than stimulating an immune response. Of course, selected fragments or portions of the naturally occurring autoantigenic polypeptides could be used to form compatible immunosuppressive factors. Those skilled in the art will appreciate that such fusion or chimeric proteins may easily be constructed using modem molecular biology techniques.

In the disclosed compounds and associated methods, the FcR ligand is associated with the immunosuppressive factor to form an immunomodulating agent so that both are internalized by the APC at substantially the same time. As alluded to above this association may be in the form of two or more molecules bound to each other as with an antibody-antigen complex or, in preferred embodiments, may comprise the formation of a single fusion or chimeric molecule incorporating both the immunosuppressive factor (i.e. one or more autoantigenic polypeptides or fragments thereof or a TCR antagonist or agonist) and FcR ligand. For example, a selected TCR antagonist could be chemically linked to an FcR ligand region produced by proteolytic techniques (i.e. an Fc fragment). Other embodiments may comprise a normal immunoglobulin comprising an FcR ligand sterically bound to an antagonistic or agonistic peptide. Particularly preferred embodiments of the invention comprise chimeric immunoglobulins produced through genetic engineering techniques. In these compounds the FcR ligand (and usually the majority of the molecule) comprises one or more immunoglobulin constant regions while one or more of the variable regions is engineered to express one or more desired peptide TCR antagonists or TCR agonists. Those skilled in the art will appreciate that any combination of the aforementioned immunomodulating agents may be associated to form compositions of the present invention as can similar immunomodulating agents comprising different immunosuppressive factors. Moreover, as previously discussed, mixtures or "cocktails" of various immunomodulating agents are specifically contemplated as falling within the scope of the present invention.

In the present invention, the immunomodulating agent is in a form which is capable of crosslinking the Fc receptors present on the cell surfaces of antigen presenting cells. For example, the immunomodulating agent may be in a polyvalent form. The immunomodulating agent or agents is/are aggregated to provide constructs or structures that are capable of crosslinking the Fc receptors present on the cells surfaces of antigen presenting cells.

Crosslinking of FcγR on macrophages has been shown to have anti-inflammatory activity. (Berger et al., *Eur. J. Immunol.,* 26:1297-1301, 1996; Berger et al., *Eur. J. Immunol.,* 27:2994-3000, 1997; Sutterwala et al., *J. Exp. Med.,* 188:217-222, 1998. Similarly, aggregation confers to Igs Fc.associated functions such as crosslinking of FcRs and complement binding (Christian, *J*. *Immunol.,* 84:112-121, 1960; Rosenqvist et al., *Mol. Immunol.,* 24:495.501, 1987.

T cell or hybridoma cell lines in which IL-10 or IL-4 are produced from plasmid or viral vectors have been shown to induce recovery from disease when injected into animals with ongoing EAE (Mathisen et al., *J*. *Exp*. *Med.,* 186:159-164, 1997; Shaw et al., *J*. *Exp. Med.,* 185:1711-1714, 1997; Ma et al., *J. Immunol.* 161:1516-1524, 1998.

IL-10 produced by macrophages upon exposure to antigen-antibody complexes has been shown to exert antagonistic effects on IL-12 production and to reverse pro inflammatory responses (Sutterwala et al., *J Exp. Med.,* 188:217.222, 1998; Berger et al., *Eur. J. Immunol.* 27:2994-3000, 1997.

In addition, IL-10 may provide bystander suppression, thereby inhibiting the activity of T cells directed against a variety of antigens responsible for autoimmune disease (Falcone et al., *J. Exp. Med.,* 185:901-907, 1997; Stohlman et al., *J. Immunol.,* 163:6338-6344, 1999. Bystander suppression has been proposed to result from antagonism of pathogenic T cells by IL-10 produced by APCs or to result from down regulation by regulatory T cells generated through the action of IL-10 (Groux et al., *Nature (Lond.),* 389:737-742, 1997. It has been suggested that IL-10 enables naïve T cells to develop into regulatory cells that could produce IL-10, IL-5 or TGFβ and inhibit the function of pathogenic T cells thereby sustaining suppression (Groux et al., *Nature (Lond.),* 389:737-742, 1997; Chen et al., *Proc. Natl. Acad. Sci. USA,* 93:388-391, 1996; Asseman et al., *J. Exp. Med*. 190:995.1003, 1999; Groux et al., *Immunol. Today,* 20:442-445, 1999; Seddon et al., *J. Exp. Med.,* 189:877-881, 1999; Seddon et al., *Immunol. Today,* 21:95-99, 2000.

Thus, m some embodiments of the present invention, the immunomodulating agent may induce the production of anti-inflammatory cytokines such as IL-10 and IL-6 and/or reduce the level of IFNγ in an individual as described in more detail below. Treatment with aggregated immunomodulating agents may also lead to upregulation of other cytokines such as IL-4, IL-9, IL-13, TGF-B.

Those skilled in the art will appreciate that the desired aggregated immunomodulating agents may be fabricated using any one of a number of well known techniques. For example, the immunomodulating agents may be chemically or thermodynamically linked or altered to form soluble or insoluble aggregates. In further embodiments, aggregated immunomodulating agents may be prepared by precipitation, such as by ammonium sulfate precipitation. These aggregates may then be combined with a pharmaceutically acceptable carrier and administered in accordance with the teachings herein.

In addition, the immunomodulating agent may reduce the level of costimulatory molecules present on the cell surfaces of antigen presenting cells, thereby leading to peripheral tolerance (Fowlkes et al., *Curr. Opin. Immunol.,* 5:873-879, 1993; Arnold et al., *Immunol. Today,* 14:12-14, 1993;. Kosaka et al., *J. Exp. Med.,* 177:367-378, 1993; McCormack et al., *J. Immunol.,* 150:3785-3792, 1993; Rocha et al., *Science (Washington, D.C.),* 251:1225-1228, 1991; Webb et al., *Cell.,* 63:1249.1256, 1990; Jenkins et al., *Curr Opin. Immunol,* 5:361-367, 1993 Peripheral tolerance results from the availability of an antigen involved in autoimmune disease in the periphery (i.e. outside of the thymus, where initial selection against T cells targetting self-antigens occurs) and the presentation of the self-antigen in the periphery by non-activated antigen presenting cells in which costimulatory molecules are absent or present at a reduced level.

More particularly, the disclosed compositions may be formulated using conventional pharmaceutical techniques and carriers and may be administered through the usual routes. Particularly preferred embodiments comprise the use of formulations or carriers that do not comprise adjuvants. Supply of antigen in an adjuvant free form might not stimulate the expression of costimulatory molecules on APCs, thereby resulting in antigen presentation which is inadequate for T cell activation (Fowlkes et al., *Curr. Opin. Immunol.,* 5:873-879, 1993; Jacobs et aL, *Immunology,* 82:294-300, 1994; Mueller et al., *Curr. Opin. Immunol.,* 7:375-381, 1995. This approach modulates autoreactive T cells, and promotes recovery from illness (Elliott et al., *J. Clin. Invest.,* 98:1602-1612, 1996; Gaur et al., *Science (Washington, D.C.*), 258:1491-1494, 1992; Liblau et al., *Immunol. Today,* 18:599-604, 1997; Critchfield et al., *Science (Washington, D.C.),* 263:1139-1143, 1994; Chen et al., *Proc. Natl. Acad Sci. USA,* 93:388-391, 1996; Devaux et al., *J. Neuroimmunol.,* 75:169-173, 1997; Leadbetter et al., *J. Immunol.,* 161:504-512, 1998; Staykova et al., *Immunol. Cell Biol.,* 75:54-64, 1997. Thus, those skilled in the art will appreciate that such preparations avoid or minimize the generation of costimulatory molecules that may provoke an immune response.

In any event the use of FcR mediated uptake of the immunomodulating agent avoids many of the problems associated with prior art compositions. More specifically, the methods of the present invention overcome many of the limitations associated with the administration of free peptide antagonists as disclosed in the prior art. Accordingly, efficient endocytic presentation of an immunosuppressive factor such as a TCR antagonist can generate significant levels of MHC-antagonist ligands to oppose naturally occurring MHC-autoantigenic complexes that are generated in spontaneous immune disorders involving the continuous presentation of an autoreactive antigen. Similarly, the efficient uptake of FcR ligand-agonist (or autoantigenic polypeptide) constructs and subsequent presentation of the desired agonist(s) may induce anergy in autoreactive T cells. As such, the invention may be used to treat any immune disorder that responds to the presentation of immunosuppressive factors. This is particularly true of T cell mediated autoimmune disorders including, for example, multiple sclerosis, lupus, rheumatoid arthritis, scleroderma, insulin-dependent diabetes and ulcerative colitis. In a like manner, the present invention can be used to selectively down-regulate the immune system with respect to continuously presented agonists such as allergens. Further, the compounds and associated compositions of the present invention may be used to selectively suppress various components of the immune system to reduce the likelihood of tissue or organ rejection following transplant.

In addition to the aforementioned advantages, it has been surprisingly found that the compounds, compositions, and methods of the present invention may be used to induce tolerance to various autoantigens in neonates and infants. More particularly, the present invention further provides compositions and methods for conferring resistance in neonate or infant mammals to the induction of an autoimmune disease during adult life. In accordance with the teachings herein, this neonatal tolerance is characterized by a deviated response in the secondary lymphoid organs and unusual gamma interferon-mediated splenic anergy upon challenge with the appropriate autoantigen. As discussed above, preferred embodiments the present invention may provide for the induction of the desired neonatal tolerance upon administration in a non-reactive carrier (i.e. those without adjuvants).

Some aspects of the present invention are summarized below.

One embodiment of the present invention is a composition comprising an aggregated immunoglobulin or portion thereof, linked to an antigen, wherein said immunoglobulin or portion thereof is capable of crosslinking Fc receptors present on the cell surfaces of antigen presenting cells. The composition may further comprise a pharmaceutically acceptable carrier. In some aspects of this embodiment, the composition does not include an adjuvant. In some aspects of this embodiment, the immunoglobulin is in a polyvalent form. In some aspects of this embodiment, the immunoglobulin is embeded or absorbed on a matrix. In further aspects of this embodiment, the immunoglobulin is an IgG molecule. In other aspects of this embodiment, the antigen comprises an antigen associated with an an autoimmune disease. For example, the antigen may be associated with an autoimmune disease selected from the group consisting of multiple sclerosis, lupus, rheumatoid arthritis, scleroderma, insulin-dependent diabetes and ulcerative colitis. In some aspects of this embodiment, the antigen is an antigen from proteolipid protein. In other aspects of this embodiment, the antigen is an antigen from myelin basic protein. In further aspects of this embodiment, the immunoglobulin or portion thereof comprises at least part of a domain of a constant region of an immunoglobulin molecule. In some aspects of this embodiment, the immunoglobulin comprises a fusion protein in which said antigen is covalently joined to said immunoglobulin or portion thereof. For example, the antigen may be positioned within at least one complementarity determining region of said immunoglobulin to partially or fully replace said complementarity determining region. In some aspects of this embodiment, the antigen is positioned within CDR3. In other aspects of this embodiment, the immunoglobulin is a human IgG molecule. In other aspects of this embodiment, the immunoglobulin is chimeric.

Another embodiment of the present invention for use in a method of alleviating symptoms associated with an autoimmune disease comprises a composition comprising an aggregated immunoglobulin or portion thereof linked to an antigen involved in said autoimmune disease, wherein said immunoglobulin or portion thereof is capable of crosslinking Fc receptors present on the cell surfaces of antigen presenting cells and administering said composition to an individual suffering from said autoimmune disease. In some aspects of this embodiment, the composition further comprises a pharmaceutically acceptable carrier. In some aspects of this embodiment, the composition does not include an adjuvant. The antigen is associated with an autoimmune disease. In some aspects of this embodiment, the antigen is associated with an autoimmune disease selected from the group consisting of multiple sclerosis, lupus, rheumatoid arthritis, scleroderma, insulin-dependent diabetes and ulcerative colitis. In some aspects of this embodiment, the antigen is an antigen from proteolipid protein. In some aspects of this embodiment, the antigen is an antigen from myelin basic protein. In some aspects of this embodiment, the immunoglobulin or portion thereof comprises at least part of a domain of a constant region of an immunoglobulin molecule. In some aspects of this embodiment, the immunoglobulin comprises a fusion protein in which said antigen is covalently joined to said immunoglobulin or portion thereof. In some aspects of this embodiment, the antigen is positioned within at least one complementarity determining region of said immunoglobulin to partially or fully replace said complementarity determining region. In some aspects of this embodiment, the antigen is positioned within CDR3. In some aspects of this embodiment, the immunoglobulin is a human IgG molecule. In some aspects of this embodiment, the immunoglobulin is chimeric.

Another embodiment of the present invention is for use in a method of reducing disease symptoms in an individual comprising identifying an individual in need of an increased level of IL-10 and increasing the level of IL-10 in said individual by administering a composition of the invention. In some aspects of this embodiment, the individual is suffering from an autoimmune disease. In some aspects of this embodiment, the composition further comprises a pharmaceutically acceptable carrier. In some aspects of this embodiment, the composition does not include an adjuvant. In some aspects of this embodiment, the antigen is associated with an autoimmune disease selected from the group consisting of multiple sclerosis, lupus, rheumatoid arthritis, scleroderma, insulin-dependent diabetes and ulcerative colitis. In some aspects of this embodiment, the antigen is an antigen from proteolipid protein. In some aspects of this embodiment, the antigen is from myelin basic protein. In some aspects of this embodiment, the immunoglobulin or portion thereof comprises at least part of a domain of a constant region of an immunoglobulin molecule. In some aspects of this embodiment, the immunoglobulin comprises a fusion protein in which said antigen is covalently joined to said immunoglobulin or portion thereof. In some aspects of this embodiment, the antigen is positioned within at least one complementarity determining region of said immunoglobulin to partially or fully replace said complementarity determining region. In some aspects of this embodiment, the antigen is positioned within CDR3. In some aspects of this embodiment, the immunoglobulin is a human IgG molecule. In some aspects of this embodiment, the immunoglobulin is chimeric.

Another embodiment of the present invention is for use in a method of reducing disease symptoms in an individual comprising identifying an individual in need of an increased level of IL-10 and in need of stimulation of peripheral tolerance and increasing the level of IL-10 and stimulating peripheral tolerance in said individual by administering a composition of the invention. In some aspects of this embodiment, the individual is suffering from an autoimmune disease. In some aspects of this embodiment, the composition further comprises a pharmaceutically acceptable carrier. In some aspects of this embodiment, the composition does not include an adjuvant. In some aspects of this embodiment, the antigen is associated with an autoimmune disease selected from the group consisting of multiple sclerosis, lupus, rheumatoid arthritis, scleroderma, insulin-dependent diabetes and ulcerative colitis. In some aspects of this embodiment, the antigen is from proteolipid protein. In some aspects of this embodiment, the antigen is from myelin basic protein. In some aspects of this embodiment, the immunoglobulin or portion thereof comprises at least part of a domain of a constant region of an immunoglobulin molecule. In some aspects of this embodiment, the immunoglobulin comprises a fusion protein in which said antigen is covalently joined to said immunoglobulin or portion thereof. In some aspects of this embodiment, the antigen is positioned within at least one complementarity determining region of said immunoglobulin to partially or fully replace said complementarity determining region. In some aspects of this embodiment, the antigen is positioned within CDR3. In some aspects of this embodiment, the immunoglobulin is a human IgG molecule. In some aspects of this embodiment, the immunoglobulin is chimeric.

Another embodiment of the present invention is for use in a method of reducing disease symptoms in an individual comprising identifying an individual in need of a reduced level of IFNγ and decreasing the level of IFNγ in said individual by administering a composition of the invention.

Another embodiment of the present invention is for use in a method of reducing the symptoms of an autoimmune disease resulting from an immune response to a plurality of self antigens comprising administering a composition of the invention.

Other objects, features and advantages of the present invention will be apparent to those skilled in the art from a consideration of the following detailed description of preferred exemplary embodiments thereof taken in conjunction with the figures which will first be described briefly.

### Brief Description of the Drawings

Figs. 1A and 1B are schematic representations of chimeric immunoglobulin G (IgG) molecules illustrating the general features thereof and the inclusion of foreign peptides within the CDR 3 loop of the heavy chain variable region wherein Fig. 1A (Ig-PLP1) shows the insertion of a naturally occurring peptide PLP1 (agonist) derived from proteolipid protein while Fig. 1B (Ig-PLP-LR) illustrates an immunomodulating agent comprising the inclusion of a peptide analog (antagonist) to PLP1 termed PLP-LR.
Figs. 2A and 2B are graphical representations illustrating the capture by radioimmunoassay (RIA) of chimeric antibodies Ig-PLP1 and Ig-PLP-LR, which correspond to those shown in Figs. 1A and 1B respectively, using antibodies directed to the corresponding free peptides wherein Fig. 2A shows capture levels by antibodies directed to PLP1 and Fig. 2B shows capture levels by antibodies directed to PLP-LR with lg-W, a wild type antibody, acting as a negative control.
Figs. 3A and 3B are graphs illustrating the presentation of Ig-PLP1 and lg-PLP-LR (as well as positive and negative controls) to PLP1-specific T cell hybridomas 4E3 (Fig. 3A) and 5B6 (Fig. 38) to determine the relative T cell activation potentials of the chimeric immunoglobulins as measured by IL-2 production.
Fig. 4 is a graphical representation illustrating the relative effectiveness of presenting PLP1 using the chimeric antibodies of the present invention (Ig-PLPl) versus the free peptide PLP1 or the native proteolipid protein (PLP) as measured by levels of IL-2 production following incubation with splenic SJL antigen presenting cells and PLP1 specific 4E3 specific 4E3 T cell hybridoma.
Figs. 5A, 5B and 5C are graphical comparisons showing Ig-PLP-LR antagonism of PLP1 (5A), Ig-PLP1 (5B) and PLP (5C) mediated T cell activation as measured by IL-2 production by T cell hybridoma 4E3 in the presence of SJL splenic APCs that were previously incubated with the respective agonist and various levels of Ig-PLP-LR or controls.
Fig. 6 is a graph showing the relative antagonism of Ig-PLP2, lg-PLP-LR and Ig-W as measured by the production of IL-2 by T cell hybridoma HT-2 in the presence of SJL splenic APCs that were previously incubated with native proteolipid protein in combination one of the aforementioned immunoglobulins.
Figs. 7A and 7B are graphs demonstrating the *in vivo* presentation of PLP1 following inoculation with Ig-PLP1 as measured by ³H-thymidine incorporation by cells from the lymph node (7A) or the spleen (7B) wherein the illustrated values represent the ability of cells harvested from individual mice to generate a T cell response as measured by ³H-thymidine incorporation when exposed to agonist PLP1 or the control peptide PLP2.
Figs. 8A and 8B are graphical representations showing the ability of Ig-PLP-LR to reduce the immune response to PLP1 peptide when co-administered with lg-PLP1 as measured in murine cells from the lymph node (8A) or the spleen (8B) wherein the illustrated values represent the ability of cells harvested from individual mice to generate a T cell response as measured by ³H-thymidine incorporation when exposed to PLP1.
Figs. 9A and 9B are graphs demonstrating that mice inoculated with a mixture of lg-PLP-LR and Ig-PLP1 develop a more vigorous immune response to the peptide analog PLP-LR than peptide PLP1 as measured in cells from the lymph node (9A) or the spleen (9B) wherein the illustrated values represent the ability of cells harvested from individual subjects to generate a T cell response as reflected by ³H-thymidine incorporation when exposed to either PLP1 peptide or the peptide analog PLP-LR.
Figs. 10A-10D are graphical representations of lymph node proliferative responses to immunization with Ig-PLP chimeras with mice individually tested in triplicate wells for each stimulator and where the indicated cpms represent the mean ± SD after deduction of background cpms.
Fig.11 is a graphical representation of lymph node T cell proliferative response to co-immunization with lg-PLP1 and lg-PLPLR with stimulators comprising PPD, 5 µg/ml; PLP 1, PLP-LR, and PLP2 at 15 µg/ml.
Fig. 12 is a graphical representation of splenic proliferative T cells responses of mice immunized with Ig-W, Ig-PLP1, IG-PLP-LR and combinations thereof when stimulated with PLP1 (filled bars) and PLP-LR (hatched bars) in triplicate wells.
Figs. 13A-13C are graphical representations of IL-2 (13A), INFγ (13B), and IL-4 (13C) production by splenic cells of mice immunized with Ig-W, Ig-PLP1, Ig-PLP-LR and combinations thereof.
Figs. 14A-14D graphically illustrate proliferation of antigen experienced T cells from mice immunized with Ig-PLP1 (a and b) or Ig-PLP-LR (c and d) in CFA upon stimulation in vitro with PLP1 peptides, PLP-LR peptides and mixtures thereof.
Figs. 15A and 15B are graphical representations of IL-2 production by antigen experienced T cells immunized with Ig-PLP1 (15A) and Ig-PLP-LR (15B) upon in vitro stimulation with PLP1 peptide, PLP-LR peptide or mixtures thereof.
Figs. 16A and 16B graphically illustrate that neonatal mice injected with Ig-PLP1 but not Ig-W resist induction of EAE with clinically derived curves shown for all mice (16A) and for surviving mice (16B).
Figs. 17A and 17B graphically show *in vivo* presentation of Ig-PLP1 by neonatal thymic (17A) and splenic (178) antigen presenting cells following injection with lg-PLP1 or Ig-W within 24 hours of birth.
Figs. 18A and 18B graphically illustrate lymph (18A) and splenic (18B) proliferative T cell response in mice injected with lg-PLP1 or Ig-W shortly after birth upon stimulation with free PLP1, PLP2 or a negative control peptide corresponding the encephalitogenic sequence 178-191 of PLP.
Figs. 19A-19C graphically represent lymph node T cell deviation as measured by production of IL-2 (19A), IL-4 (19B), and IFNγ (19C) in mice treated with lg-PLP1 shortly after birth and stimulated with free PLP1 or PLP2.
Figs. 20A-20C graphically represent splenic T cell deviation as measured by production of IL-2 (20A), IL-4 (20B), and IFNγ (20C) in mice treated with Ig-PLP1 shortly after birth and stimulated with free PLP1 or PLP2.
Fig. 21 graphically illustrates cytokine mediated restoration of splenic T cell proliferation in mice injected with Ig-PLP1 shortly after birth, immunized with free PLP1 at seven weeks and stimulated with free PLP1 with the cells grown in control media (NIL) media with IL-12 and media with INFγ with the indicated cpms for each mouse representing the mean ± SD of triplicate wells.
Figure 22 illustrates the results of administering soluble Ig-PLP1, soluble Ig-W or free PLP1 peptide to mice which were induced to develop EAE. Administration of soluble Ig-PLP1 reduced paralytic severity and suppressed relapses in mice with EAE.
Figure 23 illustrates the results of administering soluble Ig-PLP1, soluble ig-PLP-LR, or soluble Ig-W to mice which were induced to develop EAE. Both Ig-PLP1 and Ig-PLP-LR treated mice had reduced clinical severity during the initial peak of disease. While the mice given Ig-W showed relapses throughout the whole 120 days of observation, those treated with Ig-PLP1 and Ig-PLP-LR recovered from paralysis by day 31 and 38 respectively and showed no relapses.
Figure 24 illustrates the levels of costimulatory molecules after adminstering soluble Ig-PLP1 without adjuvant to mice with EAE. Mice which received Ig-PLP1 exhibited lower levels of B7.1 and CD40 than control mice which received media alone.
Fig. 25 illustrates that the administration of aggregated Ig-PLP1 effectively ameliorates EAE in mice as shown by clinical grading of the condition over an extended period.
Figs. 26A and 26B shows that incubation of aggregated Ig-PLP1 with purified APCs advantageously induces production of anti-inflammatory cytokines IL-6 (26A) and IL-10 (26B).
Figure 27a compares the abilities of aggregated Ig-PLP1 and aggregated Ig-W to alleviate EAE. Mice receiving aggregated Ig-PLP1 exhibited reduced disease severity and never relapsed while agg Ig-W treated mice never recovered and showed relapses throughout the entire period of clinical assessment.
Figure 27b is a direct comparison of the disease course of PLP1 peptide induced EAE followed by treatment with sol Ig-PLP1 vs. agg Ig-PLP1. The mean maximum clinical score of mice receiving agg Ig-PLP1 was much lower and the recovery time was faster than mice receiving sol Ig-PLP1.
Figure 28a is a comparison of the abilities of agg Ig-PLP1, sol Ig-PLP1, agg Ig-W, agg Ig-PLP2 and sol Ig-PLP2 to induce IL-10 production by splenic cells. Agg Ig-PLP1, Ig-PLP2, and Ig-W chimeras stimulated the production of IL-10 by splenic cells in a dose-dependent manner while the soluble forms of the chimeras did not induce detectable levels of IL-10.
Figure 28b illustrates the effects of agg Ig-PLP1 and IgM on IL-10 production by B cells, dendritic cells and macrophages. Macrophages and dendritic cells, but not B cells, produce IL-10 upon incubation with agg Ig-PLP1. Mouse IgM was unable to stimulate lL-10 production by any of the APCs tested.
Figure 29 illustrates IL-10 production after contacting splenocytes with 0.1 M sol lg-PLP1, 0.1 M agg lg-PLP1, 0.1 M agg Ig-PLP1 + 50 g/ml 2.4G2, or 0.1 M agg lg-PLP1 + 100 glml mouse Ig. Agg Ig-PLP1 induces IL-10 by crosslinking the FcγR1 receptors.
Figure 30a illustrates the proliferative response of TCC-PLP1-1B10 to PLP1, PLP2, agg lg-PLP1 and agg Ig-PLP2. TCC-PLP1-1B10 proliferates upon incubation with paraformaldehyde-fixed splenic APCs that were previously pulsed with free PLP1 peptide or agg lg-PLP1 but does not show significant proliferation when the APCs were pulsed with the negative control PLP2 or agg Ig-PLP2.
Figure 30b is a measurement of IL-2 production by TCC-PLP1-1B10 upon incubation with non-fixed splenic APCs and free PLP1 peptide or agg Ig-PLP1. TCC-PLP1-1B10 produced significant amounts of IL-2 when incubated with both free PLP1 peptide and agg Ig-PLP1.
Figure 30c is a measurement of IFNγ production by TCC-PLP1-1B10 upon incubation with non-fixed splenic APCs and free PLP1 peptide or agg Ig-PLP1. TCC-PLP1-1B10 produced significant amounts of IFNγ when incubated with both free PLP1 peptide and agg Ig-PLP1.
Figure 30d is a measurement of IL-4 production by TCC-PLP1-1B10 upon incubation with non-fixed splenic APCs and free PLP1 peptide or agg Ig-PLP1. TCC-PLP1-1B10 produced significant amounts of IL-4 when incubated with both free PLP1 peptide and agg Ig-PLP1.
Figure 30e is a measurement of IL-10 production by TCC-PLP1-1B10 upon incubation with non-fixed splenic APCs and free PLP1 peptide or agg Ig-PLP1. IL-10 was detectable at significant levels when the stimulator was agg lg-PLP1 but not free PLP1.
Figure 31 is a measurement of IL-10 production after incubation of fixed or live APCs with agg Ig-PLP1 and subsequent incubation with TCC-PLP1-1B10. IL-10 was undetectable with fixed APCs but was produced by live APCs.
Figure 32a measures the ability of IL-10 produced by splenocytes to antagonize the production of IFNγ by the T cells. IL-10 produced by splenocytes is able to antagonize the production of IFNγ by the T cells.
Figure 32b measures the ability of IL-10 produced by dendritic cells to antagonize the production of IFNγ by the T cells. IL-10 produced by dendritic cells is able to antagonize the production of IFNγ by the T cells.
Figure 32c measures the ability of IL-10 produced by macrophages to antagonize the production of IFNγ by the T cells. IL-10 produced by macrophages is able to antagonize the production of IFNγ by the T cells.
Figure 32d shows levels of IL-10 and IFNγ when B cells are incubated with agg Ig-PLP1 and TCC-PLP1-1B10. B cells do not produce IL-10 upon incubation with agg Ig-PLP1 and do not inhibit secretion of IFNγ by T cells.
Figure 33a shows levels of IFNγ and IL-10 produced when TCC-PLP1 1B10 was incubated with peritoneal macrophages and agg lg-PLP1. IFNγ production decreased in proportion with the level of IL-10 secreted by the presenting macrophages.
Figure 33b shows levels of IFNγ and IL-10 produced when TCC-PLP1 1B10 was incubated with peritoneal macrophages, agg Ig-PLP1, and anti-IL-10 mAb, 2A5. The inhibition of IFNγ production by the T cells was directly related to APC derived IL-10 as neutralization of such IL-10 by anti-IL-10 mAb, 2A5 restored IFN production.
Figure 33c shows levels of IFNγ and IL-10 produced when TCC-PLP1 1B10 was incubated with peritoneal macrophages, agg Ig-PLP1, and rat IgG. Incubation with isotype control rat IgG instead of anti-IL-10 had no effect on IL-10's ability to inhibit IFN production by TCC-PLP1 1B10.
Figure 34a shows the effects of treatment with agg Ig-PLP1, agg lg-PLP1 and anti-IL-10, agg Ig-PLP1 and rat IgG, agg lg-W or agg Ig-W and anti-IL-10 on mice with EAE. Antibody against IL-10 prevented agg Ig-PLP1 from alleviating disease symptoms.
Figure 34b shows the effects of treatment with sol Ig-PLP1, agg Ig-PLP1, sol lg-PLP1 and IL-10, or agg Ig-W on mice with EAE. Soluble Ig-PLP1, which does not induce detectable levels of IL-10, ameliorates the disease slightly while sol Ig-PLP1, together with exogenous IL-10, further reduces the disease to a level comparable to the level observed in mice treated with agg Ig-PLP1.
Figure 35 shows the effects of treatment with agg Ig-W, agg Ig-PLP1 or agg lg-W and PLP1 on mice with EAE. Only treatment with agg Ig-PLP1 reduced disease symptoms, demonstrating that for endogenous IL-10 to modulate the disease, a physical bridging of the APCs to the T cells is required.
Figure 36 shows the effects of administering agg Ig-PLP-LR or agg Ig-PLP1 to mice suffering from EAE. While mice receiving either of these immunomodulating agents recover from disease, recovery is faster with agg Ig-PLP1.
Figure 37 is a histopathological analysis of mice treated as in Figure 36. Mice treated with agg Ig-PLP1 had a significantly reduced number of inflammatory foci both in the cerebrum and lumbar spinal cord.
Figure 38 shows the effects of agg Ig-PLP1 on the levels of costimulatory molecules on peritoneal macrophages. Agg Ig-PLP1 down regulates B7.1, 87.2, or CD40 expression.
Figure 39a shows the effects of treatment with agg Ig-PLP1 on mice in which EAE was induced by both PLP1 and PLP2. Treatment with agg Ig-PLP1 reduced disease severity.
Figure 39b shows the effects of treatment with agg Ig-PLP1 on mice in which EAE was induced by PLP2. Treatment with agg Ig-PLP1 reduced disease severity.
Figure 40a shows T cell proliferation assay in response to PLP1, PLP2, MBP3 or HA in mice treated with agg Ig-PLP1 or Ig-W after induction of EAE with CNS homogentate.
Figure 40b shows IL-2 levels in the mice of Figure 40a.
Figure 40c shows IFN-γ levels in the mice of Figure 40a.
Figure 40d shows IL-4 levels in the mice of Figure 40a.
Figure 40e shows IL-10 levels in the mice of Figure 40a.
Figure 40f shows IL-5 levels in the mice of Figure 40a.
Figure 40g shows TGF-β levels in the mice of Figure 40a.
Figure 41 shows the effects of treatment with agg Ig-PLP1 on mice in which EAE was induced with CNS homogenate. Treatment with agg Ig-PLP1 reduced disease severity.

### Detailed Description of the Preferred Embodiment

While the present invention may be embodied in many different forms, disdosed herein are specific illustrative embodiments thereof that exemplify the principles of the invention. It should be emphasized that the present invention is not limited to the specific embodiments illustrated.

In preferred embodiments the immunomodulating compounds disclosed herein will comprise at least one FcR ligand and at least one immunosuppressive factor that is capable of down-regulating an immune response upon endocytic presentation. Particularly preferred embodiments of the invention comprise an immunomodulating agent wherein the immunosuppressive factor is one or more T cell receptor antagonists or agonists that, following endocytic processing and presentation, is capable of binding with a receptor on the surface of a primed T cell but not capable of generating an immunogenic response. In such embodiments, the presented immunosuppressive factor will prevent the activation of the relevant primed T cells and reduce the response generated. This selective suppression of the immune system may, among other indications, be used to treat symptoms associated with immune disorders, inducing T cell mediated autoimmune disorders, allergies and tissue rejection in transplant operations.

Accordingly, in one embodiment the present invention comprises an immunomodulating agent for the endocytic presentation of an immunosuppressive factor on the surface of an antigen presenting cell of a vertebrate comprising at least one Fc receptor ligand and at least one immunosuppressive factor. Preferred embodiments comprise a Fc receptor ligand corresponding to at least a part of an immunoglobulin constant region domain while the immunosuppressive factor corresponds to at least one T cell receptor antagonist. Other preferred embodiments incorporate an immunosuppressive factor comprising a T cell receptor agonist. Further, as discussed extensively above, the immunosuppressive factor may comprise one or more autoantigenic polypeptides, or fragments thereof, that provide one or more TCR agonists upon endocytic processing and presentation. In particularly preferred embodiments the immunomodulating agent comprises a recombinant polypeptide or a chimeric antibody.

By exploiting FcR mediated uptake of the selected immunomodulating agent the present invention very cleverly uses the body's own metabolic pathways to down-regulate harmful immune responses. More specifically, the present invention uses the fact that T cells recognize and respond to foreign antigens when attached to the surface of other cells. Selection of the appropriate immunomodulating agent or agents in accordance with the teachings herein provides for the efficient uptake of the administered compound. Following FcR mediated uptake, the natural endocytic pathway of antigen presenting cells provides for the effective presentation of the selected immunosuppressive factor complexed with the MHC class II molecules.

As described above, the two requisite properties that allow a cell to function as an antigen presenting cell for class II MHC-restricted helper T cell lymphocytes are the ability to process endocytosed antigens and the expression of class II MHC gene products. Most cells, including professional and nonprofessional APCs appear to be able to endocytose and process protein antigens. Accordingly, with regard to professional APCs the determining factor appears to be the expression of class II MHC molecules. In this respect, the best defined antigen presenting cells for helper T lymphocytes comprise mononuclear phagocytes, B-lymphocytes, dendritic cells, Langerhans cells of the skin and, in some mammals, endothelial cells. Of course it will be appreciated that different cells may be concentrated in different areas and may be involved in different stages of the T cell mediated immune response.

In any case, the term "professional antigen presenting cell" or "professional APC" as used herein shall be held to mean any cell capable of inducing a T cell mediated immune response and expressing a high level of costimulatory molecules. Conversely, nonprofessional APCs typically do not express a high level of costimulatory molecules. For the purposes of the present invention it will be appreciated that both types of cells may be used to present the selected immunosuppressive factors and down-regulate the immune system. In this regard the selected FcR ligand may interact with any of a number of different Fc receptors found on a variety of cell types to promote endocytosis of the immunomodulating agent. By way of example only, selected human Fc receptors that may be employed include the FcγRI, FcγRIIA, FcγRIIB, FcγRIIIA or FcγRIIIB subfamilies.

The FcR ligands of the present invention will preferably comprise at least a part of a domain of a constant region of an immunoglobulin. In particularly preferred embodiments the FcR ligand will comprise one or more domains derived from a constant region of an immunoglobulin molecule. Those skilled in the art will appreciate that various immunoglobulin isotypes and allotypes may be employed as desired. For example, compatible FcR ligands may be selected from amino acid sequences corresponding to those found in the constant regions of IgG, IgE, IgA or IgM. Among other factors, selection of a particular isotype for use as a FcR ligand may be predicated on biochemical properties such as binding coefficients or low immunoreactivity in the species to be treated. Similarly, the selection of a single domain, fragment thereof or multiple domains may be determined based on biochemical factors or, ultimately, presentation efficiency.

As discussed previously, the immunomodulating agents of the present invention further comprise an immunosuppressive factor. In accordance with the scope of the present invention the immunosuppressive factor may be any compound that, when endocytically processed and presented on the surface of an APC, will down-regulate the immune system. As such, immunosuppressive factors may comprise small molecules, peptides, protein fragments, protein derivatives, polypeptides or combinations thereof. In preferred embodiments the immunosuppressive factor acts as an antagonist when presented on the surface of the APC in that it interferes with the binding of a similarly presented agonist to a selected receptor. In particularly preferred embodiments the immunosuppressive factor comprises a T cell receptor antagonist that will associate with a T cell receptor without activating an immune response. It will be appreciated that other embodiments of the invention comprise immunomodulating agents incorporating T cell receptor agonists that reduce the immune response to the subject autoantigen. With respect to these embodiments, it will be appreciated that Fc mediated presentation of naturally occurring autoantigenic polypeptides may be used to provide the desired T cell receptor agonists via endocytic processing. That is, the administration of naturally occurring autoantigenic polypeptides, or fragments thereof, associated with a FcR ligand results in the efficient presentation of one or more T cell receptor agonists in accordance with the teachings herein.

While any functionally compatible molecule may be used as an immunosuppressive factor in accordance with the present invention, those skilled in the art will appreciate that proteins (polypeptides), protein fragments or peptides are particularly suitable for use in the disclosed compounds and methods. Such molecules are readily processed by the normal endocytic pathways and are easily presented, for example in concert with the MHC class II molecules, on the surface of the antigen presenting cell. Moreover, as the majority of compounds evoking an unwanted immune response are typically protein fragments, T cell receptors are usually most responsive to similar fragments whether they are agonists or antagonists. In particularly preferred embodiments, antagonistic immunosuppressive factors will be analogs of a selected peptide or protein fragment that is immunoreactive with a chosen T cell receptor.

"Peptide analogs" or "analogs," as used herein, contain at least one different amino acid in the respective corresponding sequences between the analog and the native protein fragment or peptide. Unless otherwise indicated a named amino acid refers to the L-form. An L-amino acid from the native peptide may be altered to any other one of the 20 L-amino acids commonly found in proteins, any one of the corresponding D-amino acids, rare amino acids, such as 4-hydroxyprofine, and hydroxylysine, or a non-protein amino acid, such as B-alanine and homoserine. Also included with the scope of the present invention are amino acids which have been altered by chemical means such as methylation (e.g., a-methylvaline), amidation of the C-terminal amino acid by an alkylamine such as ethylamine, ethanolamine, and ethylene diamine, and acylation or methylation of an amino acid side chain function (e.g., acylation of the epsilon amino group of lysine).

Methods for selecting efficient peptide antagonists for treating multiple sclerosis (MS) are provided in PCT Publication No.: WO 96/16086. The disclosed methods may be used in concert with the present invention to provide effective immunosuppressive factors for incorporation in the disclosed immunomodulating agents. For example, using assays detailed below candidate peptide analogs may be screened for their ability to treat MS by an assay measuring competitive binding to MHC, T cell proliferation assays or an assay assessing induction of experimental encephalomye6tis (EAE). Those analogs that inhibit binding of the native autoreactive peptides, do not stimulate proliferation of native peptide reactive cell lines and inhibit the development of EAE (an experimental model for MS) by known autoantigens are useful for therapeutics. Those skilled in the art will appreciate that similar types of assays may be used to screen immunosuppressive factors for other native peptides (i.e. continuously presented autoantigens) and other immune disorders. In particularly preferred embodiments the selected immunosuppressive factors comprise analogs of T cell epitopes.

More generally, immunosuppressive factors(whether agonists or antagonists) may be derived for a number of diseases having a variety of immunoreactive agents without undue experimentation. For example, peptide analog antagonists or agonists may be generated for T cell epitopes on both proteolipid protein or myelin basic protein to treat multiple sclerosis. Alternatively, the naturally occurring polypeptides (i.e. MBP or PLP) or combinations thereof may be associated with a FcR ligand and administered to provide the desired immunosuppressive effect. Similarly, naturally occurring polypeptides or fragments thereof corresponding to pyruvate dehydrogenase complex or T cell receptor antagonists or agonists derived from T cell epitopes of the same proteins may be used to treat primary biliary cirrhosis. In both cases the naturally occurring or derived immunosuppressive factors will be incorporated in a immunomodulating agent as described herein and administered to a patient in need thereof. Effective presentation of the immunosuppressive factor (including agonists resulting from administration of naturally occurring autoantigens) will selectively reduce stimulation of the autoreactive T cells by native peptide thereby relieving the symptoms of the subject immune disorder.

The selected immunosuppressive factor and FcR ligand, together comprising an immunomodulating agent, may be effectively administered in any one of a number of forms. More particularly, as described above, the immunomodulating agents of the present invention may combine any form of the respective elements that are functionally effective in selectively suppressing the immune response. For example, the immunomodulating agent may comprise a recombinant (or fusion) polypeptide or protein produced using molecular biology techniques known to those skilled in the art. In such cases the FcR ligand may comprise a fragment of a single immunoglobulin region constant domain or, preferably, the entire constant region. In other embodiments the immunomodulating agent may comprise a sterically bound antibody-antigen complex wherein the antigen comprises a T cell receptor antagonist or agonist or naturally occurring autoantigen. Other preferred embodiments feature an immunomodulating agent comprising a chimeric antibody wherein an immunosuppressive factor is expressed on the Fab fragment. In still other embodiments the immunomodulating agent may comprise two covalently linked molecules which comprise a effective FcR ligand and immunosuppressive factor respectively.

Particularly preferred embodiments of the instant invention will employ recombinant nucleotide constructs to code for immunomodulating agents comprising a single fusion polypeptide. Those skilled in the art will appreciate that standard genetic engineering technology can provide fusion proteins or chimeras that will comprise at least one FcR ligand and at least one immunosuppressive factor. As used herein the terms "chimera" or "chimeric" will be used in their broadest sense to encompass any polynucleotide or polypeptide comprising sequence fragments from more than one source. For example, a genetically engineered polypeptide incorporating a peptide TCR antagonist and a single Fc domain from an IgG molecule could properly be termed a chimeric or fusion protein. Similarly, a chimeric antibody may comprise a recombinant heavy chains engineered to incorporate a heterologous peptide immunosuppressive factor and a wild type light chains. For the purposes of the present invention, it is not necessary that the disparate regions be derived from different species. That is, a chimeric antibody may comprise human light and heavy chains and an engineered human TCR antagonist expressed in a CDR. Conversely, chimeric immunomodulating agents may comprise FcR ligands and immunosuppressive factors derived from different species such a human and mouse.

As such, one aspect of the present invention comprises recombinant polynucleotide molecule encoding a polypeptide wherein said polynucleotide molecule comprises at least one nucleotide sequence corresponding to a Fc receptor ligand and at least one nucleotide sequence corresponding to an immunosuppressive factor. Preferably the immunosuppressive factor corresponds to one or more naturally occurring autoantigenic polypeptides or fragments thereof or a T cell receptor antagonist or agonist and the Fc receptor ligand corresponds to at least one constant region domain of an immunoglobulin. In a particularly preferred embodiment the polynucleotide molecule encodes a nucleotide sequence corresponding to an immunoglobulin heavy chain wherein a complementarity determining region has been at least partially deleted and replaced with a nucleotide sequence corresponding to a T cell receptor antagonist or agonist. Compositions comprising mixtures of immunosuppressive factors may also be used effectively in accordance with the teachings herein.

In any case, DNA constructs comprising the desired immunomodulating agents may be expressed in either prokaryotic or eukaryotic cells using techniques well known in the art. See, for example, Maniatis et al., *Molecular Cloning: A Laboratory Manual,* Cold Spring Harbor Laboratory, New York, 1982. In preferred embodiments the engineered plasmid will be transfected into immortal cell lines which secrete the desired product. As known in the art, such engineered organisms can be modified to produce relatively high levels of the selected immunomodulating agent. Alternatively, the engineered molecules may be expressed in prokaryotic cells such as E. coli. Whatever production source is employed, products may be separated and subsequently formulated into deliverable compositions using common biochemical procedures such as fractionation, chromatography or other purification methodology and conventional formulation techniques.

The immunosuppressive factor preferably corresponds to one or more naturally occurring autoantigenic polypeptides or fragments thereof or a T cell receptor antagonist or agonist and the Fc receptor ligand preferably comprises at least part of an immunoglobulin constant region domain. More preferably, the immunomodulating agent comprises a poly peptide or chimeric antibody wherein at least one complementarity determining region (CDR) has been replaced with a T cell receptor antagonist or agonist.

It will further be appreciated that the chimeric antibodies, polypeptides and other constructs of the present invention may be administered either alone, or as pharmaceutical composition. Briefly, pharmaceutical compositions of the present invention may comprise one or more of the immunomodulating agents described herein, in combination with one or more pharmaceutically of physiologically acceptable carriers, diluents or excipients. Such composition may comprise buffers such as neutral buffered saline, phosphate buffered saline and the like, carbohydrates such as glucose, mannose, sucrose or dextrans, mannitol, proteins, polypeptides or amino acids such as glycine, antioxidants, chelating agents such as EDTA or glutathione, adjuvants (e.g. aluminum hydroxide) and preservatives. Yet, as set forth above, preferred embodiments comprise pharmaceutically acceptable carriers that do not include adjuvants capable of inducing costimulatory molecules. However, the pharmaceutical compositions of the present invention may contain one or more additional active ingredients, such as, for example, cytokines like B-interferon.

In this respect a further aspect of the present invention comprise pharmaceutical compositions for the endocytic presentation of an immunosuppressive factor on the surface of an antigen presenting cell of a vertebrate comprising at least one immunomodulating agent and a pharmaceutically acceptable carrier, said at least one immunomodulating agent comprising at least one Fc receptor ligand and at least one immunosuppressive factor. Similarly, the invention comprises methods for the preparation of a pharmaceutical composition to treat an immune disorder comprising combining at least one immunomodulating agent with a physiologically acceptable carrier or diluent wherein said immunomodulating agent comprises at least one Fc receptor ligand and at least one immunosuppressive factor. In both of these aspects the immunosuppressive factor may comprise one or more naturally occurring autoantigenic polypeptides or fragments thereof or a T cell receptor antagonist or agonist and the Fc receptor ligand may comprise at least part of a immunoglobulin constant region domain. Preferably, the immunomodulating agent will be in the form of a recombinant polypeptide or a chimeric antibody.

As indicated above, immunomodulating agents comprising chimeric antibodies are a particularly preferred aspect of the invention. Such antibodies may be formed by substituting a immunosuppressive factor, typically a peptide TCR antagonist or agonist, for at least part of one or more of the complementarity determining regions (CDR). As will be described more fully in the Examples below, the nucleotide sequence coding for the heavy chain may be engineered to replace all or part of at least one CDR with a peptide or peptide analog of all or part of an autoantigen. Upon expression by the proper cell line, the recombinant heavy chains can complex with wild type light chains to form an immunoreactive tetramer displaying two immunosuppressive factors. Those skilled in the art will appreciate that the immunoglobulin molecules may be selected from the species to be treated so as to minimize the generation of a harmful immune response (i.e. a human anti-mouse response). As the constant region of the selected immunoglobulin is essentially unmodified, this form of immunomodulating agent is readily endocytosed allowing for effective presentation of the associated immunosuppressive factor.

In other forms, the immunomodulating agents of the present invention may comprise an antigen-antibody complex wherein the antigen is an immunosuppressive factor. It will be appreciated that modern immunological techniques may be used to generate and purify the desired antibodies which are preferably monoclonal. By way of example only, a selected peptide antagonist (i.e. an analog of a peptide autoantigen) or agonist may be injected into a mouse to provide immunoreactive cells which may then be harvested and immortalized using standard methods. If desired, the murine monoclonal may be "humanized" using conventional recombinant procedures leaving a small murine variable region expressed on an otherwise human immunoglobulin that will not provoke a harmful immune response in a patient. In any case, the monoclonal antibody is complexed with the immunosuppressive factor to form the desired immunomodulating agent which may then be formulated and administered as described above. With the intact constant region forming the FcR ligand, phagocytation should be relatively rapid and presentation of the attached immunosuppressive factor efficient.

Although embodiments may comprise the Fc receptor ligands corresponding to the entire constant region, it must be emphasized that the present invention does not require that the administered immunomodulating agent comprise an intact immunoglobulin constant region. Rather, any FcR fig and that can bind to the FcR and undergo endocytosis may be used in conjunction with the selected immunosuppressive factor. Specifically, single domains of constant regions or fragments thereof may be combined with peptide antagonists to form monomeric polypeptides (having a single amino acid chain) that can suppress the immune system in accordance with the teachings herein. Such fusion proteins may be constructed which, having the minimum effective FcR ligand and/or immunosuppressive factor, may be much more stable thereby facilitating delivery and possibly increasing bioavailability. Moreover, these engineered polypeptides or proteins may be able to be administered over a period of time without provoking an immune response as is seen when administering whole antibodies of heterologous species. As such, relatively small chimeric polypeptides may prove to be effective immunomodulating agents.

Similarly, non-peptide based molecular entities may prove to be efficient immunosuppressive factors or, in combination, immunomodulating agents. Those skilled in the art will appreciate that molecular entities (peptide based or non-peptide based) that function effectively in a selected role (e.g. FcR ligand) may be provided using current procedures such as combinatorial chemistry, directed evolution or rational drug design. For example, it may be possible to use rational drug design to fashion a small non-peptide molecular entity that effectively binds to a previously elucidated Fc receptor. The derived FcR ligand may then be covalently linked (or otherwise reversibly associated) with an immunosuppressive factor such as a peptide antagonist to provide an immunomodulating agent that exhibits particular stability or other desirable traits.

As previously indicated, the immunomodulating agents or fusion proteins of the present invention are aggregated to provide constructs or structures that advantageously crosslink Fc receptors and/or induce the production of anti-inflammatory cytokines such as IL-10 and IL-6. The absolute form of the aggregated constructs is not critical and in the context of the present invention encompasses any configuration of the disclosed immunomodulating agents. In this respect, the aggregated contructs may be soluble or insoluble and may consist exclusively of the agent or may comprise a carrier, matrix, structure or particulate associated with the agent. For example, the disclosed agents may be associated or complexed with microparticle carriers comprising any biologically compatible material or may be embedded in or absorbed to a relatively long lasting lipid or polymer matrix. Those skilled in the art will appreciate that there are a multitude of commercially available structures and related methods for associating biological constructs. As such, it win be appreciated that exemplary microparticulate carriers may comprise proteins, saccharides, lipids or synthetic and natural polymers.

In particularly preferred embodiments the complexed agents will be aggregated using techniques well known in the art. Among other methods, the aggregates may be formed using heat, chemical crosslinking or precipitation, such as ammonium sulfate precipitation. The resulting aggregates may be soluble, insoluble or some mixture thereof. It will further be appreciated that, because of their β-pleated sheath structure, denaturation of immunoglobulins exposes hydrophobic groups that favor intermolecular rather than intramolecular interactions. These intermolecular interactions promote aggregation. For example, heating immunoglobulins (i.e. immunomodulating agents) for 15 minutes at 63°C leads to formation of soluble aggregates that possess many biological properties similar to immune complexes. Such aggregates or constructs are particularly effective for providing the desired immune response in a patient in need thereof.

While not wishing to be bound by any particular theory, it is believed that the aggregation of the immunomodulating agents allows them to imitate opsonized antigens. Typically, target cells efficiently digest opsonized particles and secrete biological response modifiers to enhance or down-regulate the inflammatory response as appropriate. In the case of aggregated or immobilized agents, it is believed that the constructs act to mimic a late stage immune response where the inflammatory reaction to the initial infection is being quelled. That is, the aggregated or immobilized immunomodilating agents "fool" the immunoactive cells into thinking that the infective agent has been eliminated and that the protective immune response is no longer needed. More particularly, the activated APCs secrete biological response modifiers such as IL-10 and IL-6 that down-regulate active T cells. As described herein, IL-10 production inhibits the activity of T cells specific for multiple epitopes involved in autoimmune disease (i.e. IL-10 provides bystander suppression), further alleviating the symptoms of the autoimmune disease. In addition, the immunomodulating agents may reduce the level of IFNγ in the subject to whom they are administered. In the context of the present invention, the secretion of such biological response modifiers will act to down-regulate autoreactive T cells that are responsible for the subject autoimmune disorder.

As indicated, the compositions of the present invention provide for the induction of biological response modifiers including cytokines involved in the pathways of Th₁ / Th₂ development and known to be produced by cells able to function as APCs. In this respect, the cytokines that seem to be involved in Th₁/Th₂ development include IL-4, IL-12, and IL-10. IL-6 that is produced by monocytes and appears to induce IL-4 synthesis, is involved in the development of Th₂ T cells. IL-10 can be produced by monocytes and function to inhibit APC dependent T cell activation by apparently down-regulating MHC class II expression and inhibiting the up-regulation of costimulatory molecules. The presentation of antigen by APCs on which costimulatory molecules are present at a reduced level or are absent stimulates peripheral tolerance. Advantageously, the present invention allows for the selective stimulation of these and other beneficial biological response modifiers.

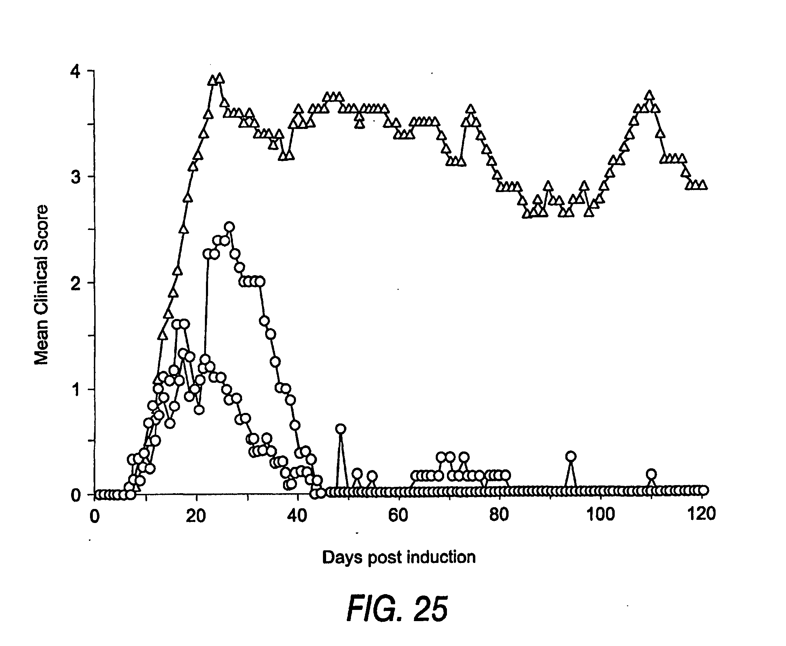

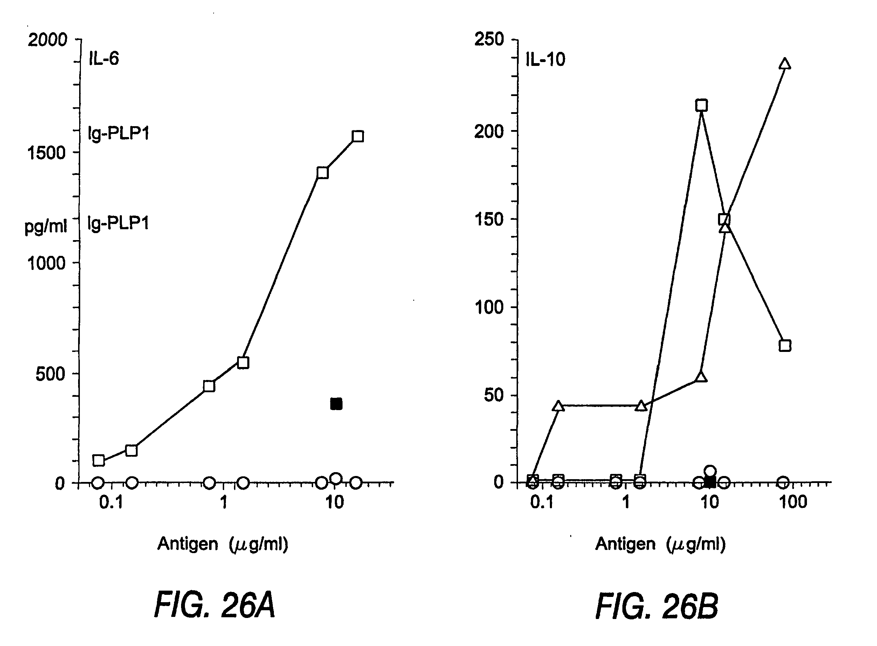

More specifically, as described in Examples XXVII and XXVIII below, aggregated immunomodulating agents may be used to ameliorate symptoms in EAE mice (Ex. XXVII) and induce the production of selected biological response modifiers in activated cells (Ex. XXVIII). In this regard, Fig. 25 shows that the administration of aggregated constructs dramatically reduces the clinical indications of disease in EAE mice. For the latter example, macrophages, dendritic cells and B cells were purified, incubated with aggregated Ig-PLP1 and tested for production of IL-6 and IL-10. The results, shown in Figs. 26A and 26B, indicate that macrophages produce both IL-5 and IL-10 while dendritic cells produce only IL-10. It appears that B cells do not produce either cytokines when stimulated with aggregated immunomodulating agents.

Those skilled in the art will appreciate that IL-10 is an anti-proliferative cytokine and is known to inhibit the production of other cytokines. APCs that bind Ig-PLP1 (particularly aggregated Ig-PLP1) and subsequently produce IL-10 could affect T cell-APC interactions in at least two ways. First, IL-10 is known to both inhibit the expression of class II molecules and the up-regulation of costimulatory molecules (Steinbrink et al., *J. Immunol.,* 159:4772-4780, 1997; Ding et al., *J. lmmunol.,* 151:1224-1234, 1993; Willems et al., *Eur. J. Immunol.,* 24:1007-1009, 1994; Moore et al., *Annu. Rev. lmmunol.,* 19:683-765, 2001; Peguet-Navarro et al., *J. lmmunol.,* 24:884-891, 1994. In addition, IL-10 may inhibit the synthesis of cytokines that are required for the activation of the T cells. The Example further shows that treated APCs could produce IL-6, a cytokine known to favor the development of Th₂ type cells. It should be appreciated that IL-6 produced by the APCs upon binding of the immunomodulating agents, may favorably affect T cell-APC interactions and induce modulation of the T cells. Further, if TGFB is produced by the APCs this also could have a modulatory effect on T cells. In this regard, aggregated or immobilized immunomodulating agents may be particularly effective for the induction of anti-inflammatory cytokine production.

In fact as shown in Examples XXIX and XXXIV, aggregated immunomodulating agents are highly effective in treating EAE. As shown in Examples XXX and XXXI, aggregated immunomodulating agents induce IL-10 production by crosslinking FcγR1 receptors. Aggregated immunomodulating agents also decrease the level of IFNγ (Example XXXII). In addition, IL-10 acts in synergy with peripheral tolerance to reduce the activity of T cells involved in autoimmune reaction (Example XXXIII). As demonstrated in Example XXXV, IL-10 produced in response to aggregated immunomodulating agents down regulates costimulatory molecules on macrophages. Aggregated immunoglobulins are also able to suppress the activity of T cells specific for multiple antigens involved in autoimmune disease through bystander suppression. (Examples XXXVI and XXXVII).

While not wishing to be bound by any particular theory, immobilized or aggregated immunomodulating agents crosslinks Fcγ receptors and induces the production of IL-10. IL-10 leads to a decrease in IFNγ production. In addition, IL-10 down regulates costimulatory molecules on the surface of the APCs, thereby leading to peripheral tolerance. IL-10 also provides bystander suppression, thereby reducing the activity of T cells directed against multiple antigens involved in autoimmune disease. This is directly supported in Example XXXIII, demonstrating the abrogation of protective effects by in vivo neutralization of IL-10.

In summary, treatment with immobilized or aggregated immunomodulating agents may induce: production of soluble mediators by APCs, including IL-10, that can directly or indirectly down-regulate the activity of pathogenic T cells; may suppress the function of APCs by down-regulating the expression of MHC class II molecules and costimulatory molecules or by reducing the level of IFNγ; may lead to presentation of therapeutic epitopes by nonprofessional APCs in which costimulatory molecules are absent or present at a reduced level, leading to antigen activated cell death or anergy. In addition, immobilized or aggregated immunomodulating agents may stimulate peripheral tolerance and/or bystander suppression. Together, these effects may translate into: prevention of generation of pathogenic T cells; functional switch of T cells from pathogenic to non-pathogenic state; generation of disease suppressing T cells; and elimination of pathogenic T cells. This can lead to prevention, stabilization or remission of autoimmune disorders in accordance with the teachings herein.

Whatever form of immunomodulating agent selected the compositions of the present invention may be formulated to provide desired stability and facilitate the selected form of administration. For example, the compositions may be administered using all the conventional routes including, but not limited to, oral, vaginal, aural, nasal, pulmonary, intravenous, intracranial, intraperitoneal, subcutaneous, or intramuscular administration. Within other embodiments of the invention, the compositions described herein may be administered as part of a sustained release implant. Within yet other embodiments, compositions of the present invention may be formulated as a lyophilizate or spray dried formulation, utilizing appropriate excipients which provide enhanced stability. These preferred formulations may then be administered using dry powder inhalers or, when combined with a carrier or propellant, from a metered dose inhaler, nebulizer, atomizer, spray bottle or dropper.

The present invention is useful for the treatment of any vertebrate comprising an immune system subject to down-regulation. The invention is particularly useful in those vertebrates such as mammals that possess cellular immune responses. In preferred embodiments the vertebrate to be treated will be in a neonatal or infant state.

In this respect, a further aspect of the invention comprises a method for treating an immune disorder comprising administering to a patient a therapeutically effective amount of a pharmaceutical composition comprising an immunomodulating agent in combination with a physiologically acceptable carrier or diluent wherein said immunomodulating agent comprises at least one Fc receptor ligand and at least one immunosuppressive factor. For this aspect, the immunosuppressive factor may comprise a T cell receptor antagonist or agonist and the Fc receptor ligand may comprise at least part of a immunoglobulin constant region domain. As previously alluded to, the immunomodulating agent will preferably be in the form of a recombinant polypeptide or a chimeric antibody. The methods may be used treat immune disorders comprising autoimmune disorders, allergic responses and transplant rejection and are particularly useful in treating autoimmune disorders selected from the group consisting of multiple sclerosis, lupus, rheumatoid arthritis, scleroderma, insulin-dependent diabetes and ulcerative colitis.

As discussed above, the compositions, compounds and methods of the present invention are particularly useful for inducing tolerance in neonatal or infant mammals thereby preventing or reducing future autoimmunity. The term "infant" as used herein, refers to a human or non-human mammal during the period of life following birth wherein the immune system has not yet fully matured. In humans, this period extends from birth to the age of about nine months while in mice, this period extends from birth to about four weeks of age. The terms "newborn" and "neonate" refer to a subset of infant mammals which have essentially just been born. Other characteristics associated with *"*infants*"* according to the present invention include an immune response which has (i) susceptibility to high zone tolerance (deletion/anergy of T cell precursors, increased tendency for apoptosis); (ii) a Th₂ biased helper response (phenotypical particularities of neonatal T cells; decreased CD40L expression on neonatal T cells); (iii) reduced magnitude of the cellular response (reduced number of functional T cells; reduced antigen-presenting cell function); and (iv) reduced magnitude and restricted type of humoral response (predominance of IgM^{high}, IgD^{kw}, B cells, reduced cooperation between Th and B cells). In specific nonlimiting embodiments of the invention the disclosed immunomodulating agents may be administered to an infant mammal wherein maternal antibodies remain present in detectable amounts. In a related embodiment, the pregnant mother may be inoculated with the disclosed compositions so as to produce the desired T cell tolerance in the fetus. In any case the induced T cell tolerance may confer resistance to the later development of an autoimmune disease associated with the administered immunomodulating agent.

Regardless as to whether the subject is an infant or an adult, the pharmaceutical compositions of the present invention may be administered in a manner appropriate to the disease to be treated (or prevented). The quantity and frequency of administration will be determined by such factors as the condition of the patient, and the type and severity of the patients disease. Within particularly preferred embodiments of the invention, the pharmaceutical compositions described herein may be administered at a dosage ranging from 1 mg to 50 mg/kg, although appropriate dosages may be determined by clinical trials. Those skilled in the art will appreciate that patients may be monitored for therapeutic effectiveness by MRI or signs of clinical exacerbation.

Following administration, it is believed that the immunomodulating agent binds to one or more Fc receptors present on the surface of at least one type of antigen presenting cell. Those skilled in the art will appreciate that selection of the FcR ligand will, at least to some extent, determine which class of Fc receptor is used to internalize the immunomodulating agent. That is, a FcR ligand corresponding to an IgG constant region will be bound by a different class of Fc receptor than a FcR ligand corresponding to an IgE constant region. Moreover, as different classes of Fc receptors are expressed on different types of antigen presenting cells it is possible to present the immunosuppressive factor on selected APCs. For example, an FcR ligand corresponding to an IgG constant region is likely to be endocytosed by a macrophage or neutrophil and presented accordingly. This is of interest in that certain APCs are more efficient at presenting various types of antigens which, in turn, may influence which T cells are activated.

In any case, the entire immunomodulating agent is subjected to receptor mediated endocytosis by the APC and usually becomes localized in clathrin-coated vesicles. After internalization, the immunomodulating agent is processed for eventual presentation at the surface of the APC. Processing generally entails vesicle transport of the immunomodulating agent to the lysosome, an organelle comprising an acidic pH and selected enzymes including proteases. Here the immunomodulating agent is digested to provide a free immunosuppressive factor which, for the purposes of the instant invention, may be in the form of a protein, polypeptide or peptide. When the released immunosuppressive factor comprises an autoantigenic polypeptide or protein, or fragment thereof, it will be understood that the factor will be further digested to provide one or more T cell receptor agonists. Whether the peptide is an antagonist or an agonist (either administered directly as part of the immunomodulating agent or derived from an administered autoantigenic polypeptide), average presented peptide lengths may be, for example, on the order of 5 to 30 amino acids. Following digestion, at least some of the immunomodulating agent fragments, including immunosuppressive factor fragments, are associated with MHC molecules in exocytic vesicles. The MHC-immunosuppressive factor complex is then transported to the surface of the APC and presented to helper T cells.

As pointed out above, preferred embodiments of the invention use a TCR antagonist as the immunosuppressive factor presented in concert with the class II MHC molecules. Accordingly, such antagonists (which may be peptide analogs) will be used for the purposes of the following discussion. However, it must be emphasized that the present invention may be used for the receptor mediated endocytic presentation of any immunosuppressive factor that down-regulates an immune response. As such, T cell receptor agonists which provide the desired reduction in immunogenic response may be used as immunosuppressive factors and are in the purview of the present invention. Moreover, as previously indicated the presented agonist or agonists may be administered directly as the immunosuppressive factor or may be derived from an immunosuppressive agent comprising one or more autoantigenic polypeptides or fragments thereof.

That is, in selected embodiments the administered immunosuppressive factor may be an agonist peptide that will be presented in concert with MHC complexes without substantial processing following endocytic separation from the FcR ligand. For other embodiments, the immunosuppressive factor will preferably comprise at least one autoantigenic polypeptide or fragments thereof. In such cases the immunosuppressive factor will typically be processed (digested) following cleavage from the FcR ligand to provide one or more peptide agonists that will then be presented in concert with the MHC class II molecules in accordance with the teachings herein. In either case, efficient presentation of the appropriate agonist to the T cell receptor may be used to down-regulate the immune response.

Accordingly, by way of example only, a T cell may have previously been sensitized to a peptide agonist corresponding to a fragment of myelin basic protein. In multiple sclerosis this autoagonist is continuously presented thereby activating an immune response directed to constituents of the myelin sheath. More particularly, the sensitized individual T cells express thousands of receptors which selectively bind to the presented autoagonist and signal the cell. When enough of the receptors are bound, the sensitized T cell acts to mount a response i.e. secrete interleukin. In the cases where a TCR antagonist is presented in concert with MHC class II molecules the T cell will recognize the presented complex but will not be activated.

Thus, in accordance with the present invention, efficient endocytic presentation of an immunosuppressive factor (i.e. an antagonist) inhibits agonist-TCR binding through competition for the receptors. That is, the presented TCR antagonist binds effectively to the TCR of a sensitized T cell thereby precluding binding of a presented autoantigen or fragment thereof. Yet, unlike an autoantigen-TCR complex, the immunosuppressive factor-TCR complex does not signal the T cell to mount a response. Thus, the binding of the immunosuppressive factor (non-reactive agonist or antagonist) can prevent a T cell from binding enough autoantigen to reach the threshold activation level that induces the cell to act. Hence, a harmful immune response to the continuously presented autoantigen comprising a natural agonist is averted.

Alternatively, efficient FcR mediated presentation of agonists may be used to down-regulate the immune response of a mammal in accordance with the teachings herein. In this regard, the ultimately presented agonist(s) may be administered directly as the immunosuppressive factor or may be derived from autoantigenic polypeptide immunosuppressive factors which are endocytically proteolyzed. While not wishing to be bound to any particular theory, it is believed that the autoantigenic agonists may be presented by nonprofessional and/or non-activated APCs lacking costimulatory molecules or having costimulatory molecules present at reduced levels. As discussed above it was suprisingly found that this type of presentation ultimately induces the inactivation of T cells. In particular, as described below, the immunomodulating agents of the present invention induce the production of IL-10, decrease IFNγ levels, and stimulate peripheral tolerance and/or bystander suppression. In such embodiments it is preferable that the immunomodulating agent constructs are administered in vehicles which do not contain an adjuvant so as to minimize or eliminate the activation and/or production of costimulatory molecules. Particularly preferred embodiments of this aspect of the present invention may encompass immunosuppressive factors comprising one or more autoantigenic polypeptides or fragments thereof. For example, constructs in accordance with this embodiment may comprise a fusion or chimeric IgG wherein at least one of the CDR regions has been at least partially replaced with a peptide agonist derived from PLP. Such constructs, when administered in therapeutically effective amounts in an adjuvant free pharmaceutically effective carrier should be able to alleviate at least some symptoms associated with multiple sclerosis. Other effective constructs for the treatment of multiple sclerosis may include fusion polypeptides comprising the Fc region of an IgG covalently linked to a immunosuppressive factor comprising the autoantigenic proteins MBP and PLP. These constructs would again be administered in adjuvant free carriers.

It will be appreciated that the administration of one or more autoantigenic polypeptides, or fragments thereof, will typically result in the efficient endocytic presentation of more than one peptide agonist at the surface of the APCs. That is, the administered autoantigenic polypeptide(s) will likely be endocytically proteolyzed to provide several different peptide agonists which Will then be presented concomitantly. Such presentation of multiple agonists provides a solution to any difficulties associated with epitope spreading and population diversity. In this respect, it should be appreciated that autoimmune disorders may be the result of more than one autoantigenic epitope on one or more polypeptides. Similarly, different individuals in a subject population may develop autoimmunity to different epitopes on one or more autoantigenic polypeptides. Yet, as set forth above, the present invention can very cleverly obviate such difficulties by administering one or more autoantigenic polypeptides, or fragments thereof which, following normal endocytic processing, will result in the presentation of more than one agonist peptide on the surface of the APCs. That is, a full spectrum of peptide agonists may be efficiently presented using the compositions and techniques disclosed herein. It should be emphasized that the presentation of such agonists would not likely be accomplished without efficient FcR mediated uptake as is provided by the present invention. More particularly, it is unlikely that one could achieve therapeutically effective levels of agonist presentation through the simple administration of naturally occurring autoantigenic polypeptides (i.e. with out the FcR ligand) or of cocktails of free agonist peptides. It is doubtful that such compositions would be internalized efficiently enough to result in the therapeutically effective presentation of the administered agonists. Conversely, the present invention provides for effective presentation of the desired agonists at relatively low dosing.

In addition, as described below the immunomodulating agents of the present invention stimulate bystander suppression. While not wishing to be bound by any particular theory, it is believed that IL-10 secreted by APCs which have internalized the immunomodulating agents of the present invention may reduce the immune response by neighboring T cells having specificity for antigens other than those included in the immunomodulating agents.

Presentation of the following non-limiting Examples will serve to further illustrate the principles of the present invention. In this regard, a list of abbreviations and corresponding definition used throughout the following discussion and the Examples is provided:
MBP: myelin basic protein, has been implicated in the etiology of multiple sclerosis;
PLP: proteolipid protein, has been implicated in the etiology of multiple sclerosis;
PLP1: a peptide fragment of PLP comprising aa residues 139-151;
PLP-LR: a peptide analog of PLP1, does not activate PLP1 pulsed cells;
PLP2: a peptide fragment of PLP comprising aa residues 178-191;
Ig-W: an Ig construct (used herein as a control) comprising the heavy chain variable region of the anti-arsonate antibody 91 A3, linked to a Balb/cg2b constant region, and the parental 91 A3 kappa light chain;
Ig-PLP1: the same construct as Ig-W except that the heavy chain CDR3 was replaced with aa residues 139-151 of PLP,
Ig-PLP-LR: the same construct as Ig-W except that the heavy chain CDR3 was replaced with a peptide analog of aa residues 139-151 of PLP,
Ig-HA: (used as a control herein) the same construct as lg-W except that the heavy chain CDR3 was replaced with aa residues 110-120 of influenza virus HA;
PPD: purified protein derivative, whole *Mycobacterium tubercuolosis* extract used as a control activator.

For obvious practical and moral reasons, initial work in humans to determine the efficacy of experimental compositions or methods with regard to many diseases is infeasible. Thus, during early development of any drug it is standard procedure to employ appropriate animal models for reasons of safety and expense. The success of implementing laboratory animal models is predicated on the understanding that immunodominant epitopes are frequently active in different host species. Thus, methods of treating autoimmunity effective in one species, such as rodents or pigs, are effective in other species, such as humans. Only after the appropriate animal models are sufficiently developed will clinical trials in humans be carried out to further demonstrate the safety and efficacy of a vaccine in man. Accordingly, for purposes of explanation only and not for purposes of limitation, the present invention will be primarily demonstrated in the exemplary context of mice as the mammalian host. Those skilled in the art will appreciate that the present invention may be practiced with other mammalian hosts including humans and domesticated animals.

In this respect, experimental encephalomyelitis (EAE), which is used as an animal model for MS, can be induced in susceptible strains of mice with myelin auto antigens such as PLP and myelin basic protein (MBP). The encephalitogenic activity of these proteins correlates with the presence of peptides which induce in vivo class II restricted encephalitogenic T cells and consequently EAE. The peptide corresponding to aa residues 139-151 of PLP (PLP1) is encephalitogenic in H-2s SJL mice, and T cell lines specific for PLP1 transfer EAE into naive animals. Although the target antigen(s) in human MS is still debatable, the frequency of T cells specific for myelin proteins are higher in MS patients than in normal subjects. Silencing those myelin-reactive T cells may be a logical approach to reverse MS. As such, this model will be used to demonstrate the advantages of the present invention.

### Example I

### Preparation of Peptides

For the purposes of this application the amino acids are referred to by their standard three-letter or one-letter code. Unless otherwise specified, the L-form of the amino acid is intended. When the 1-letter code is used, a capital letter denotes the L-form and a small letter denotes the D-form. The one letter code is as follows: A, alanine; C, cysteine; D, aspartic acid; E, glutamic acid; F, phenylalanine; G, glycine; H, histidine; I, isoleucine; K, lysine; L, leucine; M, methionine; N, asparagine; P, proline; Q, glutamine; R, arginine; S, serine; T, threonine; V, valine; W, tryptophan; and Y, tyrosine.

All peptides used in the following examples were produced by Research Genetic, Inc. (Huntsville, Alabama) using solid state methodology and purified on HPLC columns to >90% purity using conventional methods. PLP1 peptide (HSLGKWLGHPNKF: SEQ. ID No. 1) encompasses an encephalitogenic sequence corresponding to aa residues 139-151 of naturally occurring proteolipid protein. PLP-LR (HSLGKLLGRPNKF:SEQ. ID No. 2) is an analog of PLP1 in which Trp144 and His147 were replaced with Leu and Arg (underlined), respectively. PLP1 and PLP-LR bind well to I-A^{s} class II molecules (i.e. an MHC class II structure produced by a specific strain of mice). PLP2 peptide (NTWTTCQSIAFPSK:SEQ. ID No. 3) encompasses an encephalitogenic sequence corresponding to aa residues 178-191 of PLP. This peptide also binds to I-A^{S} class II molecules and induces EAE in SJL mice. HA peptide (sequence not shown) corresponds to aa residues 110-120 of the hemagglutinin of the Influenza virus. HA binds to I-E^{D} class II molecules and is used here as control peptide.

### Example II

### Production of Murine Chimeric Immunoglobulins Comprising Exogenous Peptides

Two immunoglobulin-peptide chimeras, designated Ig-PLP1 and Ig-PLP-LR and shown schematically in Figure 1, were constructed to express peptides PLP1 and PLP-LR as described in Example 1. In both cases, the heavy chain CDR 3 loop was deleted and replaced with nucleotide sequences coding for the selected peptide. Conventional DNA sequencing analysis indicated insertion of peptide nucleotide sequences in the correct reading frame.

The genes used to construct these chimeras include the gene coding for the BALBK IgG₂b constant region as described by Gillian et aL, *Cell.* 33:717,1983, the gene coding for the 91A3 heavy chain variable region as described by Ruthban et al., *J. Mol. Bio.,* 202:383-398, 1988, and the gene coding for the entire 91 A3 kappa light chain as described by Gary et aL, *Proc. Natl. Acad Sci,* 84:1085-1089, 1987. The procedures for deletion of the heavy chain CDR3 region and replacement with nucleotide sequences coding for PLP1 and PLP-LR are similar to those described by Zaghouani et al., *J.Immunol.,* 148:3604-3609, 1992 for the generation of Ig-NP a chimera carrying a CTL epitope corresponding to aa residues 147-161 of the nucleoprotein of PR8 influenza A virus. The same reference reports that the CDR3 of the 91A3 IgG is compatible for peptide expression, and that both class I and class II-restricted epitopes have been efficiently processed and presented to T cells when grafted in place of the naturally occurring segment.

Briefly, The 91A3V_{H} gene was subcloned into the EcoRl site of pUC19 plasmid and used as template DNA in PCR mutagenesis reactions to generate 91A3V_{H} fragments carrying PLP1 (91A3V_{H}-PLP1) and PLP-LR (91A3V_{H}-PLP-LR) sequences in place of CDR3. Nucleotide sequencing analysis indicated that full PLP1 and PLP-LR sequences were inserted in the correct reading frame (not shown). The 91A3V_{H}-PLP1 and 91A3V_{H}-PLP-LR fragments were then subcloned into the EcoRl site of pSV2-gpt-Cg2b in front of the exons coding for the constant region of a Balb/cg2b which generated pSV2-gpt-91A3V_{H}-PLP1-Cg2b and pSV2-gpt-91A3V_{H}-PLP1-LR-Cg2b plasmids, respectively. These plasmids were then separately cotransfected into the non-Ig producing SP2/0 B myeloma cells with an expression vector carrying the parental 91 A3 light chain, pSV2-neo-91A31. Transfectants producing Ig chimeras were selected in the presence of geneticin and mycophenolic acid. Transfectants were cloned by limiting dilution and final clones secreted 1 to 4 mg/mL of lg-PLP1 or Ig-PLP-LR (collectively, the Ig-PLP chimeras). The selected cell lines, designated Ig-PLP1-9B11 and Ig-PLP-LR-21A10, are maintained in permanent storage in the inventor's laboratory.

Chimeric and wild-type antibodies were also used as controls. For example Ig-HA, an IgG molecule carrying in place of the D segment the HA110-120 T helper epitope from the HA of influenza virus that differs from Ig-PLP1 and Ig-PLP-LR only by the peptide inserted within CDR3. Ig-W is the product of unmodified (wild-type) 91A3V_{H} gene, Balb/cg2b constant region and 91A3 kappa light chain. Therefore it differs from Ig-PLP1 and Ig-PLP-LR in the CDR3 region which comprises the parental D segment. Finally, Ig-PLP2, is a chimeric antibody that carries within the heavy chain CDR3 loop aa residues 178-191 of PLP. Conventional cloning, sequencing, and purification procedures were used to generate the appropriate cell lines and are similar to those described by Zaghouani et al. (previously cited) and those previously used to generate Ig-HA, Zaghouani et al., *Science.* 259:224-227, 1993.

Large scale cultures of transfectants were carried out in DMEM media containing 10% iron enriched calf serum (Intergen, New York). Ig-PLP chimeras were purified from culture supernatant on columns made of rat-anti-mouse kappa chain mAb and coupled to CNBr activated Sepharose 4B (Pharmacia). Rat-anti-mouse kappa chain mAb (RAM 187.1 or ATCC denotation, HB-58) and mouse anti-rat kappa light chain mAb (MAR 18.5 or ATCC denotation, TIB 216) were obtained from the ATCC. These hybridomas were grown to large scale and purified from culture supernatant on each other. The rat anti-mouse kappa mAb was used to prepare the columns on which the Ig-PLP chimeras were purified from culture supernatant. To avoid cross contamination separate columns were used to purify the individual chimeras.

### Example III

### Purification of Proteolipid Protein

Native proteolipid protein or PLP was purified from rat brain according to the previously described procedure of Lees et al., in Preparation of Proteolipids, *Research Methods in Neurochemistry,* N. Marks and R. Rodnight, editors. Plunemum Press, New York, 1978.

Briefly, brain tissue was homogenized in 2/1 v/v chloroform/methanol, and the soluble crude lipid extract was separated by filtration through a scintered glass funnel. PLP was then precipitated with acetone and the pellet was redissolved in a mixture of chloroform/methanol/acetic acid and passed through an LH-20-100 sephadex column (Sigma) to remove residual lipids. Removal of chloroform from the elutes and conversion of PLP into its apoprotein form were carried out simultaneously through gradual addition of water under a gentle stream of nitrogen. Subsequently, extensive dialysis against water was performed to remove residual acetic acid and methanol.

### Example IV

### Production of Rabbit Anti-Peptide Antibodies

PLP1 and PLP-LR peptides prepared in Example I were coupled to KLH and BSA as described in Zaghouani et al., *Proc. Natl. Acad Sci USA.* 88:5645.5649, 1991 and incorporated herein by reference. New Zealand white rabbits were purchased from Myrtle's Rabbitry (Thompson Station, TN). The rabbits were immunized with 1 mg peptide-KLH conjugates in complete Freund's adjuvant (CFA) and challenged monthly with 1 mg conjugate in incomplete Freund's adjuvant (IFA) until a high antibody titer was reached. The peptide-BSA conjugates were coupled to sepharose and used to purify anti-peptide antibodies from the rabbit anti-serum.

### Example V

### Characterization of Rabbit Anti-Peptide Antibodies

Capture radioimmnoassays (RIA) were used to assess expression of PLP1 and PLP-LR peptides on an IgG molecule using Ig-PLP1 and Ig-PLP-LR made as described in Example II.

Microtiter 96-well plates were coated with the rabbit anti-peptide antibodies made in Example IV (5 mg/mL) overnight at 4°C and blocked with 2% BSA in PBS for 1 hour at room temperature. The plates were then washed 3 times with PBS, and graded amounts of Ig-PLP1 and Ig-PLP-LR were added and incubated for 2 hours at room temperature. After 3 washes with PBS, the captured Ig-PLP1 and Ig-PLP-LR were detected by incubating the plates with 100 x 10³ cpm ¹²⁵I-labeled rat anti-mouse kappa mAb for 2 hours at 37°C. The plates were then washed 5 times with PBS and counted using an LKB gamma counter. Shown are the mean ± SD of triplicates obtained with 27 mg/mL of chimeras.

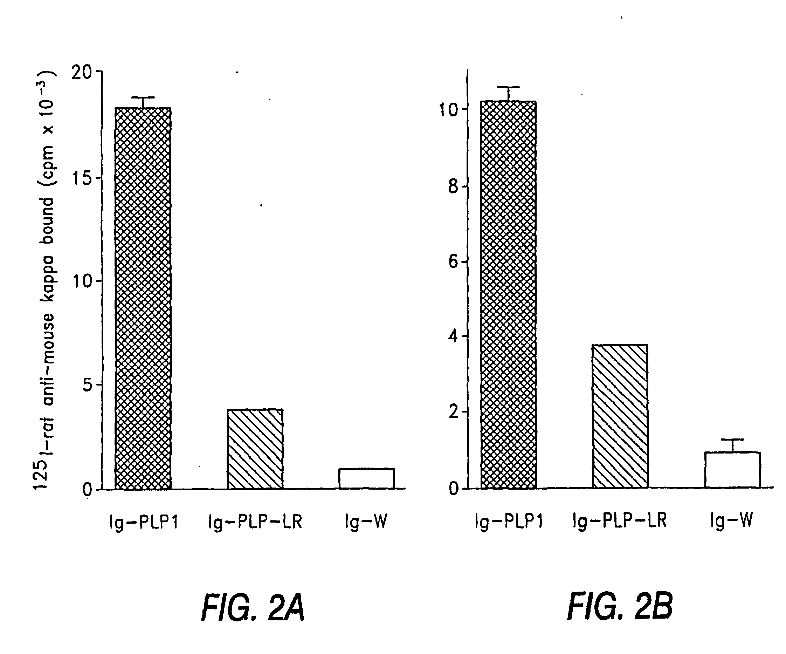

As shown in Figure 2, the rabbit antibodies directed to synthetic PLP1 and PLP-LR peptides recognized the chimeric antibodies lg-PLP1 and lg-PLP-LR produced in Example II. More specifically, when Ig-PLP1 and Ig-PLP-LR were incubated on plates coated with rabbit anti-PLP1 they were captured in significant quantity and bound labeled rat anti-mouse kappa chain mAb (Fig. 2A). Similarly, both lg-PLP1 and Ig-PLP-LR were captured by rabbit anti-PLP-LR (Fig. 2B). Conversely, Ig-W, the wild type 91A3 murine antibody without an exogenous peptide and an IgM control antibodies (not shown), did not show significant binding to the rabbit antibodies. lg-PLP1 bound to both anti-PLP1 and anti-PLP-LR better than did Ig-PLP-LR, indicating that structural differences affected accessibility of the peptides to the rabbit antibodies. Further, the results shown in Figure 2 indicate that peptide expression on the chimeras did not alter heavy and light chain pairing because the rabbit antibodies bind to the PLP peptide on the heavy chain and the labeled rat anti-mouse kappa binds on the light chain.

### Example VI

### Antigen Specific T Cell Line Proliferation Assays

PLP1-specific T cell hybridomas 5B6 and 4E3 and the IL-2 dependent HT-2 T helper cells were obtained from The Eunice Kennedy Shriver Center, Waltham, MA. The 5B6 and 4E3 T cells recognize the peptide PLP1 in association with I-A^{s} class II MHC and produces IL-2 when incubated with it as reported by Kuchroo et al., *J. Immunol.* 153:3326-3336, 1994, which is incorporated herein by reference. Conversely, Kuchroo et al. report that when stimulated with PLP1 and then with PLP-LR both 5B6 and 4E3 cells no longer produce IL-2. Similarly, stimulation of T cell hybridomas with PLP1 in the presence of PLP-LR apparently inhibits IL-2 production.

Using substantially the same technique as Kuchroo et al., activation of the T cell hybridomas for various agonists was performed as follows. Irradiated (3,000 rads) splenocytes from SJL mice were used as antigen presenting cells (APCs) for this Example. The irradiated splenocytes were incubated in 96-well round bottom plates (5 x 10⁵ cells/well/50 ml) with graded concentrations of antigens (100 ml/well). After one hour, T cell hybridomas, i.e. 5B6 or 4E3 (5 X 10⁴ cells/well/50 ml) were added and the culture was continued overnight. Activation (or proliferation) of the T cells was assessed by measuring production of IL-2 in the culture supernatant. This was done by ³H-thymidine incorporation using the IL-2 dependent HT-2 cells. That is, when IL-2 is present (i.e. secreted by activated T cells) the HT-2 cells proliferate, incorporating labeled thymidine from the surrounding media.

The culture media used to carry out these assays was DMEM supplemented with 10% FBS, 0.05 mM 2-mercaptoethenol, 2 mM glutamine, 1 mM sodium puryvate and 50 mg/mL gentamycin sulfate. Briefly, culture supernatants (100 ml/well) were incubated with HT-2 cells (1x 10⁴ cells/well/100 ml) in 96-well flat bottom plates for 24 hours. Subsequently 1 mCi ³H.thymidine was added per well and the culture was continued for an additional 12-14 hours. The cells were then harvested on glass fiber filters and the non incorporated ³H-thymidine was washed away. Incorporated thymidine was then counted using the trace 96 program and an Inotech b counter. It will be appreciated that those wells containing higher levels of IL-2 (secreted by the activated T cell hybridoma lines) will induce higher levels of HT-2 cell proliferation and register increased levels of ³H-thymidine incorporation.

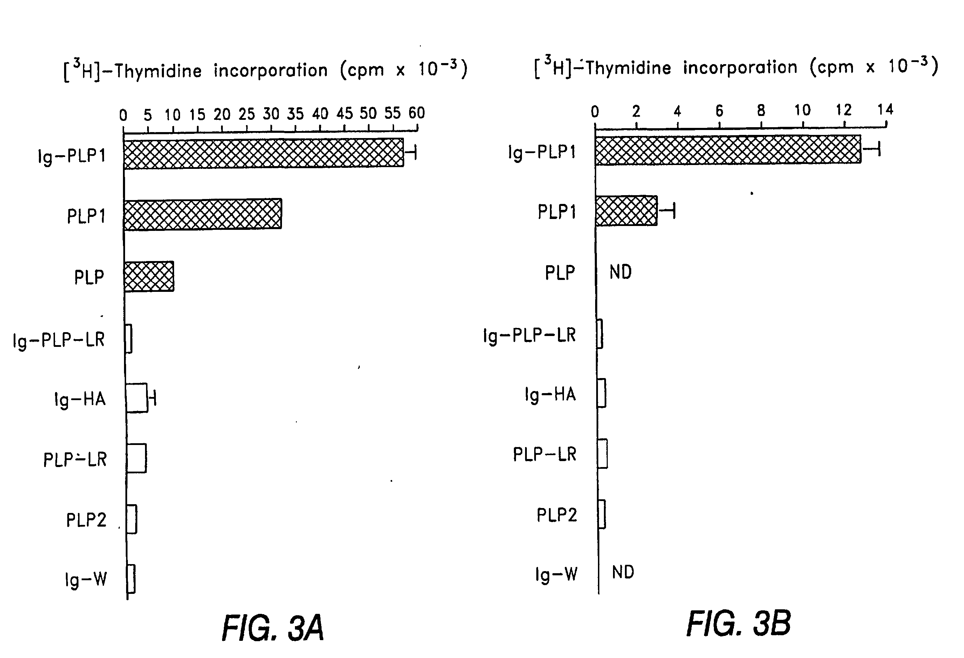

The results of the aforementioned assay using two different T cell lines are shown in Figure 3. Specifically, T cell hybridomas 4E3 (Fig. 3A) and 5B6 (Fig. 3B) produced substantial levels of IL-2 following stimulation by APCs previously incubated with lg-PLP1, PLP1 and native PLP. The negative controls lg-W, Ig-HA, and PLP2 peptide did not induce the production of IL-2 by the T cells. Similarly, both lg-PLP-LR and PLP-LR peptide did not stimulate 5B6 and 4E3 to produce significant levels of IL-2. These last results are not unexpected because the PLP-LR peptide is known to negate rather than stimulate IL-2 production. The concentration of antigen was 0.1 mM for Ig-PLP1, Ig-PLP-LR, Ig-HA, and Ig-W; 1 mM for PLP1, and PLP2 peptides; and 1.7 mM for PLP. Each value represents the mean ± SD of triplicate wells.

These results indicate that Ig-PLP1 was presented to the T cell hybridomas in a manner conducive to activation. Steric hindrance appears to preclude the simultaneous direct binding of the whole antibody to the MHC structure and TCR. As T cells will not react to soluble proteins, it appears that the PLP1 peptide was released from the lg by endocytic processing and bound MHC class II I-A^{s} molecules. Accordingly, the regions flanking the PLP1 peptide do not appear to interfere with the endocytic processing of Ig-PLP1 or the binding of the PLP1 peptide to the MHC class II structure.

### Example VII

### Presentation of PLP1 Peptide to T Cells Via Ig-PLP1

In spontaneous immune disorders, exposure and continuous endocytic presentation of an autoantigen may generate significant levels of MHC-autoantigen complexes. Currently many immune diseases lack an effective *in vitro* model for replicating this continuous presentation affording a serious impediment to the development of effective treatments. Due to relatively inefficient internalization mechanisms or the previously discussed limitations relating to free peptides, relatively high levels of natural antigens are required to provide the desired stimulation. Accordingly, one aspect of the present invention is to provide an *in vitro* model for the continuous endocytic presentation of agonist ligands.

More particularly, the present invention provides methods for the effective *in vitro* endocytic presentation of a T cell antagonist comprising the steps of:
a. providing a medium comprising a plurality of antigen presenting cells expressing Fc receptors; and
b. combining said medium with a immunomodulating agent containing composition wherein the composition comprises an immunomodulating agent having at least one Fc receptor ligand and at least one immunosuppressive factor and a compatible carrier.

Preferably the immunosuppressive factor will be at least one T cell receptor antagonist and the Fc receptor ligand will be at least part of a immunoglobulin constant region domain. Further, in preferred aspects of the invention the immunomodulating agent will comprise a recombinant polypeptide or a chimeric antibody.

In this respect, lg-PLP1 (or any immunoglobulin associated agonist) may be used for the purpose of establishing a peptide delivery system that could efficiently operate through the endocytic pathway and generate high levels of agonist ligands such that it provides an *in vitro* system to investigate the immune system. In particular, the disclosed system may be used to investigate antagonism in a situation similar to the *in vivo* presentation of autoantigens.

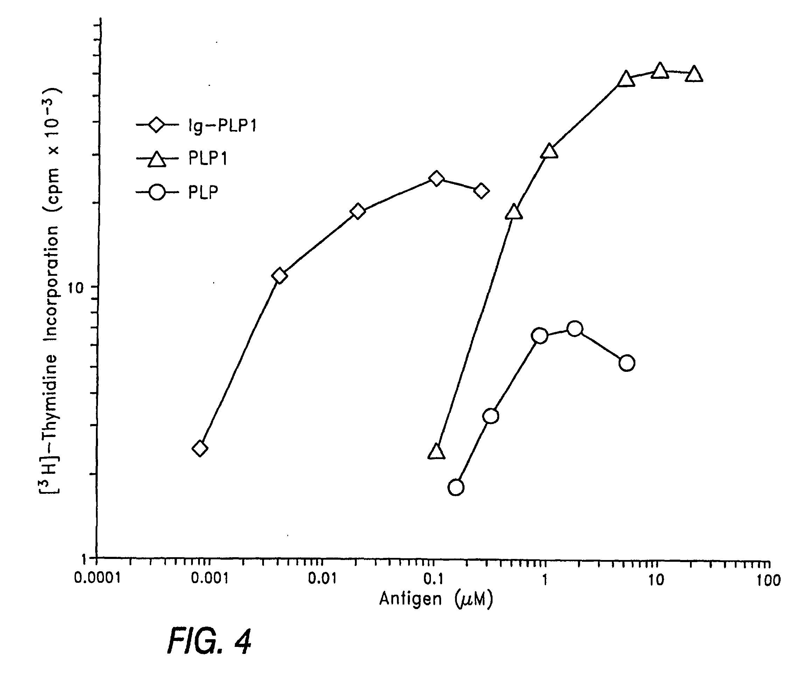

To demonstrate that immunoglobulin associated agonists may be used to mimic continuous endocytic presentation of antigens, T cell activation assays were performed with free PLP1 peptide, native PLP, and Ig-PLP1. The results of the assays are shown in Fig. 4.

Specifically, different concentrations of the three antigens (i.e. agonists) were incubated with irradiated SJL/J splenocytes which were subsequently associated with 4E3 T cell hybridomas. IL-2 production was measured by ³H-thymidine incorporation using the IL-2 dependent HT-2 cells as described in Example VI. Each point represents the mean of triplicates. The standard deviation did not exceed 10% of the mean value.

Fig. 4 shows that, although the maximum activation levels varied among the three different agonists, the levels required to stimulate the T cells were much lower for Ig-PLP1 than for either free PLP1 or native PLP. That is, it took substantially less Ig-PLP1 to stimulate the cell line than either the native PLP or the free peptide (on the order of 1/100). Specifically, stimulation to half the maximum level required less Ig-PLP1 (0.005 mM) than PLP (0.5 mM) or PLP1 peptide (0.6 mM). These results indicate that the PLP1 T cell epitope is better presented by Ig-PLP1 than by native PLP or by synthetic PLP1 peptide. Although the plateau of IL-2 production was higher when the T cell activator is free PLP1 synthetic peptide it requires substantially higher agonist levels that may be difficult to obtain *in vivo* over an extended period.

While not limiting the present invention in any way, it appears that the efficacy of Ig-PLP1 in peptide delivery is related to FcR mediated internalization and access to newly synthesized MHC molecules. More particularly, native PLP appears to internalize rather ineffectively by simple fluid phase pinocytosis while free PLP1 peptide appears to simply bind to empty MHC class II molecules at the cell surface. The ineffectual presentation of these forms of the autoantigen is clearly illustrated by Fig. 4 which unambiguously shows that lg-PLP1 is more efficient in presenting PLP1 peptide in combination with MHC class II molecules than either the free peptide or the native protein.

### Example VIII

### Inhibition of T Cell Activation In vitro

Antagonism of PLP1, PLP, and Ig-PLP1 T cell activation by Ig-PLP-LR was detected using a prepulsed proliferation assay.

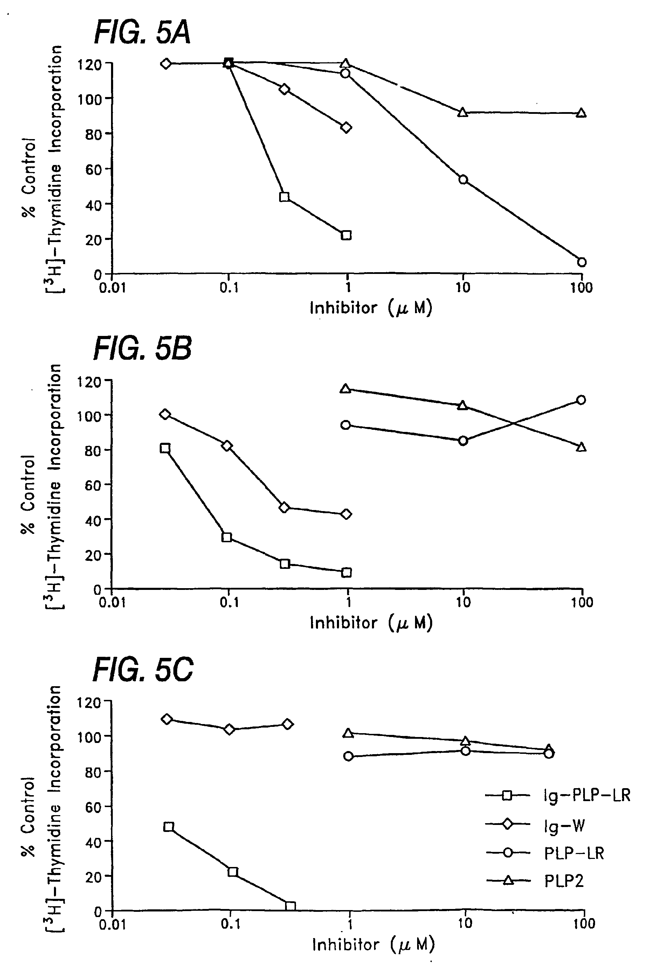

Irradiated (3,000 rads) SJL splenocytes (used as APCs) were incubated in 96-well round bottom plates (5 x 10⁵ cells/well/50 ml) with the selected agonist (1 mM PLP1 peptide, 0.05 mM Ig-PLP1 or 7 mM PLP) and various concentrations of antagonist (100 ml/well) for 1 hour. Subsequently, 4E3 T cell hybridomas (5 X 10⁴ cells/well/50 ml) were added and the culture was continued overnight. IL-2 production in the supernatant, determined as in Example VI using HT-2 cells, was used as measure of T cell activation. The results of this assay are shown in Figure 5.

More particularly, Figures 5A, 5B and 5C show antagonism of free PLP1 peptide (5A), Ig-PLP1 chimeric immunoglobulin (5B) and native PLP (5C) respectively. The antagonists were Ig-PLP-LR (squares) and PLP-LR (circles) with controls of Ig-W (diamonds) and PLP2 (triangles).

Cpm values obtained when the APCs were incubated with the agonist but no antagonist was used as control thymidine incorporation. This value was 7,503 ± 1,302 for Ig-PLP1; 31,089 ± 3,860 for PLP1 peptide; and 8,268 ± 915 for PLP. The cpm value obtained when the APCs were incubated with no agonist or antagonist was used as background (BG). This value was 1,560 ± 323 for Ig-PLP1; 2,574 ± 290 for PLP1 peptide; and 2,127 ± 177 for PLP. The percent control thymidine incorporation was calculated as follows: [(cpm obtained in the presence of test antagonist) - (BG)] / [(cpm control thymidine incorporation value)· (BG)]. Each point represents the mean of triplicates.

As previously discussed, the potency of Ig-PLP1 chimeras in peptide loading onto MHC class II molecules may resemble *in vivo* autoimmune circumstances where a continuous supply of antigen often allows for abundant generation of self peptides which can trigger T cell aggressively. Figure 5A (PLP1 agonist) shows that when T cells were incubated with APCs in the presence of both PLP1 and Ig-PLP-LR, a substantial decrease in IL-2 production occurred as the concentration of Ig-PLP-LR increased. A similar decline in IL-2 production was evident when the synthetic PLP-LR peptide was used during T cell activation with PLP1 peptide. Conversely, antagonistic effects were not observed with the control lg-W immunoglobulin and the PLP2 peptide. Inhibition of IL-2 production to half the maximum level (60% control thymidine incorporation) required only 0.4 mM Ig-PLP-LR versus 9 mM PLP-LR peptide indicating a much more efficient presentation of, and T cell antagonism by, Ig-PLP-LR.

Further evidence that the chimeric immunoglobulin is more efficient than the free peptide in T cell-antagonism is shown in Figs. 5B and 5C. Specifically, Fig. 5B shows that Ig-PLP-LR inhibited T cell activation mediated by Ig-PLP1 while free PLP-LR, like the negative control PLP2 peptide, did not show any significant antagonism. Significantly, Fig. 5B also shows that Ig-W, the wild type 91A3 immunoglobulin without any exogenous peptide exhibits partial inhibitory activity in Ig-PLP1 mediated T cell activation. It is believed that this may be the result of competition for binding to the FcR on the APCs because both Ig-PLP1 and Ig-W share identical IgG2b constant regions. A maximum of 50% inhibition in IL-2 production was seen when the activation of T cells by Ig-PLP1 was carried out in the presence of Ig-W. Thus, Ig-W would compete with Ig-PLP1 for FcR binding and internalization thereby diminishing the activation of T cells. That is, as the concentration of Ig-W increases, less Ig-PLP1 will bind to FcR and be internalize by the APCs resulting in a diminished presentation and corresponding IL-2 production. It is important to note that this Ig-W mediated reduction in response is not the result of antagonistic effects but rather simply a result of competition for FcR binding. That is, the presented Ig-W epitopes are not TCR antagonists for PLP1 and do not interact with the PLP1 specific TCRs.

In contrast to Fig. 5B, Fig. 5C shows that Ig-PLP-LR, but not lg-W, significantly reduces the activation of T cells by native PLP. As Ig-W is likely internalized in a different manner than native PLP, (Fc receptor versus simple fluid phase pinocytosis) there should not be any direct competition for uptake and processing and hence no inhibition.

For the sake of convenience the results shown in Figure 5 are summarized in Table 1 immediately below. When APCs were incubated with PLP1 peptide in the presence of lg-PLP-LR there was no activation of the PLP1-specific T cell hybridomas (Figure 5a). Moreover, when the activation of T cells by native PLP and Ig-PLP1 was carried out in the presence of various concentrations of lg-PLP-LR, IL-2 production (i.e. T-cell activation) declined as Ig-PLP-LR increased. However, free PLP-LR peptide failed to inhibit T cell activation mediated by native PLP or Ig-PLP1. These two lines of evidence indicate that the principal mechanism for lg-PLP-LR mediated inactivation of T cells was likely to be endocytic presentation and TCR antagonism rather than direct blockage of MHC class II molecules on the cell surface.

In the table below a plus sign indicates inhibition of IL-2 production and therefore antagonism, while a minus sign indicates little or no inhibition of IL-2 production and therefore little or no antagonism.

**Table 1.**

| lg-PLP-LR and PLP-LR Mediated T Cell Antagonism. | | | |
|---|---|---|---|
| Antagonist | PLP1 | PLP | Stimulator (Agonist) Ig-PLP1 |
| PLP-LR | + | - | - |
| Ig-PLP-LR | + | + | + |

The results of the foregoing example indicate that the FcR mediated uptake and subsequent processing of a peptide antagonist are compatible with efficient presentation by the antigen presenting cell. This is extremely unexpected in view of the prior art where the delivery of free peptide analogs was assumed to provide efficient antagonism through direct competition for MHC or TCR binding sites.

### Example IX

### Characterization of Mechanism for Antagonism by In-PLP-LR

Using an assay similar to the one performed in Example VIII, it was demonstrated that competition for direct binding to the Fc receptor is not, in and of itself, a likely mechanism for lg-PLP-LR mediated antagonism.

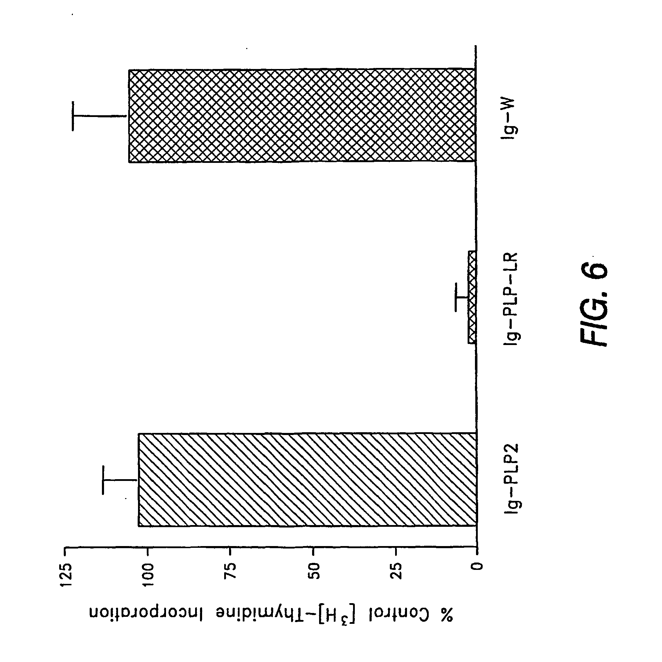

SJL splenic APCs were incubated with native PLP (6.8 mM) in the presence of 2 mM Ig-PLP2, lg-PLP-LR, or Ig-W and assayed for IL-2 production by ³H-thymidine incorporation using HT-2 cells as described in the previous Examples. Ig-PLP2 was prepared as in Example II using the sequence detailed in Example I. The % control thymidine incorporation was calculated as in Example VIII. Results of the assay are shown in Fig. 6 wherein each column represents the mean ± SO of triplicates.

As with the results shown in Fig. 5B, the present Example supports the position that both efficient presentation on the MHC class II structure and an effective peptide analog provide the most significant results. That is, even though the Ig-PLP2 chimeric antibody is taken up and processed, efficient presentation of the PLP2 peptide by I-A^{S} will not preclude activation of the T-cells as it is not an analog of the native PLP agonist. Accordingly, simple competition binding to MHC class II molecules on the antigen presenting cells is not likely to produce the desire antagonism.

### Example X

### In vivo Induction of a T Cell Response to PLP1

By this Example it was demonstrated that, in addition to generating a T cell response *in vitro* (Example VII), the chimeric antibodies of the present invention could be used to generate a cellular response *in vivo.* Specifically, the following Example demonstrates the *in vivo* priming of PLP1 specific T cells by Ig-PLP1.

Six to eight week old SJL mice (H-2^{s}) were purchased from Harlan Sprague Dawley (Frederick, MD) and maintained in an animal facility for the duration of experiments.

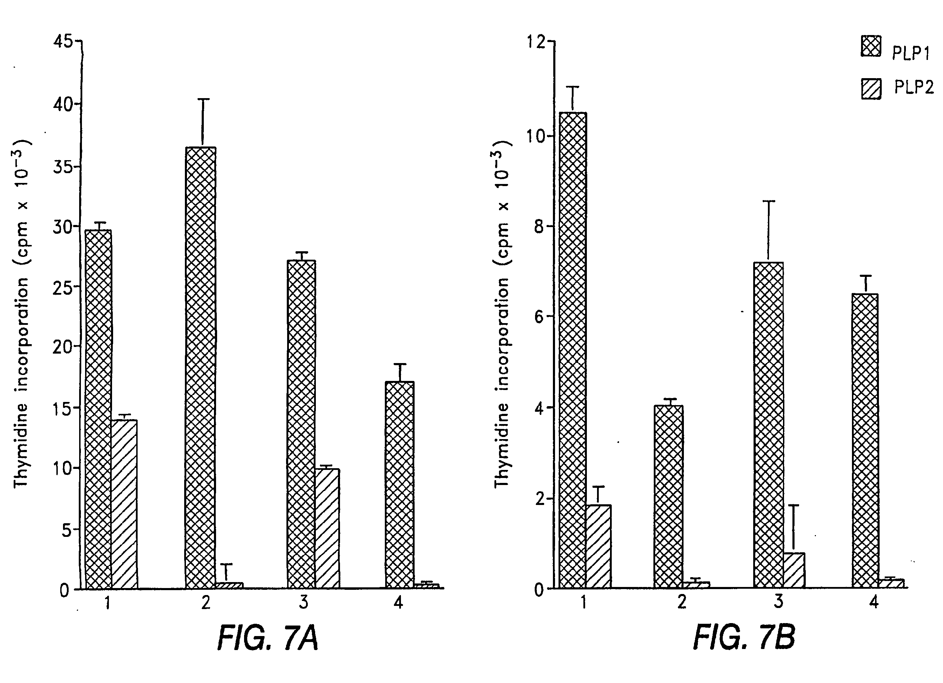

The mice were immunized subcutaneously in the foot pads and at the base of the limbs and tail with 50 mg of lg-PLP1 emulsified in a 200 ml mixture of 1:1 v/v PBS/CFA. Ten days later the mice were sacrificed by cervical dislocation, the spleens and lymph nodes (axillary, inguinal, popliteal, and sacral) were removed, single cell suspension were prepared, and the T cell responses were analyzed. The results shown in Figure 7 are those obtained with 4 x 10⁵ lymph node cells/well (7A) and 10 x 10⁵ spleen cells/well (7B). The activators PLP1 and PLP2 were used at 15 mg/mL and PPD was used at 5 mg/mL.

As with the previous Examples, T cell activation was monitored using a proliferation assay comprising ³H-thymidine incorporation. Here, lymph node and spleen cells were incubated for three days in 96-well round bottom plates, along with 100 ml of a single selected activator, at 4 and 10 x 10⁵ cells/100 ml/well, respectively. Subsequently.1 mCi ³H-thymidine was added per well, and the culture was continued for an additional 12-14 hours. The cells were then harvested on glass fiber filters, and incorporated ³H-thymidine was counted using the trace 96 program and an lnotech b counter. A control media with no stimulator was included for each mouse and used as background.

Each value shown in Figure 7 was calculated as described in Example VIII and represents the mean ± SD of triplicates after deduction of background cpms obtained with no activator in the media. Similar results were obtained when mice were immunized with 150 mg of Ig-PLP per mouse (not shown).

Figures 7A and 7B clearly show that, when lg-PLP1 was injected subcutaneously in the foot pads and at the base of the limbs and tail, a strong specific T cell response to the PLP1 peptide was induced. While there was some variation as to the strength of the reaction among the individual mice, the lymph node and spleen cells of each produced a significant response upon challenge with the PLP1 peptide. Interestingly there is a significant PLP1 specific response detected in the spleen, an organ that mostly filters and responds to systemic antigens. One possibility that can be put forth to explain these results is that Ig-PLP1, because of its long half life, was able to circulate and reach both the lymphatic and blood circulation and consequently be presented at both systemic and lymphatic sites. This is potentially very beneficial when implementing therapeutic regimens for autoimmune disorders. It was also interesting that some mice show proliferation when the cells are stimulated with PLP2 peptide *in vitro.* Possibly, the fact that this peptide is presented by I-A^{s} like PLP1 allows low affinity cells to bind and generate a response. In any case the results are consistent with those provided by the earlier Examples where it was shown that lg-PLP1 was efficient in presenting the peptide to T cells *in vitro.*

### Example XI

### In vivo Inhibition of a T Cell Response to PLP1

As seen in the previous Example, Ig-PLP1 is capable of priming T cells *in vivo* and generates a potent immune response when exposed to the agonist PLP1 peptide. This Example demonstrates that the administration of a peptide antagonist in the form of a chimeric antibody immunomodulating agent can substantially reduce the immune response generated by the endocytic presentation of an agonist ligand. Specifically, this Example demonstrates that co-administration of Ig-PLP-LR with Ig-PLP1 significantly reduces the immune response to PLP1 peptide.

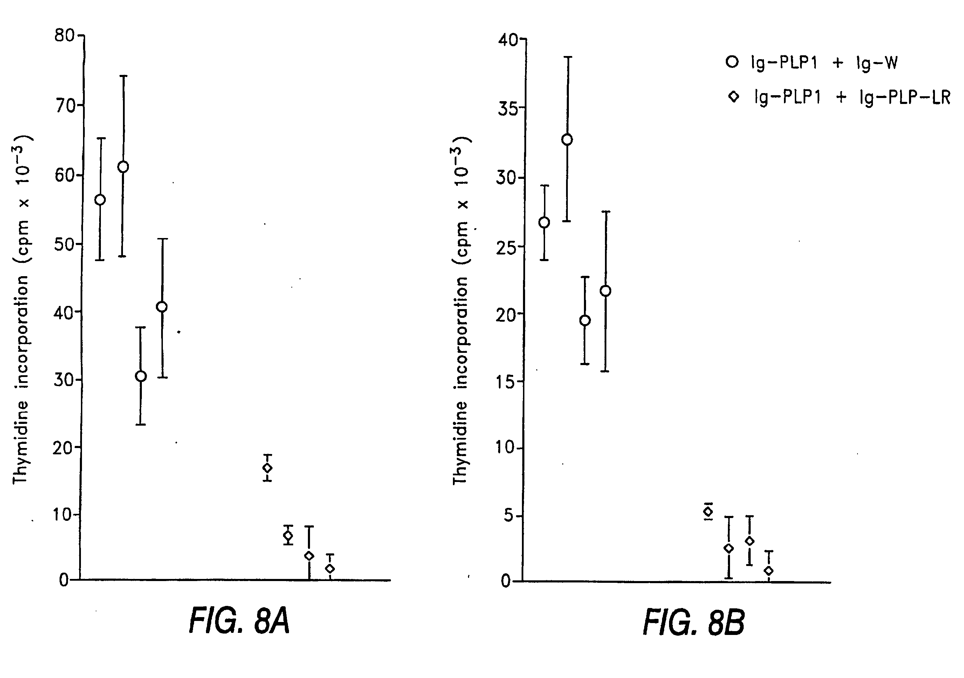

Mice were co-immunized with mixtures of either 50 mg Ig-PLP1 and 150 mg Ig-PLP-LR or 50 mg Ig-PLP1 combined with 150 mg Ig-W. In particular, individual mice from three groups (4 mice per group) were injected sc. as in Example X with a 200 ml mixture (PBS/CFA, 1:1 v/v) containing one of the following mixtures: 50 mg Ig-PLP1 and 150 mg Ig-PLP-LR; 50 mg Ig-PLP1 and 150 mg Ig-W; or Ig-PLP1 and 100 mg PLP-LR peptide. Splenic and lymph node T cell responses were analyzed at day 10 post immunization using the protocol set forth in Example X. The lymph node cells were assayed at 4 x 10⁵ cells/well and the spleen cells at 10 x 10⁵ cells/well. The agonist ligand was PLP1 at 15 mg/mL. Results for the lymph node and spleen cells, shown in Figs. 8A and 8B respectively and summarized in Table 2 below, represent the mean ± SD of triplicates after deduction of background cpm obtained with no agonist in the media.

Figures 8A and 8B show that, although Ig-PLP1 was efficiently presented and induced a strong *in vivo* T cell response (Example X), it was possible to antagonize such a response by including Ig-PLP-LR in the mixture administered to mice. Indeed, when Ig-PLP1 was co-administered to mice with Ig-PLP-LR, the subsequent immune response to free PLP1 peptide was markedly reduced as shown on the right half of Figs. 8A and 8B. It appears that the low PLP1 response for both the spleen and lymph node tissue was a result of PLP-LR antagonism, since the co-administration with Ig-PLP1 of the wild type antibody, Ig-W, did not significantly reduce the T cell response. These results strongly indicate that it is the efficient *in vivo* presentation of PLP-LR through the FcR binding and endocytic processing of lg-PLP-LR that is responsible for the reduced cellular response.

Moreover, as seen in Table 2 immediately below, when free PLP-LR peptide was co-administered with Ig-PLP1 there was no indication that the PLP1 response was reduced. The numbers provided in the table represent the percentage values of PLP1 specific proliferation relative to PPD specific proliferation and were derived as follows:
(mean cpm of triplicates obtained with PLP1 stimulation - mean cpm triplicate BG) /(mean cpm of triplicates obtained with PPD. mean cpm triplicate BG) x 100.

**Table 2**

| Ig-PLP-LR But Not Free PLP-LR Peptide Mediates T Cell Antagonism In Vitro | | | |
|---|---|---|---|
| | Ig-PLP1 co-administered with: | | |
| Mouse | Ig-W | Ig-PLP-LR | PLP-LR peptide |
| | | PLP1/PPD (%) | |
| 1 | 100 | 28 | 81 |
| 2 | 95 | 40 | 91 |
| 3 | 78 | 37 | 93 |
| 4 | 79 | 25 | 100 |

The results above clearly show that co-administration of the free antagonist peptide or the control Ig-W lacking an antagonist peptide have little effect on the generated immune response. The lack of antagonist effect by free PLP-LR peptide was not due to a net lower amount of injected peptide because the mice were given approximately 34 fold more PLP-LR in the free peptide form than in the lg-PLPLR form (on the basis of a MW of 150,000 D, the 150 µg of lg-PLP-LR given to the mice correspond to 1 nmole of Ig that contains 2 nmoles of PLP-LR peptide, while with a MW of 1,468 Daltons the 100 µg of free PLP-LR peptide corresponds to 68 nmoles of peptide). The failure of PLP-LR peptide to inhibit Ig-PLP1 mediated T cell activation coupled with the potency of lg-PLP-LR in antagonizing Ig-PLP1 T cell stimulation supports the belief that Ig-PLP-LR mediated *in vivo* antagonism is likely related to efficient presentation.

### Example XII

### Induction of a T Cell Response to an Endocytically Presented Antagonist

Previous Examples have shown that administration of chimeric antibodies comprising a agonist ligand can prime immune cells *in vivo.* It was also shown that administration of a chimeric antibody comprising an antagonist can reduce a subsequent response to challenge by an agonist ligand. This Example demonstrates that efficient presentation of an antagonist can prime immune cells *in vivo* and mount a strong response that could effect the reaction of the T cells to an agonist peptide. Specifically, mice co-injected with Ig-PLP1 and lg-PLP-LR develop a relatively high proliferative response to PLP-LR and practically no response to PLP1 peptide.

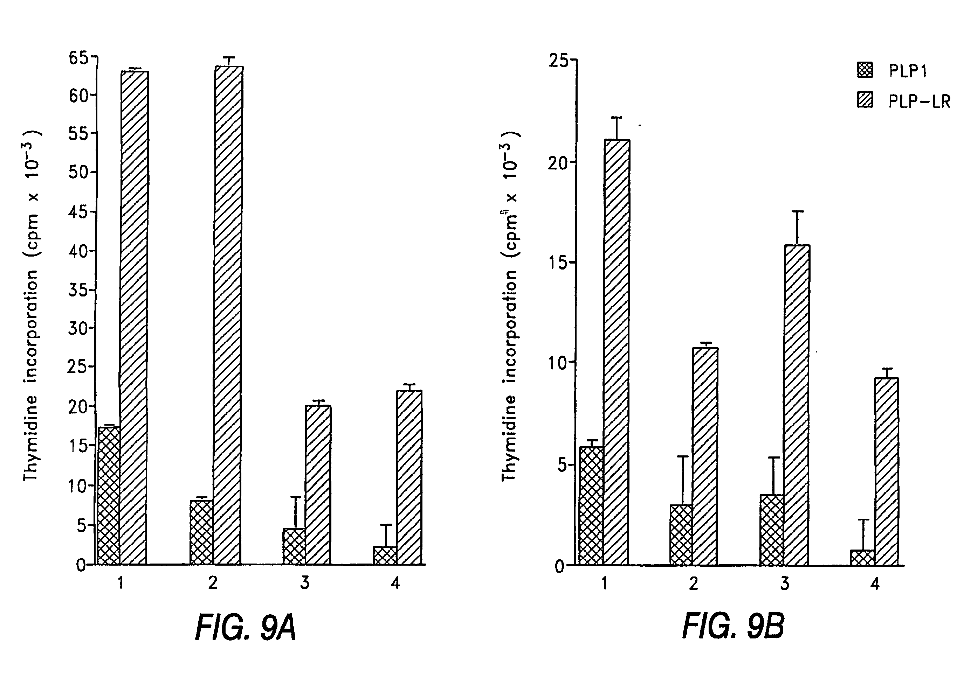

Lymph node and spleen cells were obtained in the same manner as set forth in Example X following co-administration of Ig-PLP1 and Ig-PLP-LR. Proliferative responses in individual mice were also measured using the methods set out in the previous Example following in vitro stimulation with either free PLP1 peptide or PLP-LR peptide at 15 µg/ml. The results of the assays using lymph node and spleen cells are detailed in Figures 9A and 9B respectively.

As can be seen from Figure 9, both spleen and lymph nodes developed responses to the antagonist PLP-LR but not to the PLP agonist PLP1. Knowing that Ig-PLP-LR induced PLP-LR specific T cells when it was co-administered with Ig-PLP1, it can be speculated that these PLP-LR-specific T cells down-regulate PLP1 specific T cells. Conversely, although there was induction of PLP-LR-specific response when free PLP-LR peptide was administered with Ig-PLP1 (not shown), there was no evident reduction in the proliferative response to PLP1. Accordingly, the data set forth in the instant example demonstrates that the use of chimeric antibodies comprising an antagonist are much more effective for modulating the immune response to an antigen agonist than the free peptide antagonist.

More particularly, in view of the foregoing examples it appears that TCR engagement with PLP-LR-I-A^{s} complexes (i.e. MHC-PLP-LR complexes) on the surface of APCs antagonizes T cells rather than stimulates them. Accordingly, antagonism by Ig-PLP-LR may occur because efficient presentation of Ig-PLP-LR in endocytic vacuoles ensures significant levels of PLP-LR-I-A^{s} complexes (antagonist complexes) are generated. The amount of complexes on the cell surface is proportional to the amount of Ig-PLP-LR offered to the APCs. When PLP1 stimulation is carried out in the presence of Ig-PLP-LR, both PLP-LR-I-A^{s} and PLP1-I-A^{s} are present on the surface of a given APC where an increase in the concentration of Ig-PLP-LR leads to higher number of PLP-LR-I-A^{s} complexes. It will be appreciated that approximately 3500 TCR have to be engaged in order for a T cell to be activated and that a given complex of MHC class II-peptide complex serially engages approximately 200 TCRs. As such, it appears that a T cell is antagonized when TCR engagement with PLP-LR-I-A^{s} complexes override engagement with the agonist PLP1-I.A^{s}. Overall, because of efficient loading of PLP-LR by Ig-PLP-LR, T cell antagonism is achieved by a higher frequency of serial triggering of TCR by PLP-LR-I-A^{s} complexes. That is, the efficient uptake and processing of Ig-PLP-LR simply means that too many of the surface MHC complexes present the PLP-LR antagonist to allow the remaining surface complexes presenting the PLP1 agonist ligand to engage the number of TCRs to activate the T cell. Therefore, the T cells will not be activated as long as the antagonist is presented at a rate that ensures the activation concentration of MHC class II-agonist complexes is not reached on the APC.

### Example XIII

### Lymph Node Proliferative Responses to Immunization With Ig-PLP Chimeras

Proliferative responses were measured in mice immunized with individual Ig-PLP chimeras or varying mixtures of Ig-PLP1 and Ig-PLP-LR. It was observed that Ig-PLP-LR given alone to mice induced T cells which, like those induced by Ig-PLP1, cross-reacted with both PLP1 and PLP-LR peptides. Surprisingly, however, despite the cross-reactivity of the responses, when the chimeras were administered together they displayed a dose dependent antagonism on one another resulting in down-regulation of both T cell responses. Finally, antigen specific T cells induced either by IG-PLP 1 or by IG. PLP-LR were refractory to down-regulation by peptide mixtures and proliferated significantly when they were in vitro stimulated simultaneously with both PLP1 and PLP-LR. These findings indicate that both agonist and antagonist peptides exert adverse reactions on one another and reveal an anti-parallel antagonism and a stringent control of TCR triggering at the level of naive T cells.

Materials were obtained and mice immunized as described above. Proliferative responses were measured by thymidine incorporation as set forth in Example VI above. Lymph node and spleen cells were obtained in the same manner as set forth in Example X following co-administration of Ig-PLP1 and Ig-PLP-LR. Mice were injected with 50 µg Ig-PLPI (10A), 50 µg Ig-PLP-LR (10B), 100 µg PLP1 (10C) or 100 µg PLP-LR (10D) in CFA, and 10 days later the lymph node cells were in vitro stimulated with the indicated free peptides. The stimulators PLP1, PLP-LR and PLP2 were used at the defined optimal concentration of 15 µg/ml.

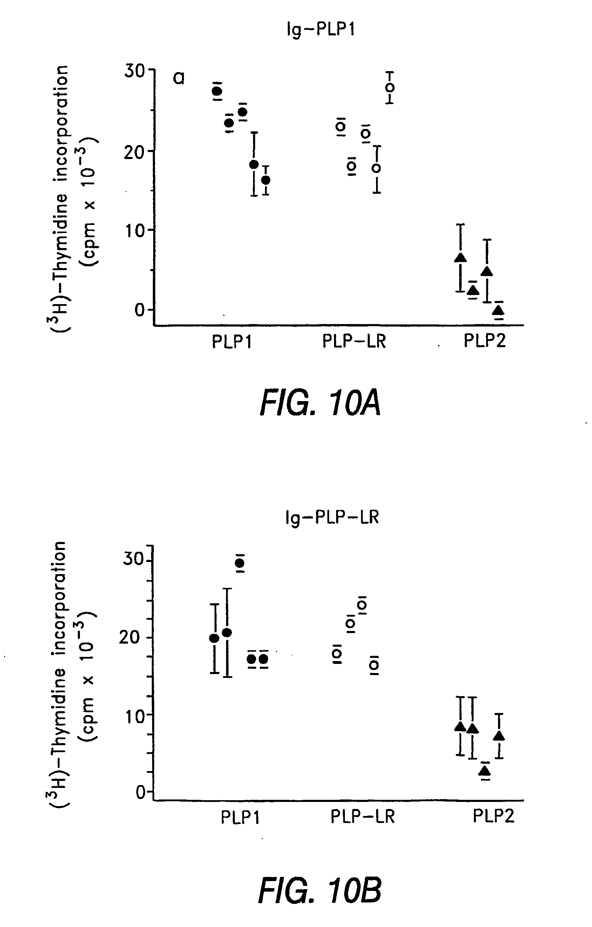

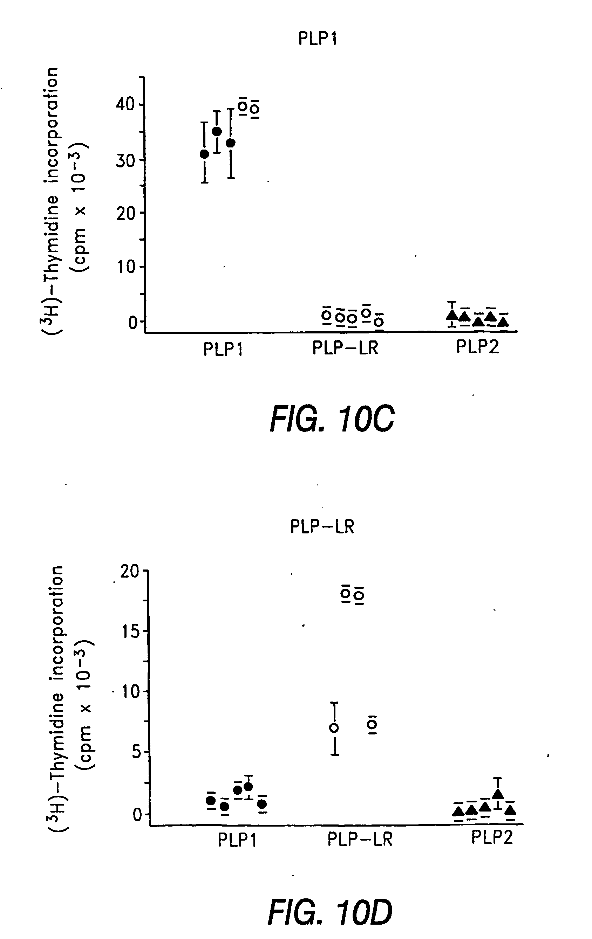

The data illustrated in figs 10A-10D indicate that Ig-PLP1, like PLP1 peptide, induced a specific T cell response to PLP1 peptide. Similarly, Ig-PLP-LR, like PLP-LR peptide, induced a specific T cell response to PLP-LR peptide. Neither the Ig chimera nor the free peptides induced T cells that significantly reacted with the negative control PLP2, a peptide that is also presented by I-A^{s} class II molecules. Surprisingly, however, the response induced by Ig-PLP1 cross-reacted with PLP-LR peptide, while the response induced by Ig-PLP-LR cross-reacted with PLP1. The responses induced with free PLP1 or free PLP-LR were not cross-reactive.

### Example XIV

### Lymph Node T cell Proliferative Response to Co-Immunization With Ig-PLP1 and Ig-PLP-LR

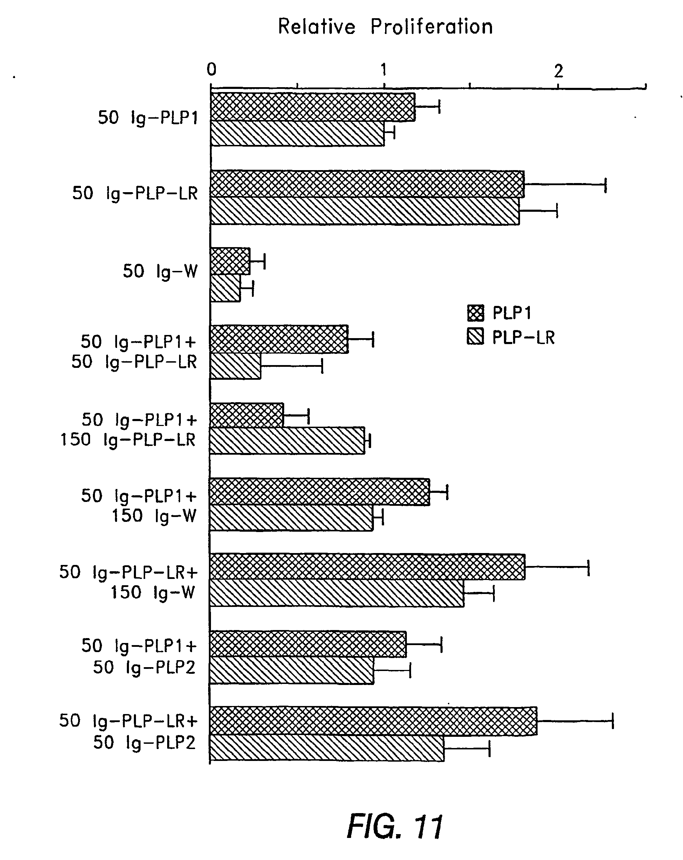

Mice were injected with the indicated chimeras and 10 days later the lymph nodes cells were in vitro stimulated with free peptides, and assayed for proliferation by [³H]thymidine incorporation as detailed above. The results are shown in Fig. 11.

The number preceding the Ig chimera label indicates the µg amount injected per mouse. The stimulators were PPD, 5 µg/ml; PLP 1, PLP-LR, and PLP2 at 15 µg/ml. Cells incubated without stimulator were used as background (BG). The mice were tested individually and triplicate wells were assayed for each stimulator. To standardize the results and eliminate intrinsic individual variability we expressed the results as relative proliferation estimated as follows: (mean test peptide cpm - mean BG cpm)/(mean PPD cpm - mean BG cpm). The indicated relative proliferation represents the mean ± SD of 5 mice tested individually. The mean cpms ± SD obtained with PPD stimulation for the different groups of mice were as follows: 50µg Ig-PLP1:16,413 ± 1330; 50µg lg-PLP-LR: 11,224 ± 3481; 50µg Ig-W: 11,513 ± 1,572; 50µg Ig-PLP1 + 50µg Ig-PLP-LR: 16,817 ± 2,869; 50µg Ig-PLP1 + 150µg Ig-PLP-LR: 16,156 ± 2006; 50µg Ig-PLP1 + 150µg Ig-W: 11,699 ± 1,142; 50µg Ig-PLP-LR + 150µg Ig-W: 13,435 ± 1,650; 50µg Ig-PLP1 + 50µg Ig-PLP2:10,056 ± 1,407; and 50µg Ig-PLP-LR + 50µg Ig-PLP2:10,877 ± 563. Filled and hatched bars indicate proliferation to PLP1 and PLP-LR respectively. The proliferation to PLP2 peptide was at background levels except where Ig-PLP2 was used in the immunization mixture.

As can be seen in Figure 11, lymph node T cells from a group of mice that were immunized with Ig-PLP1 proliferated equally well to PLP1 and to PLP-LR whereas Ig-W control caused little reaction. Surprisingly, the PLP.LR response was at background levels. Accordingly, although the responses to the Ig chimeras share cross-reactivity between PLP1 and PLP-LR peptides, the mixture yielded down-regulation rather than additive responses. In fact, the data suggest an anti-parallel down-regulation among lg-PLP1 (agonist) and Ig-PLP-LR (antagonist). This down-regulation appeared to be dose dependent because mice that were injected with a mixture of 50 µg Ig-PLP1 and 150 µg Ig-PLP-LR failed to respond to PLPI and mounted responses to PLP-LR that were reduced to levels observed with mice injected with lg-PLP1 alone.

One possible explanation for the observed opposite down-regulation between IG-PLPI and Ig-PLP-LR is that clonal expansion requires an optimal serial triggering with an homogeneous peptide (i.e. all or most of the receptors on a single naive T cell must engage one type of peptide in order to expand). Simultaneous stimulation of naive T cells with peptides encompassing subtle differences at the TCR contact residues, which may be occurring during immunizations involving mixtures of Ig-PLP1 and lg-PLP-LR, fails to cause T cell expansion and in vitro proliferation.

### Example XV

### Splenic Proliferative T Cell Responses of Mice Co-Immunized with In-PLP1 and IG-PLP-LR

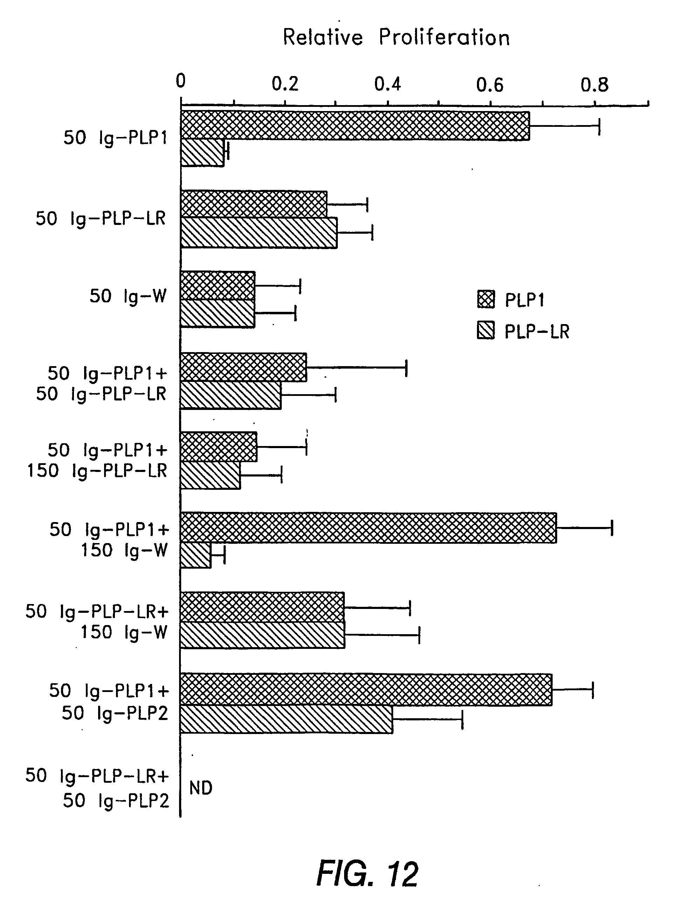

As shown in Figure 12, spleen cells from the mice described in Example XIV were stimulated with PLP1 (filled bars) and PLP-LR ( hatched bars.) in triplicate wells and proliferation was measured as above. The results were standardized as above using PPD cpms obtained with lymph node T cells because the proliferation of spleen cells upon stimulation with PPD was minimal The indicated relative proliferation represents the meant ± SD of 5 individually tested mice.

Splenic T cells from these mice failed to respond to PLP-LR stimulation. However, when an additional group of mice was immunized with lg-PLP-LR, both lymph node and splenic cells proliferated to PLP1 as well as to PLP-LR peptide. In the spleen, although the proliferative responses were much lower than in the lymph nodes, additive responses were still not observed. Rather, an opposite down-regulatory effect between lg-PLP1 and lg-PLP-LR was observed. Although co-injection of Ig-W with either lg-PLP1 or lg-PLP-LR did not affect either response, co-injection of Ig-PLP2 with lg-PLP1 increased reactivity to PLP-LR among the T cells induced by Ig-PLP1.

### Example XVI

### IL-2 Production by Splenic Cells of Mice Co-Immunized With Ig-PLP1 and Ig-PLP-LR

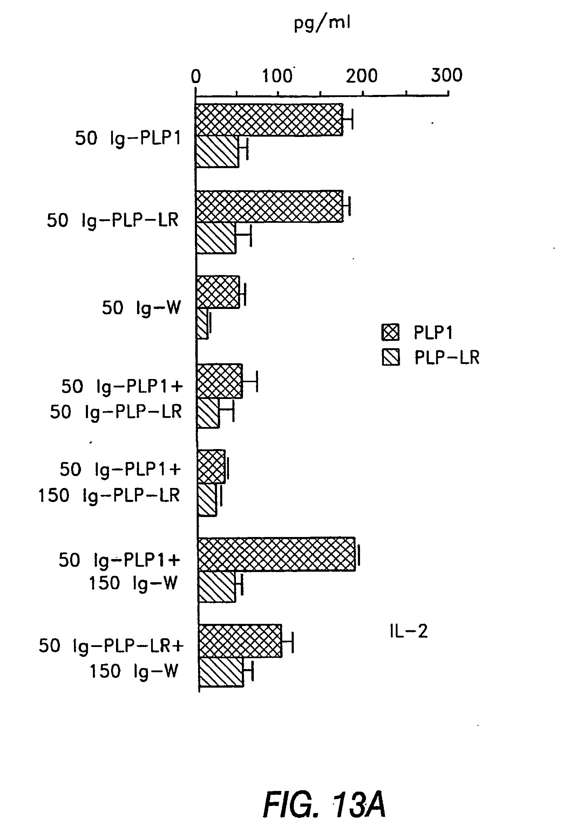

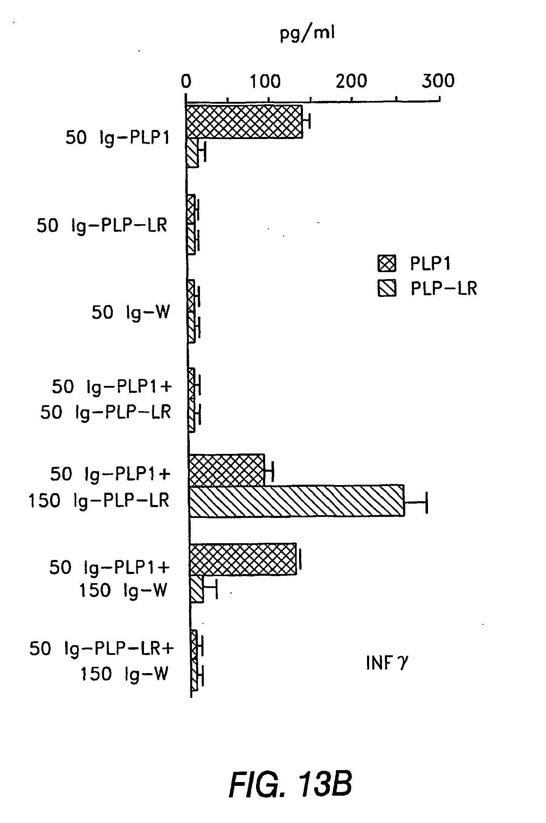

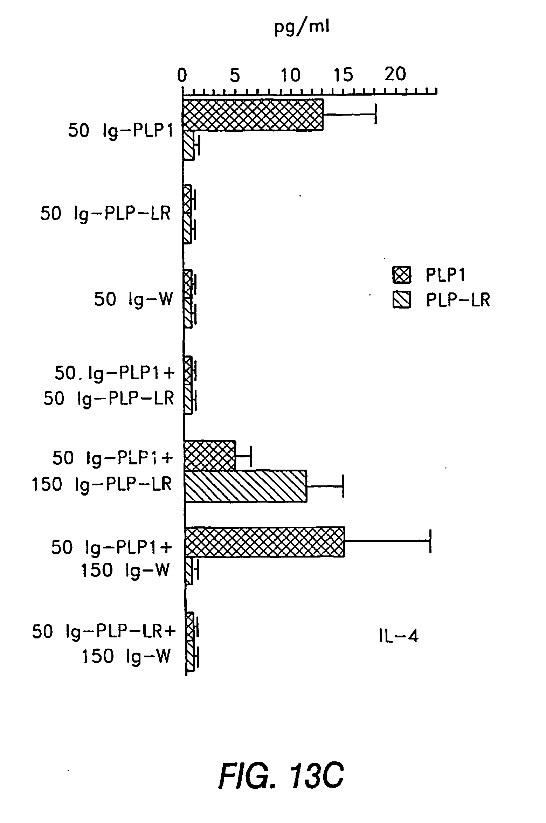

To further investigate the opposing down-regulation among Ig-PLP1 and lg-PLP-LR, splenic antigen induced cytokine responses were measured in animals immunized with either a single or both Ig-chimeras. As shown in Fig. 13, spleen cells (1 X 10⁶ per well) from the mice described in Example XIV were stimulated with PLP1 (filled bars) and PLP-LR (hatched bars) for 24 hours. Production of IL-2 (13A), IFNγ (13B), and IL-4 (13C) were measured as set forth below.

Cells were incubated in 96 well round-bottom plates at 10 x 10⁵ cells/100µl/well with 100µl of stimulator, as above, for 24 hours. Cytokine production was measured by ELISA according to Pharmingen's instructions using 100 µl culture supernatant. Capture antibodies were rat anti-mouse IL-2, JES6-IA12;rat anti-mouse IL-4, 11B11;rat anti-mouse IFNγ, R4-6A2; and rat anti-mouse IL10, JES5-2A5. Biotinylated anti-cytokine antibodies were rat anti-mouse IL-2, JES6-5H4; rat anti-mouse IL-4, BVD6-24G2; rat anti-mouse IFNγ, XMG 12; and rat anti-mouse IL-10, JES5-16E3) The OD405 was measured on a Spec 340 counter (Molecular Devices) using SOH MAX PRO version 1.2.0 software. Graded amounts of recombinant mouse IL-2, IL-4, IFNγ, and IL-10 were included in all experiments in order to construct standard curves. The concentration of cytokines in culture supernatants was estimated by extrapolation from the linear portion of the standard curve. Cells incubated without stimulator were used as background (BG). Each mouse was individually tested in triplicate wells for each stimulator and the indicated cpms represent the mean ± SD after deduction of BG cpms. Production of IL-10 was also measured, but the results were at background levels (not shown).

Upon in vitro stimulation with PLP1 peptide, T cells from Ig-PLP1 immunized mice produced IL-2, IFNγ, and small amounts of IL-4. However, stimulation of the same cells with PLP-LR yielded minimal IL-2 and undetectable IFNγ or IL-4. Spleen cells from Ig-PLP-LR immunized mice generated IL-2 but no IFNγ or IL-4 upon stimulation with PLP1 peptide. Moreover, PLP-LR peptide stimulation produced only a minimal IL-2 response. In mice immunized with equal amounts of lg-PLP1 and Ig-PLP-LR all cytokine production was reduced to minimal or background levels upon stimulation with either peptide. Co-immunization of Ig-W with either chimera had no measurable effect on cytokine production pattern. When the animals were given a 3:1 ratio of Ig-PLP-LR: Ig-PLP1, although the splenic proliferative responses and IL-2 production were at background levels, significant amounts of IL-4 and IFNγ were evident upon stimulation with PLP-LR peptide. Consequently, the excess of Ig-PLP-LR may lead to a mixed but PLP-LR dominant TCR triggering that induces cells able to produce cytokine but which exhibit no proliferative response. These data indicated that lg-PLP1 and Ig-PLP-LR exerted adverse reactions on one another leading to down-regulation of both T cell responses.

### Example XVII

### Proliferation of Antigen Experienced T Cells Upon Stimulation In Vitro With Mixtures of PLPI and PLP-LR Peptides

To investigate whether lg-PLP1 and Ig-PLP-LR could display adverse reactions on each other at the level of antigen experienced cross-reactive T cells, mice were immunized with lg-PLP1 or lg-PLP-LR alone and assessed for proliferative T cell responses upon *in vitro* stimulation with varying mixtures of free PLP1 and PLP-LR peptides.

More particularly Mice (4 per group) were immunized with 50µg Ig-PLP1 (14A and 14B) or 50µg Ig-PLP-LR (14C and 14D) in CFA, and 10 days later the lymph node (14A and 14C) and spleen (14B and 14D) cells were stimulated with the indicated peptides and assayed for [³H]thymidine incorporation as above. The number preceding the peptide label indicates the µg/ml amount used for in vitro stimulation. The specific proliferation was estimated by deducting the mean BG (obtained by incubating cells without stimulator) cpm from the test sample cpm. The indicated cpms represent the mean ± SD of 4 individually tested mice. ND, not determined.

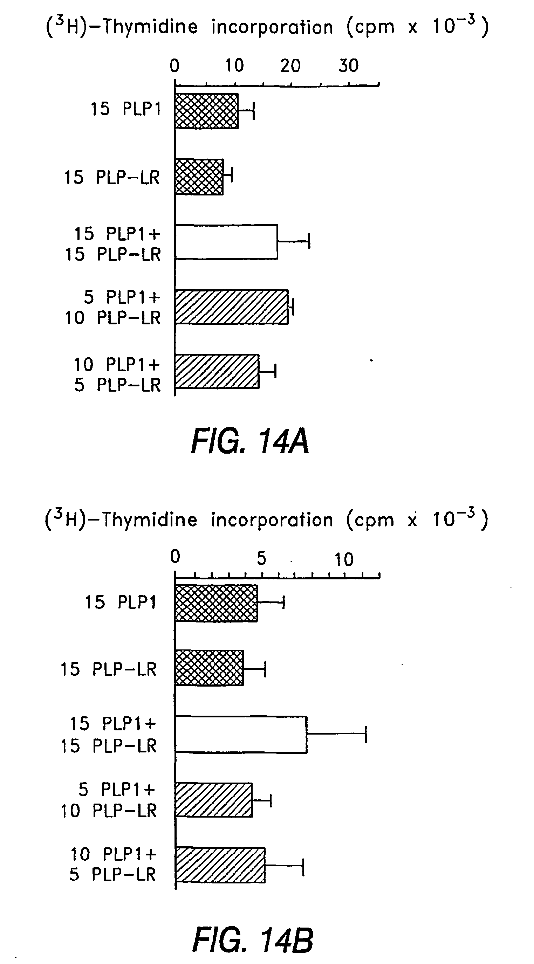

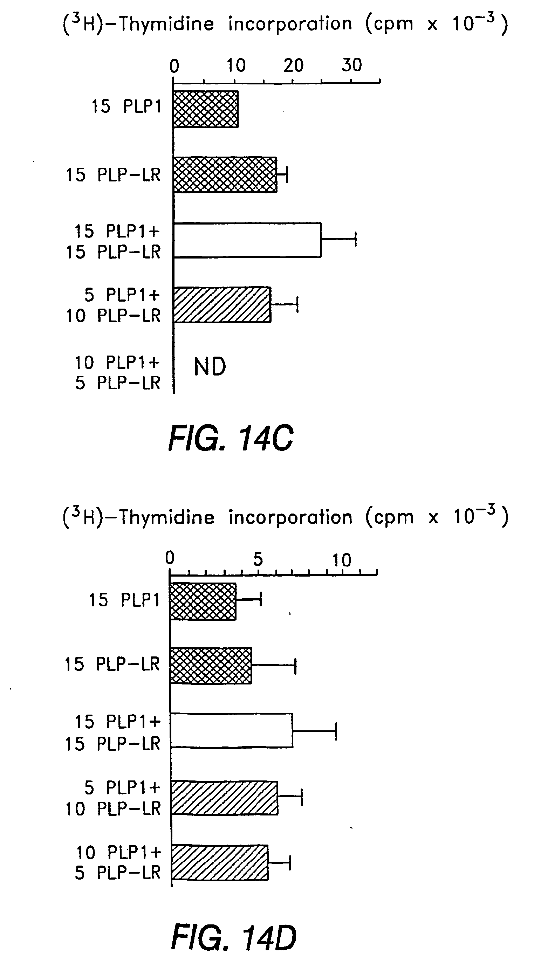

As can be seen in Figs. 14A-14D, both lymph node and spleen cells from mice immunized with Ig-PLP1 or Ig-PLP-LR proliferated equally as well to stimulation with a single peptide as to a mixture of PLP1 and PLP-LR. The proliferative response to the mixture, in most cases, was even higher than the response to a single peptide stimulation.

### Example XVIII

### IL-2 Production by Antigen Experienced T Cells Upon In Vitro Stimulation With PLP1/PLP-LR Peptide Mixtures

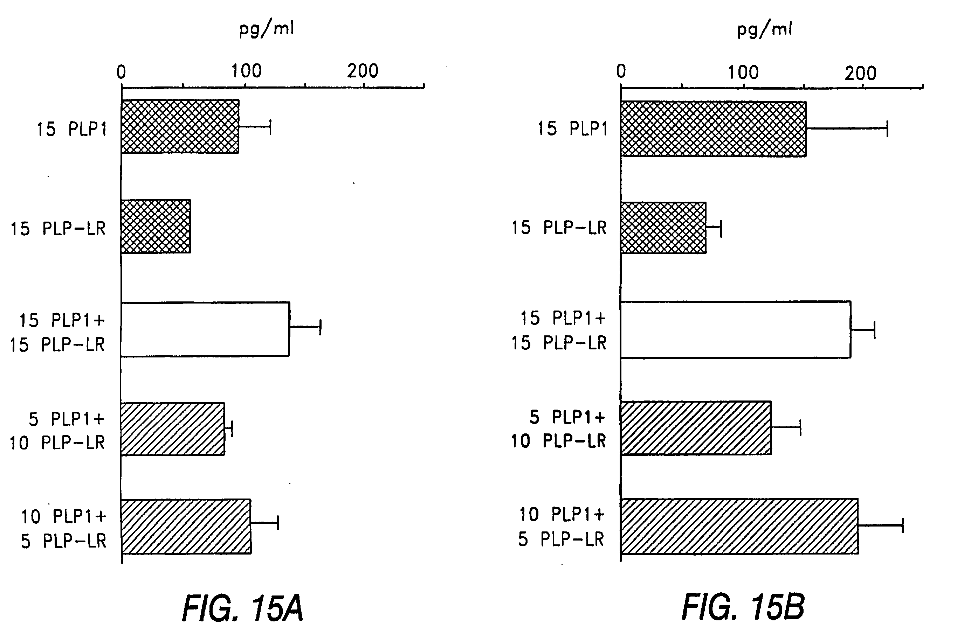

To further investigate whether lg-PLP1 and Ig-PLP-LR could display adverse reactions on each other at the level of antigen experienced cross-reactive T cells, mice were immunized with Ig-PLP1 or Ig-PLP-LR alone and assessed for cytokine responses upon *in vitro* stimulation with varying mixtures of free PLP1 and PLP-LR peptides. The results are shown in Figs. 15A and 15B.

Spleen cells from lg-PLP1 (15A) and Ig-PLP-LR (15B) immunized mice were stimulated with the indicated peptides and tested for IL-2 production by ELISA as in Example XVI. The spleen cells used in these experiments were from the mice described in Example XVII. The number preceding the name of the peptide represents the µg/ml amount used for stimulation. The indicated µg/ml IL-2 values represent the mean d: SD of 4 individually tested mice.

As indicated by Example XVII, IL-2 production was not decreased upon stimulation of spleen cells with varying mixtures of PLP1 and PLP-LR. To the contrary, in most cases of stimulation with peptide mixture IL-2 production was higher than in stimulation with a single peptide. Again these findings indicate that both agonist and antagonist peptides exert adverse reactions on one another and reveal an anti-parallel antagonism and a stringent control of TCR triggering at the level of naive T cells.

In addition to the use of immunomodulating agents comprising T cell receptor antagonists and agonists for attenuation of adult immune responses, the same compositions may advantageously be used for the induction of tolerance in neonates and infants as demonstrated in the following Examples.

### Example XIX

### SJL/J Mice Injected with Ig-PLP1 at Birth Resist Induction of EAE During Adult Life

To demonstrate the advantages of inoculating neonates or infants with the compositions of the present invention, newborn mice were administered immunomodulating agents as described herein and exposed to agents for the inducement of an autoimmune condition.

More specifically, neonatal mice (10 mice per group) were injected with 100 µg of affinity chromatography purified Ig-PLP1 or lg-W within 24 hours of birth and were induced for EAE with free PLP1 peptide at 7 weeks of age. Mice were scored daily for clinical signs as follows: 0, no clinical signs; 1, loss of tail tone; 2 , hind limb weakness; 3, hind limb paralysis; 4, forelimb paralysis; and 5, moribund or death. Panel A shows the mean clinical score of all mice and panel B shows the mean score of the surviving animals only. EAE was induced by subcutaneous injection in the foot pads and at the base of the limbs and tail with a 200µl IFA/PBS (1vol/1vol) solution containing 100 µg free PLP1 peptide and 200 µg *M. tuberculosis* H37Ra. Six hours later 5 x 10⁹ inactivated B. *pertussis* were given intravenously. After 48 hours another 5 x 10⁹ inactivated *B. pertussis* were given to the mice.

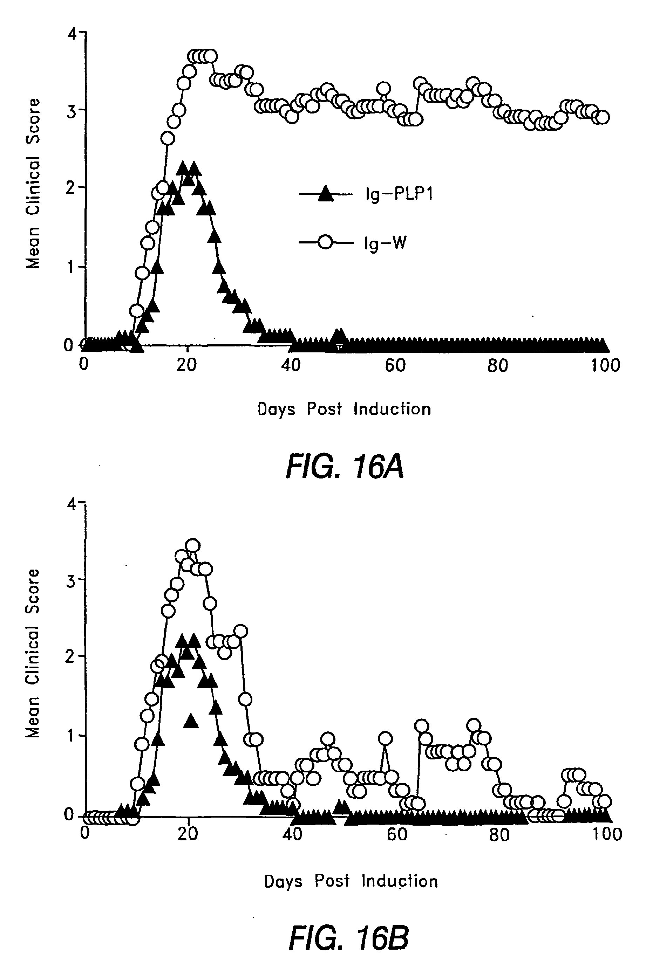

As may be seen in Figs 16A and 16B adult mice recipient of Ig-PLP1 in saline at birth resisted the induction of EAE by free PLP1 peptide. Indeed, the clinical scores were much less severe in those mice than in animals recipient of Ig-W, the parental wild type Ig without any PLP peptide. In addition, contrary to those mice which received Ig-W, mice injected with Ig-PLP1 showed no relapses (figure 16B).

### Example XX

### In Vivo Presentation of Ig-PLP1 by Neonatal Thymic and Splenic Antigen Presenting Cells

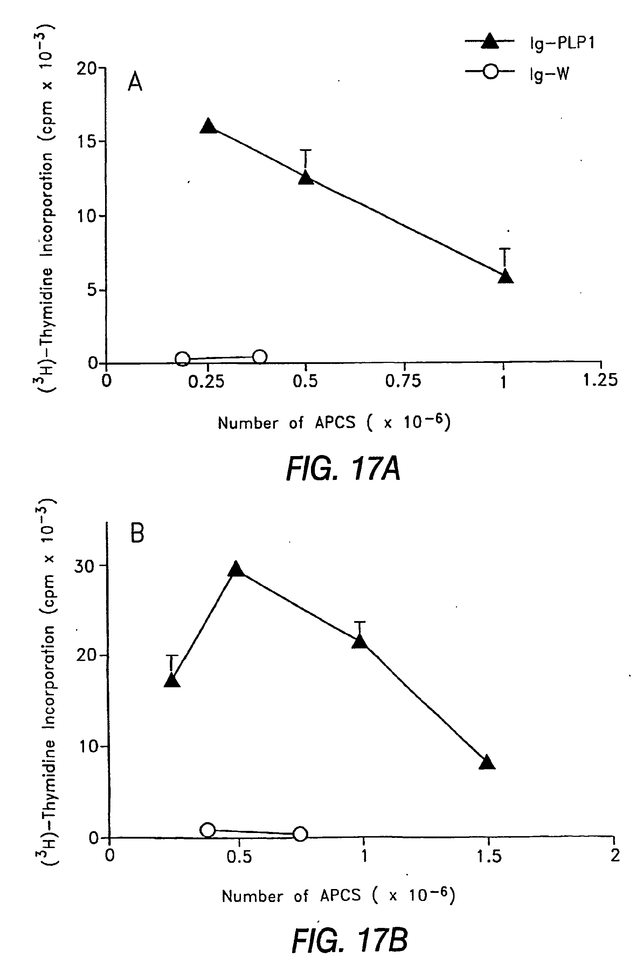

In order to confirm the clinical results observed in Example XIX, cytokine responses were measured in neonatal mice. The data obtained is shown in Fig. 17.

Specifically, neonates (5 mice per group) were injected with 100 µg Ig-PLP1 or lg-W within 24 hours of birth. Two days later the mice were sacrificed, and pooled thymic (17A) and splenic (17B) cells were irradiated and used as APCs for stimulation of the PLP1-specific T cell hybridoma 4E3 as described above. IL-2 production in the supernatant which was used as a measure of T cell activation was determined using the IL-2 dependent HT-2 cell line as described by Kuchroo et al., *J. lmmunol.,* 153:3326, 1994. The indicated cpms represent the mean ± SD of triplicates.

The administered Ig-PLP1 was efficiently presented by neonatal APCs. Both thymic (17A) and splenic (17B) APCs from neonate recipients of IG-PLP1 activated a T cell hybridoma specific for PLP1 peptide without addition of exogenous antigen. APCs from neonate recipients of Ig-W were unable to activate the T cell hybridoma.

### Example XXI

### Reduced Splenic Proliferative T cell Response in Mice Recipient of Ig-PLP1 at Birth

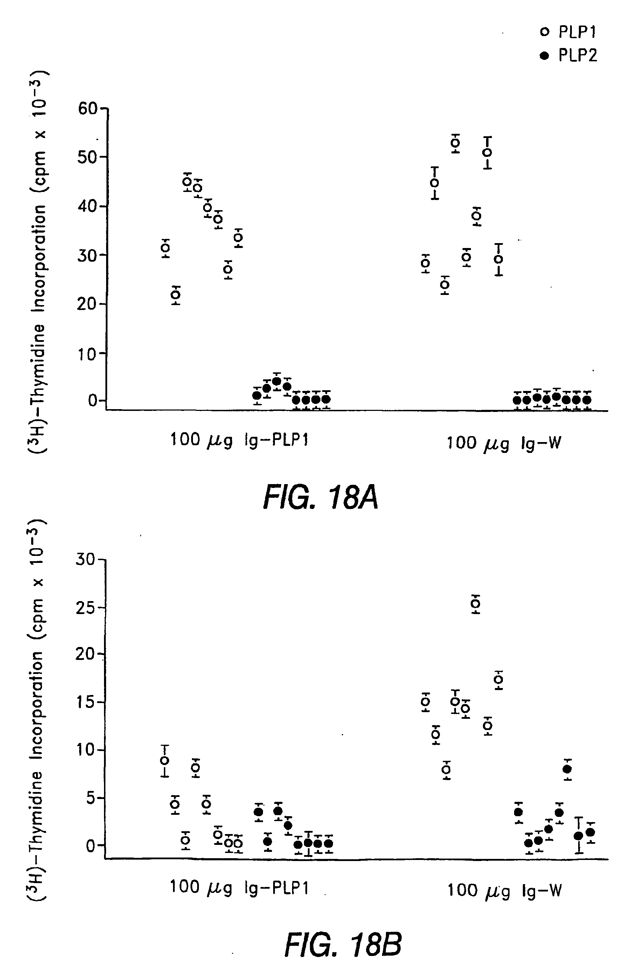

To further confirm the results observed in the previous two Examples, proliferative responses were measured in mice inoculated with an immunomodulating agent at birth. The results are shown in Figs.18A and 18B.

Neonates were injected intraperitoneal (i.p.)within 24 hours of birth with 100 µg lg-PLPI or Ig-W in saline. When the mice reached 7 weeks of age they were immunized with 100 µg free PLP1 peptide in 200 µl CFA/PBS (1vol/1vol) s.c. in the foot pads and at the base of the limbs and tail. Ten days later the mice were sacrificed, and (18A) the lymph node ( 0.4 x 10⁶ cells/well) and (18B) the splenic (1 X 10⁶ cells/well) cells were in vitro stimulated for four days with 15 µg/ml free PLP1 or PLP2, a negative control peptide corresponding the encephalitogenic sequence 178-191 of PLP (13). One µCi/well of[³H]thymidine was added during the last 14.5 hours of stimulation, and proliferation was measured using an Inotech γ-counter and the trace 96 Inotech program. The indicated cpms represent the mean ± SD of triplicate wells for individually tested mice. The mean cpm ± SD of lymph node proliferative response of all mice recipient of Ig-PLP1 and Ig-W was 34,812 ± 7,508 and 37,026 ± 10,133, respectively. The mean splenic proliferative response was 3,300 ± 3,400 for the Ig-PLP1 recipient group and 14,892 ± 4,769 for the lg-W recipient group.

Mice recipient of Ig-PLP1 at the day of birth, like those injected with lg-W, developed equivalent adult lymph node T cell proliferative responses to PLP1 when they were immunized with free PLPI peptide in CFA (18A). However, the splenic proliferative response was markedly reduced in the mice recipient of Ig-PLP1 (18B) thus indicating the inducement of tolerance. Neither group of mice showed a significant proliferative response to PLP2, a negative control peptide presented by I.A^{s} class II molecules like PLP1.

### Example XXII

### Lymph Node T Cell Deviation in Mice Treated With Ig-PLP1 at Birth

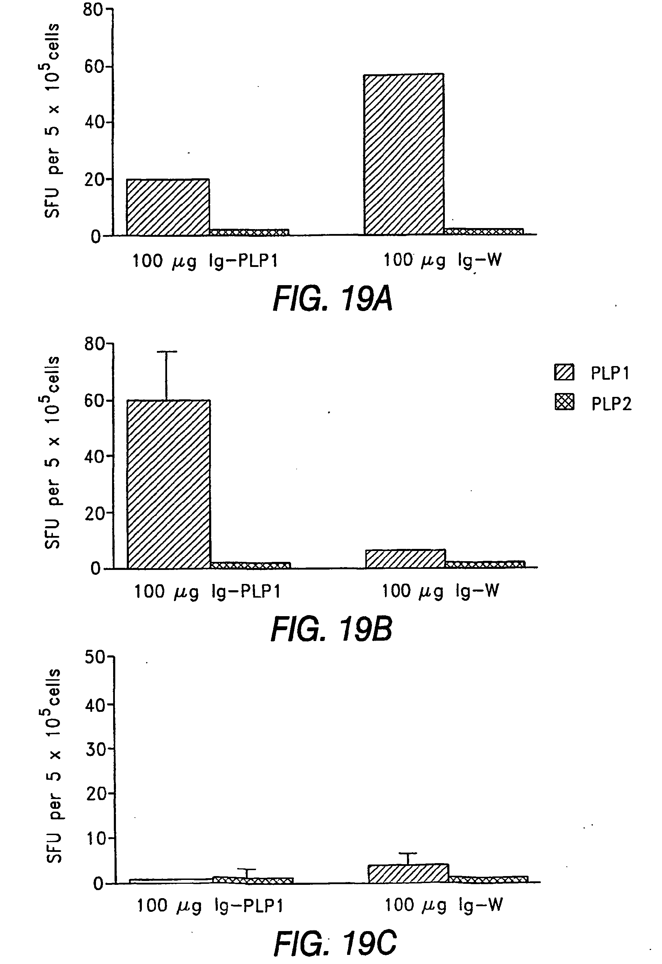

To further demonstrate the induction of tolerance in infants or neonates, cytokine responses were measured in were measured in mice inoculated with an immunomodulating agent at birth. The results are shown in Figs. 19A-19C.

In particular, lymph node cells (4 x 10⁵ cells/well) from the mice described in Example XXI were stimulated *in vitro* with free PLP1 or PLP2 (15 µg/ml) for 24 hours, and the production of IL-2 (19A), IL-4 (19B), and IFNγ (19C) was measured by ELISPOT as described in Example XVI using Pharmingen anti-cytokine antibody pairs. The indicated values (spot forming units) represent the mean ± SD of 8 individually tested mice.

The results show cytokine production patterns were affected by the inoculation of the neonatal mice. Lymph node cells from mice recipient of lg-W at birth produced, upon stimulation with PLP1, IL-2 but not IFNγ or IL-4. In contrast, cells from mice recipient of lg-PLP1 were deviated and instead produced IL-4. No cytokine production was observed upon stimulation with PLP2 peptide.

### Example XXIII

### Reduced IFNγ Production by Splenic T Cells From Mice Injected With lg-PLP1 at the Day of Birth

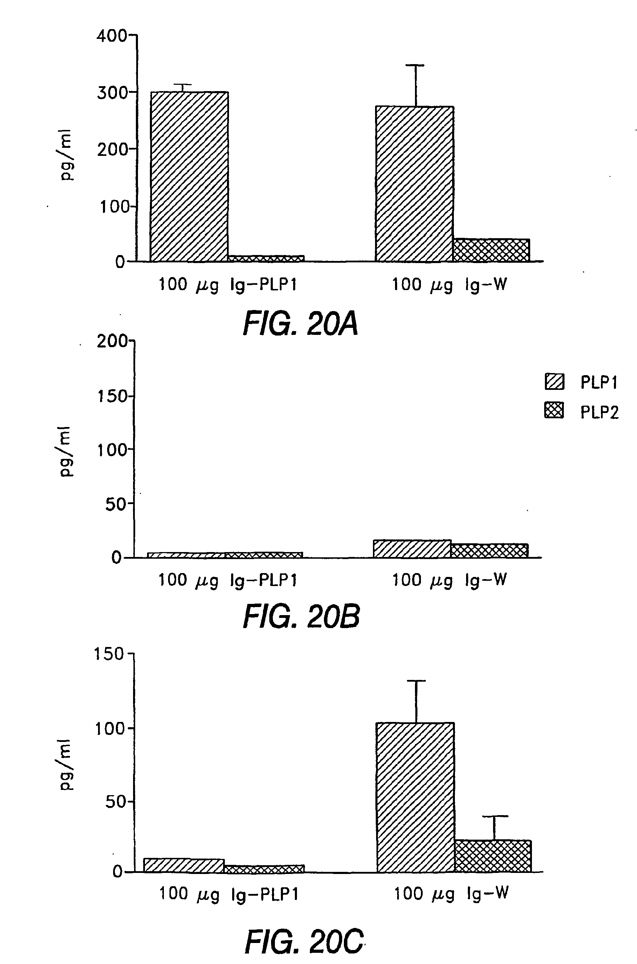

To confirm the results obtained in Example XXII, spleen cells from the same mice were assayed for cytokine responses. The results are shown in Figs. 20A and 20B.

More specifically, splenic cells (1 X 10⁶ cells/well) from the mice were stimulated *in vitro* with free PLP1 or PLP2 (15 µg/mi) for 24 hours, and the production of IL-2 (20A), IL-4 (20B), and IFNγ (20C) in the supernatant was measured by ELISA using pairs of anti-cytokine antibodies from Pharmingen according to the manufacture's instructions (Example XVI). The indicated amounts of cytokine represent the mean ± SD of 8 individually tested mice.

In the spleen, while cells from mice inoculate with Ig-W produced IL-2 and IFNγ. Conversely, cells from mice injected with Ig-PLP1 produced IL-2 but failed to produce detectable levels of IFNγ. The negative control, PLP2 peptide, failed to induce cytokine production.

### Example XXIV

### Cytokine Mediated Restoration of Splenic T Cell Proliferation in Mice Injected With Ig-PLP1 at Birth

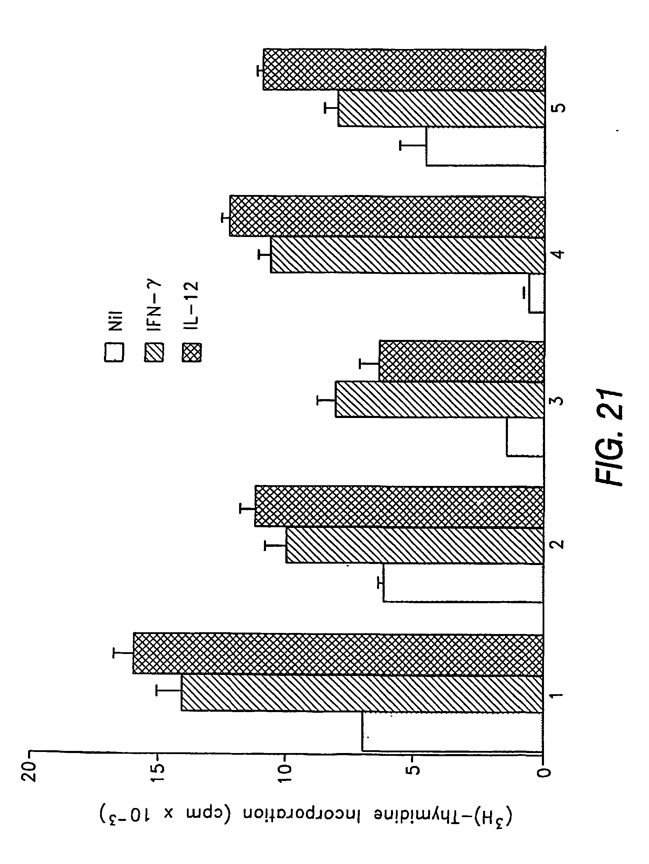

To demonstrate that proliferative responses may be restored, cells from inoculated neonatal mice were exposed to exogenous IFNγ. The results are shown in Fig. 21.

In particular, a group of neonates injected i.p. with 100 µg of Ig-PLP1 at birth were immunized with 100 µg PLP1 peptide in CFA, as in Example XXI, and *in vitro* stimulation of splenic cells (1 x 10⁸ cells/well) with free PLP1 peptide (15 µg/ml) was carried out as described in Example XXI but in the presence of 100 units IFNγ or IL- 12. The indicated cpms for each mouse represent the mean ± SD of triplicate wells.

Surprisingly, addition of erogenous IFNγ to splenic cells from the mice recipient of Ig-PLP1 at birth restored the proliferative response. IL-12, an inducer of IFNγ (14), also restored the splenic proliferative response.

Overall, mice injected at birth with Ig-PLP1 develop a lymph node T cell deviation and an unusual IFNγ-mediated splenic anergy. Interestingly, when these mice were induced for EAE with free PLP1 peptide they developed a mild monophasic disease without relapses. Since Igs have long half-lives, an Ig based immunomodulating agent may endure for an extended period of time resulting in a continuous and slow release of the immunosuppressive factor, as may occur in the usual neonatal tolerization procedures using incomplete Freund's adjuvant with a conventional antigen. Consequently, delivery on Igs may allow one to circumvent the use of adjuvant to induce neonatal tolerance. Further, internalization of an immunosuppressive factor via FcR and the subsequent processing in the endocytic pathway grants access to newly synthesized MHC class II molecules, generating significant amounts of MHC-immunosuppressive factor complexes. These favorable parameters (i.e. FcR-mediated APCs activation, slow peptide release, and efficient peptide presentation), may contribute to the induction of lymph node deviation and splenic anergy. As with administration of the disclosed compositions to adults, the adjuvant free tolerization strategy may be used to silence autoreactive T cells and prevent autoimmunity.

### Example XXV

### Soluble Ig-PLP1 Reduces Paralytic Severity and Suppresses Clinical Relapses in Mice with Ongoing EAE

SJUJ mice were induced for EAE with free PLP1 peptide, and when the clinical signs of disease became apparent the animals were given 3 injections of soluble lg-PLP1 (sol Ig-PLP1) in saline at 4 day intervals and assessed for reduction in disease severity. Control mice were given soluble Ig-W (sol Ig-W), the parental Ig without any PLP1 peptide. Groups of 6-8 wk old SJUJ mice were induced for EAE with 100µg PLP1 peptide, and then treated i.p. with 500µg sol lg-PLP1, 500µg sol lg-W, or 100µg free PLP1 peptide in PBS on days 9, 13, and 17 post disease induction. On the basis of 150 kD mol wt for Ig-PLP1 and 1.5 kD for free PLP1, the 300 µg free PLP1 given during the three injections correspond to ≈ 200 nmoles of PLP1 peptide, and the 1500 µg sol Ig-PLP1 encompasses ≈ 20 nmoles PLP1 peptide. Therefore, when free PLP1 is used for treatment the mice were given ≈10-fold more peptide than for treatment with sol Ig-PLP1.

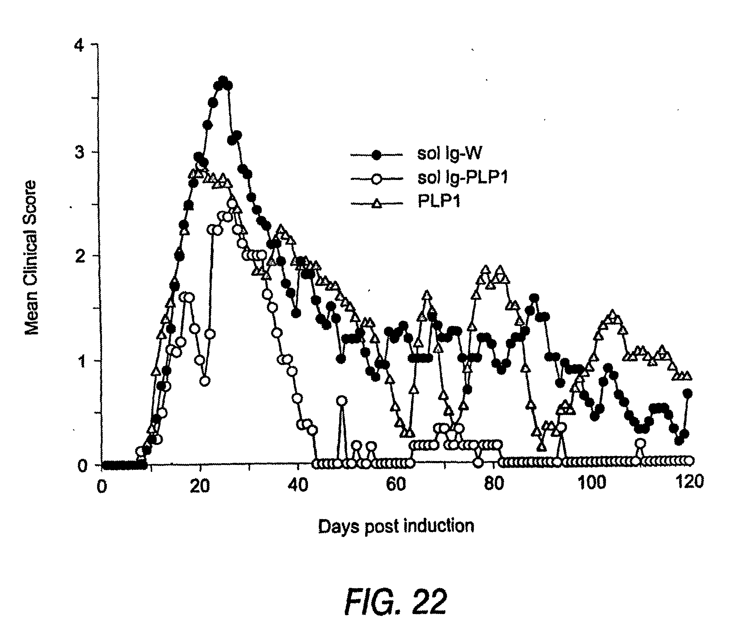

The results are illustrated in Figure 22. Each point represents the mean clinical score of 8 mice. The results presented in Figure 22 are representative of 2 independent experiments.

As shown in Figure 22, mice treated with the sol Ig-W had an initial severe phase of paralysis with a mean maximal score of 3.7 ± 0.5 and displayed relapses throughout the 120 day period of examination. The mice treated with sol Ig-PLP1, however, had a reduced severity of paralysis at the initial phase of disease with a mean maximal score of 2.5 ± 0.3 (p< 0.005) and fully recovered by day 42. Mice treated with 10-fold excess of free PLP1 peptide had a slight reduction in the severity of paralysis at the initial phase of disease (mean maximal clinical score 3.0 ± 0.2) but never recovered and underwent relapses throughout the entire 120-day observation period (Figure 22).

As illustrated in Figure 22, the efficacy of peptide delivery by Igs seems to extend to peripheral APCs expressing minimal or no costimulatory molecules since injection of the Ig-PLP1 chimera without adjuvant into diseased mice modulates PLP1-specific pathogenic T cells and ameliorates EAE (Figure 22). This conclusion is supported by the finding that 200 nmoles of PLP1 in the form of free peptide reduced the severity of disease only slightly and the animals never recovered, but 20 nmoles of peptide in the form of sol lg-PLP1 reduced the severity of the initial phase of disease and most of the animals fully recovered by day 42 (Figure 22).

### Example XXVI

### Administration of Soluble Immunoglobulins Comprising Agonists or Antagonists Without Adjuvant Reduces Disease Severity and Provides Peptide Presentation Without Costimulation

Groups of SJL mice were induced for EAE with PLP1 peptide and when the disease became clinically evident, the animals were given 3 injections of soluble lg-PLP1 or soluble Ig-PLP-LR in saline at 4 day intervals and assessed for reduction in disease severity. For control purposes a group of mice that was given lg-W, the parental lg that does not contain any myelin peptide was included.

Groups of SJL/J mice were induced for EAE by subcutaneous injection of 100 g of PLP1 peptide in PBS/IFA (vol/vol) containing 200 g *Mycobacterium tuberculosis* H37Ra. Six and 48 hours after injection, 5 x 10⁹ *Bordetella pertussis* were given intravenously and the mice were scored daily for signs of paralysis as follows: 0, no clinical signs; 1, loss of tail tone; 2, hindlimb weakness; 3, hindlimb paralysis; 4, forelimb paralysis; and 5, moribund or death. On days 9, 13, and 17 following disease induction, mice were treated intraperitoneally with 500 g sol Ig-PLP1, Ig-PLP-LR, or Ig-W in 500 I PBS.

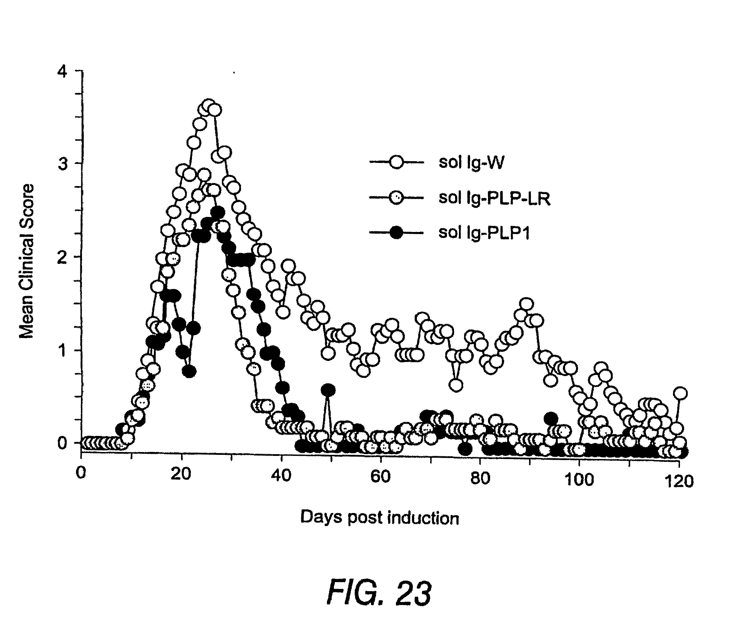

The results presented in Figure 23 show that both lg-PLP1 and Ig-PLP-LR treated mice had reduced clinical severity during the initial peak of disease and exhibited a drop in the mean maximal score from 3.7 ± 0.5 for Ig-W treated mice to 2.9 ± 0.2 and 2.4 ± 0.3 for Ig-PLP-LR and lg-PLP1 treated mice, respectively (Table 3). Interestingly while the mice given Ig-W showed relapses throughout the whole 120 days of observation, those treated with lg-PLP1 and Ig-PLP-LR recovered from paralysis by day 31 and 38 respectively and showed no relapses. Mice that were treated with 10 fold excess of free PLP1 or PLP-LR peptides had little reduction in clinical severity and continued to relapse throughout the 120 day observation period (data not shown).

**Table 3. Characteristics of clinical disease following treatment with sol Ig chimeras**

| Treatment | Incidence | Day of Onset* | Mean Maximum Disease Severity" | Day of Recovery*** |
|---|---|---|---|---|
| sol Ig-W | 10/10 | 9.8± 0.6 | 3.7± 0.5 | > 120 |
| sol Ig-PLP-LR | 10/10 | 9.9± 0.6 | 2.9± 0.2 | 38.8± 4.6 |
| sol Ig-PLP1 | 10/10 | 9.1±0.9 | 2.4± 0.3 | 31.0± 10.1 |

| | | | | |
|---|---|---|---|---|
| * Mean ± SC of the day of disease onset ** Mean ± SD of the maximal clinical scores *** Mice were considered recovered when their clinical score was < 0.5 for at least 5 days | | | | |

As the Ig-chimeras were administered to the mice without adjuvant, up-regulation of costimulatory molecules on the peripheral APCs may not have occurred leading to peptide presentation by cells lacking or having a reduced level of costimulatory molecules, thereby leading to tolerization of the autoreactive T cells.

To investigate this issue the expression of key costimulatory molecules on the surface of APCs was assessed upon incubation with soluble Ig-chimeras. Macrophages were harvested from the peritoneal cells of mice five days after injection with 2ml of thioglycolate broth by washing the peritoneal cavity with 8 ml of HBSS 40M EDTA. Macrophages (1.0 x 10⁶ cells/ml) were subsequently incubated with 0.3 OM soluble Ig-PLP chimera (black line) or media alone (NIL, grey). After 24 hours the cells were harvested and stained with anti-F4/80 (HB-198, ATCC), and either anti-B7.1 (1G10; CRL-2223, ATCC), anti-B7.2 (2010; CRL-2226, ATCC), or anti-CD40. Histograms represent F4/80⁺ gated cells and show the intensity of either B7.1, B7.2, or CD40.

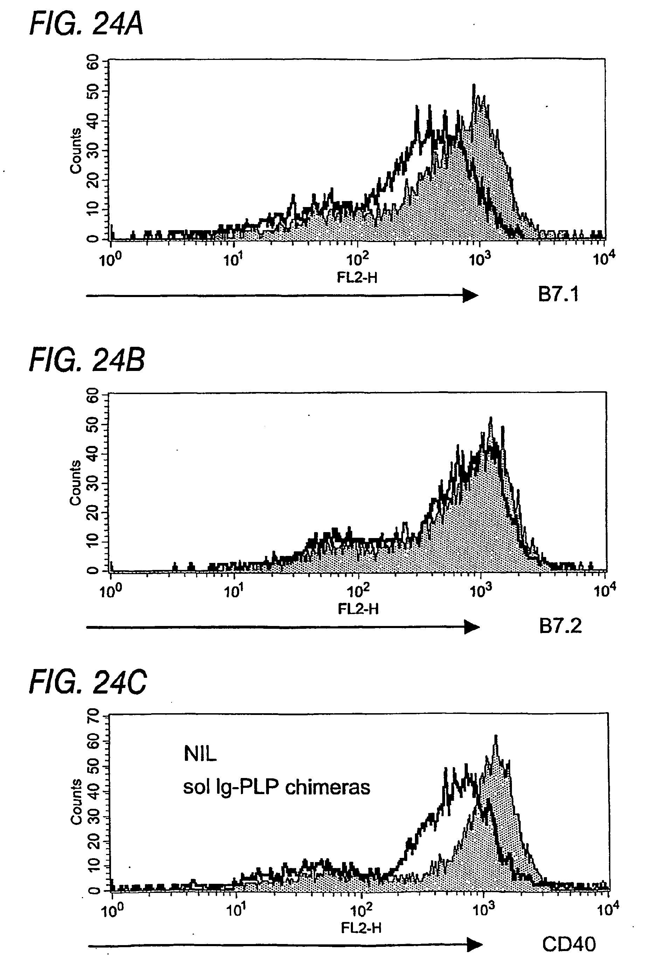

The results presented in Figure 24 show that peritoneal macrophages cultured in the presence of soluble Ig-chimera for 24 hours had similar levels of B7.2 as those cultured without Ig-chimeras. However, B7.1 and CD40 expression were decreased relative to the basal level of expression seen with the cells cultured in media without sol lg-chimeras. Thus, treatment with soluble immunoglobulins containing agonists or antagonists drives peptide presentation without costimulation, thereby simulating natural peripheral tolerance to modulate autoreactive T cells.

### Example XXVII

### Amelioration of EAE bv Aggregated Ig-PLP1

To further delineate the clinical advantages associated with the compositions and methods of the present invention, EAE mice were inoculated with aggregated Ig-PLP1. The results are shown in Fig. 25.

EAE was induced in a group of 10 mice with 100 µg of free PLP1 peptide as described above. Soluble aggregated Ig-PLP1 was prepared by heating a solution of Ig-PLP1 for 15 minutes at 63°C and then centrifuging and filtering the resulting preparation to remove any insoluble aggregates that were formed during the process. The concentration of solubilized aggregates was then quantified using standard biochemical techniques.

When the clinical signs of EAE started to develop at day 10 post disease induction, the mice were injected with a saline solution containing 300 µg of the heat aggregated Ig-PLP1. A second and third injection of 300 µg of aggregated lg-PLP1 were given at days 14 and 17, respectively. Control treatments using aggregated lg-W and soluble (unaggregated) Ig-PLP1 were run in parallel. The grading of the clinical condition was done as described in Example XIX. In Fig. 25, the mice treated with aggregated Ig-PLP1 are represented by dark circles while the controls are represented by light circles (soluble Ig-PLP1) and triangles (aggregated Ig-W).

The results clearly show that aggregated arrangements of the disclosed immunomodulating agents may be used to effectively reduce the symptoms associated with immune disorders.

### Example XXVIII

### Incubation of Aggregated Ig-PLP1 With Purified APCs Induces IL-6 and IL-10 Production

To demonstrate that the compositions of the present invention advantageously induce anti-inflammatory cytokines, antigen presenting cells were exposed to aggregated Ig-PLP1. The results are shown in Figs. 26A and 26B.

Three types of cells were tested for production of cytokines upon incubation with aggregated Ig-peptide constructs. These include B cells, macrophages/manocytes, and dendritic cells. To obtain macrophages, adult SJL/J mice were injected with thioglycolate and, at day 5 post injection, cells were harvested from the peritoneal cavity by extensive washing with ice cold sucrose (0.34 M ), and allowed to adhere to a plastic culture flask for 4 hours. Non-adherent cells were removed by vigorous pipetting. After an additional overnight culture, adherent cells were collected by a cell scraper. Dendritic cells were purified from the spleen and enriched using standard biochemical techniques. B cells were purified from the spleen by panning with a rat anti-kappa mab. For enrichment of resting B cells, macrophages, dendritic cells, and large (activated) B cells were removed using a sephadex 610-column. Subsequently, the eluted cells were depleted of T lymphocytes by treatment with an anti-Thy 1.2 antibody and complement. FACS analysis is then performed to ensure that only preparations that are enriched to 90% or higher are stimulated with the aggregated constructs.

The enriched macrophages were tested for the production of IL-6 and IL-10 by ELISA using anti-cytokine antibody pairs from Pharmingen (San Diego, CA). The macrophages were used at 100 x 10³ cells/well and the B cells and dendritic cells at 50 x 10³ cellslwell. The cells (triplicate wells) were incubated with graded amounts of aggregated Ig-PLP1 or a mouse myeloma IgM for 24 hours and the supernatant was used for measuring cytokine production. The amount of cytokine in the supernatant was estimated by extrapolation on a standard curve constructed with known amounts of cytokine.

Figs. 26A and 26B show that the administration of aggregated lg-PLP1 enhances the production of anti-inflammatory cytokines such as IL-6 and IL-10. More particularly, Fig. 26A shows that exposure to aggregated constructs induces relatively high levels of IL-6 in macrophages (squares) while Fig. 26B shows that the same constructs enhances production of IL-10 in macrophages (squares) and dendritic cells (triangles). It should be appreciated that the production of such cytokines can inhibit the expression of MHC class II molecules and the up-regulation of costimulatory molecules while favoring development of Th₂ type cells.

### Example XXIX

### Aggregated Ig.PLP1 Displays Higher Efficacy than Soluble Ig-PLP1 in Reversing Active EAE

Although soluble immunomodulating agents are capable of alleviating the symptoms of autoimmune disease and fall within the scope of the present invention, the ability of immunomodulating agents capable of crossling Fc receptors on target cells to further alleviate autoimmune disease was investigated as follows.

The ability of aggregated of Ig-PLP1 to cross-link FcRs and induce IL-10 production, thereby providing greater alleviation of autoimmune disease than soluble Ig-PLP1 was investigated as follows. Large-scale cultures of lg-W, Ig-PLP1, and lg-PLP2 transfectants were preformed in DMEM containing 10% serum supreme (BioWhittaker, Walkersville, MD) and purified on separate rat anti-mouse κ chain sepharose columns to avoid cross-contamination. Subsequently the lg-chimeras were dialyzed against PBS and concentrated on collodion membranes (Schleicher & Schuall, Keene, NH). The chimeras were aggregated by precipitation with 50%-saturated (NH₄)₂SO₄ as described (Chase et al., Chemical Analyses, In Methods in Immunology and lmmnochemistry, Williams et al., eds., Academic Press, New York, 2:249-341, 1968. Briefly, filtered 100% saturated (NH₄)₂SO₄ was added at an equal volume to the sol lg-chimera preparation. The mixture was incubated at 24 C for 1 h with gentle agitation every 20 min. Subsequently, the samples were spun down at 10,000 rpms and the pellet resuspended at 1mg/ml in PBS. Electrophoresis on a 10% acrylamide gel indicated that the sol lg-chimera entered the gel and migrated around 160 kD. However, the agg lg-chimera did not enter the gel. Knowing that we applied the equivalent of 2 g of agg lg-chimera and that the sensitivity of the technology is 0.1 g, we concluded that at least 95% of the agg Ig-chimera preparation is in an aggregate form.

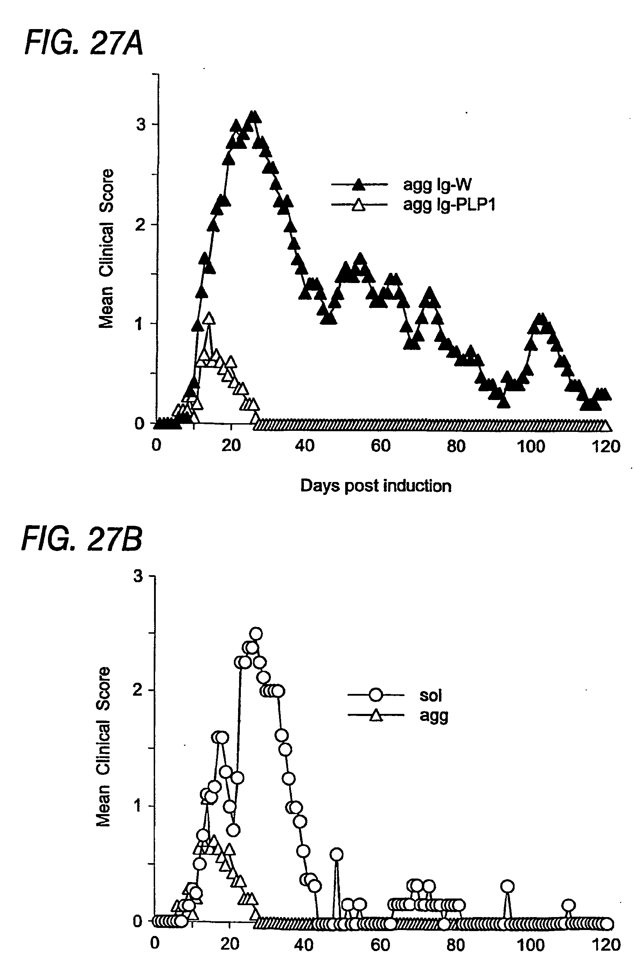

Groups of mice (8 per group) were induced for EAE with 100µg PLP1 and then treated with 300µg of agg lg-PLP1 or agg lg-W in PBS on days 9, 13, and 17 post disease induction. The results are shown in Figure 27a and Figure 27b.

As can be seen in Figure 27a, the initial phase of paralytic disease severity was reduced from a mean maximum score of 3.3 ± 0.3 in agg lg-W treated animals to 1.1 ± 0.5 (p < 0.001) in the agg lg-PLP1 recipient mice. In addition, the animals fully recovered within 9 days of completion of the treatment and never relapsed throughout the entire 120-day observation period while agg lg-W treated mice never recovered and showed relapses throughout the entire period of clinical assessment.

Figure 27b is a direct comparison of the disease course of PLP1 peptide induced EAE following treatment with sol Ig-PLP1 (from Figure 22) vs. agg lg-PLP1 (from figure 27a). Each point represents the mean clinical score of 8 mice. These results are representative of 3 independent experiments. As illustrated in Figure 27b, although the 900 µg agg Ig-PLP1 given to mice contains ≈12 nmoles PLP1 and is ≈ 17 fold lower than the 200 nmoles given as free PLP1, disease modulation by aggregated Ig-PLP1 was much more effective. The effectiveness of agg Ig-PLP1 is also apparent when the paralytic clinical signs of agg lg-PLP1 treated animals were compared to those of animals injected with sol lg-PLP1 (p < 0.001) (Figure 27b). Indeed, the mean maximum clinical score was much lower and the recovery faster.

A histologic examination of mice treated with agg lg-PLP1 or agg lg-W was also performed. Mice treated with agg Ig-PLP1 or agg lg-W were sacrificed at the peak of the initial phase of disease (day 28 post disease induction), and the brain and spinal cord were removed, fixed with formalin, and embedded in paraffin. Serial cross-sections (6µm) from the cerebellum, cerebrum, and lumbar cord were cut and stained with hematoxylin-eosin (H&E). Perivascular clusters containing at least 20 mononuclear cells were counted as an inflammatory focus.

Histologic examination of the cerebellum at the peak of disease indicated a lower number of foci and a reduced number of infiltrating mononuclear cells per foci in the mice treated with agg lg-PLP1 versus those given agg lg-W (data not shown). Moreover, when serial histologic cross sections were prepared from both the brain and spinal cord and the mean foci per cross section estimated, there was a two to three fold reduction in the number of foci in agg lg-PLP1 treated mice versus mice recipient of agg Ig-W (Table 4). Furthermore, the foci in agg lg-PLP1 treated mice had less infiltrating mononuclear cells than those of agg Ig-W treated mice (agg lg-W: 73 ±39, agg Ig-PLP1: 32 ± 14, p < 0.005).

**Table 4. Treatment with agg Ig-PLP1 ablates clinical and histologic EAE.**

| | Clinical EAE | Histologic EAE | |
|---|---|---|---|
| Treatment | Mean Maximum Severity | Cerebrum | Lumbar spinal cord |
| | | Foci/cross-section | |
| agg Ig-W | 3.3±0.3 | 11.7±2.1 | 18.5±3.3 |
| | (p<0.001) | (p<0.001) | (p<0.001) |
| agg Ig-PLP1 | 1.1±0.5 | 6.5±0.5 | 6.9±1.1 |

6-8 wk old mice were induced for EAE with PLP1 and then subsequently treated with 300µg agg Ig-PLP1 or agg Ig-W on days 9, 13, and 17 post disease induction and scored daily for clinical disease. The mean maximum severity was determined by averaging the maximal clinical score obtained from each mouse within a group. In order to determine histological disease, brains and spinal cords were removed from mice on day 28 post disease induction (peak of disease), fixed in formalin, paraffin embedded, serially cross-sectioned at 6µm, and then stained with hemotoxylin-eosin (H&E). An inflammatory foci represents a minimum of 20 mononucular cells/perivascular cluster.

### Example XXX

### Aggregated Ig-PLP1 Induces the Production of IL-10 by APCs

To delineate the mechanism underlying the effective modulation of EAE by agg Ig-PLP1, the ability of agg lg-PLP1 to stimulate the production of IL-10 by APCs and the ability of IL-10 to inhibit T cells engaged in the recognition of the PLP1 peptide presented by IL-10 producing APCs were investigated. To this end, naive splenocytes were incubated with sol or agg Ig chimeras, and the supernatants were used for IL-10 detection.

Irradiated (3000 rads) SJL/J splenocytes (5 x 10⁵cells/well) were incubated with graded amounts of sol Ig-PLP1, agg Ig-PLP1, agg Ig-W, sol Ig-PLP2, or agg Ig-PLP2 for 24 h, and the supernatant was used to quantitate IL-10 production by ELISA as follows.

ELISA was done according to PharMingen's standard protocol. The capture Ab was rat anti-mouse IL-10 and the biotinylated anti-cytokine Ab was rat anti-mouse IL-10. Bound ligand was revealed using the TMB microwell peroxidase substrate system (Kirkegaard & Perry Laboratories, Gaitherburg, MA). Assays were read on a SpectraMAX 340 counter. Graded amounts of recombinant mouse IL-10 were included in all experiments for construction of standard curves. The cytokine concentration in culture supernatants was estimated by extrapolation from the linear portion of the standard curve. Each point represents the mean of triplicate wells and the data are representative of 4 independent experiments.

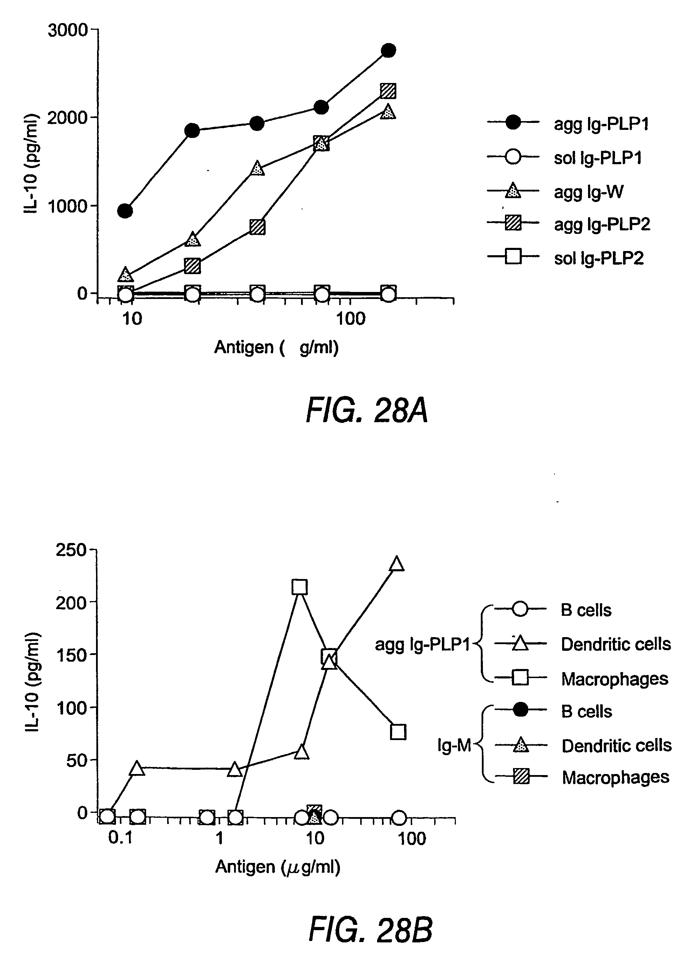

As indicated in Figure 28a, agg Ig-PLP1, Ig-PLP2, and Ig-W chimeras stimulated the production of IL-10 by splenic cells in a dose-dependent manner. The soluble forms of the chimeras did not induce detectable levels of IL-10.

To investigate whether cells known to function as professional APCs are able to produce IL-10 upon incubation with agg Ig chimeras the following experiments were performed. Thioglycolate-induced peritoneal macrophages and splenic B and dendritic cells were isolated and tested for IL-10 production upon incubation with agg Ig-PLP1 as follows.

Macrophages were obtained from the peritoneal cells of mice injected with thioglycolate broth as previously described (Doyle et al., Murine macrophages: Isolation, cultivation, and characterization, *In* Weirs Handbook of Experimental Immunology, Herzenberg et al., eds., Blackwell Science, Cambridge, MA, 154.1-154.8, 1996. Briefly, 2 ml of thioglycolate broth was injected i.p., and after 5 d the macrophages were removed by washing the peritoneal cavity with 8 ml of HBSS 4µM EDTA. Macrophage purity was ≥ 93% as determined by FACS® analysis using antibody to F4/80 marker.

Dendritic cells were purified from SJL/J spleen according to the standard collagenase/differential adherence method (Romani et al., Dendritic cells. *In* Weirs Handbook of Experimental Immunology, Herzenberg et al., eds., Blackwell Science, Cambridge, MA, 156.1-156.14, 1996. Cell purity was ≥ 94% as determined by FACS® analysis using antibody to the 33D1 marker.

SJL/J splenocytes were panned on plates coated with rat anti-mouse κ (1mg/ml) for 15 min at 25°C. Non-adherent cells were washed out with PBS. B cells were then dissociated from the plate by incubation with lidocaine HCI (0.8mg/ml) followed by vigorous pipeting. Cell purity was ≥ 90% as determined by FACS® analysis for expression of B220 marker.

Irradiated (3000 rads) B cells (2x10⁵cells/well), macrophages (0.2x 10⁵cells/well), and dendritic cells (0.2x 10⁵cellslwell) were incubated with graded amounts of agg lg-PLP1 (open symbols) or mouse IgM (closed symbols) for 24 h, and cell culture supernatant was used to measure IL-10 production. Each point represents the mean of triplicate wells. These data are representative of 4 independent experiments.

Figure 28b indicates that macrophages and dendritic cells, but not B cells, produce IL-10 upon incubation with agg Ig-PLP1. Mouse IgM was unable to stimulate IL-10 production by any of the APCs tested. These results indicate that agg Ig-PLP1 crosslinks FcγR and induces the production of IL-10 by APCs. Furthermore, pre-incubation of APCs with soluble mouse IgG inhibited agg Ig-PLP1-induced IL-10 production (data not shown).

These results indicate that the IL-10 produced by the APCs was due to crosslinking of FcγR rather than to contamination with endotoxin.

### Example XXXI

### Aggregated Ig-PLP1 Induces IL-10 by Crosslinking FcγR1 Receptors

The ability of agg Ig-PLP1 to crosslink Fc receptors was investigated as follows. Ig-PLP1 was aggregated using ammonium sulfate and tested for induction of IL-10. SJUJ splenocytes (0.5 x 10⁸ cells/well) were incubated with 0.1 M sol Ig-PLP1, 0.1 M agg Ig-PLP1, 0.1 M agg Ig-PLP1 + 50 g/ml 2.4G2, or 0.1 M agg Ig-PLP1 + 100 g/ml mouse Ig. After 24 hours the cells were pelleted and 100 ml of culture supernatant was used to assess IL-10 production by ELISA according to PharMingen's standard protocol.

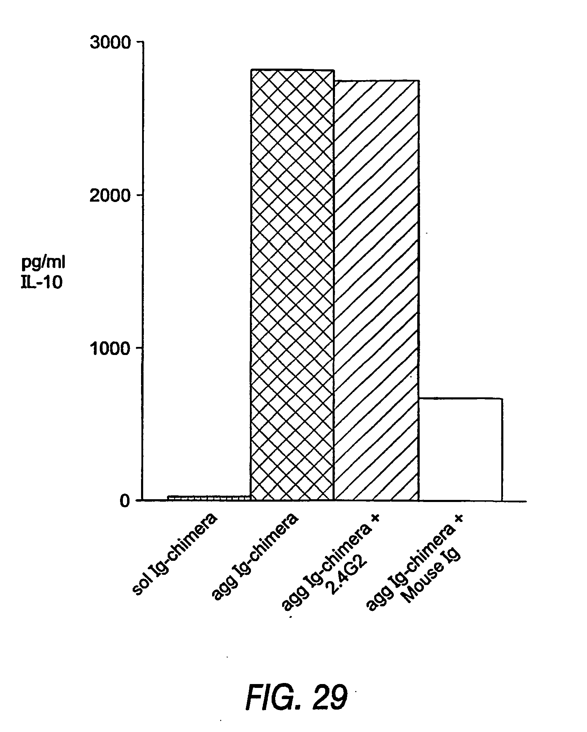

As can be seen in Figure 29, incubation of splenocytes with agg lg-chimeras, but not sol lg-chimeras, led to the induction of IL-10 by the APCs. IL-10 production appeared to be Fc R1 dependent as blocking of Fc R2 and Fc R3 with 2.462 mAb did not inhibit IL-10 production, while blockade of all three Fc Rs by incubation with mouse IgG did significantly decrease agg lg-chimera induced IL-10. Overall these results suggested that the aggregation of the Ig-chimeras led the APCs to produce IL-10 in an Fc R1-dependent manner.

### Example XXXII

### Aggregated Ig-PLP1 Down Regulates IFNγ Secretion by Specific T Cells In Vitro

To investigate the effect that APC-derived IL-10 might have on T cells specifically engaged with the APCs through antigen presentation, a PLP1-specific Th0 clone able to produce both type I and type II cytokines upon peptide stimulation was used. This clone, designated TCC-PLP1-1B10 was prepared as follows.

Adult SJL mice were immunized subcutaneously with 100µg PLP1 peptide in CFA, and 10 days later the draining lymph nodes were removed and the cells (5 x 10⁶ cells/ml) were stimulated with PLP1 (15µg/ml). After 5 days the blasts were separated on a Histopaque gradient (Sigma, St. Louis, M0), and then restimulated with peptide and fresh irradiated (3000 rad) syngenic APCs. Ten days later, the cells were washed, resuspended in media containing 10% T-Stim (Collaborative Research, Boston, MA) and rested for 7 days. After three cycles of stimulation/resting, the cells were cloned by limiting dilution (1 cell/3 wells) and positives were subjected to a second round of limiting dilution cloning. Subsequently, one clone, designated TCC-PLP1-1B10, was further characterized as follows.

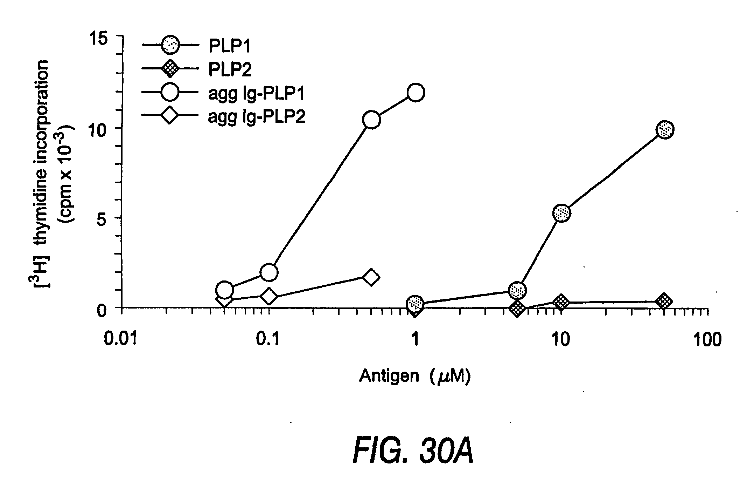

The proliferative response of TCC-PLP1-1B10 to PLP1, PLP2, agg Ig-PLP1 and agg Ig-PLP2 was examined as follows. SJL/J splenocytes (10 x 10⁵cells/well/100µl) were pulsed with graded amounts of antigen on round-bottom 96-well plates for 4 hours, pelleted, fixed with 1% paraformaldehyde for 15 minutes washed, and transferred to a fresh 96-well plate. TCC-PLP1-1B10 cells (0.5 x 10⁵cells/well/100µl) were then added and incubated for 3 days. Subsequently, 1µCi [³H] thymidine was added per well, and the incubation continued for an additional 14.5 hours. The cells were then harvested on glass fiber filters, and incorporated [³H] thymidine was counted using an Inotech β counter (Wohlen, Switzerland). The results are shown in Figure 30a.

As illustrated in Figure 30a, TCC-PLP1-1 B10 proliferates upon incubation with paraformaldehyde-fixed splenic APCs that were previously pulsed with free PLP1 peptide or agg Ig-PLP1. TCC-PLP1-1 B10 did not show significant proliferation when the APCs were pulsed with the negative control PLP2 or agg Ig-PLP2.

The cytokines produced by TCC-PLP1-1B10 upon stimulation with free PLP1 or agg Ig-PLP1 were investigated as follows. Irradiated (3000 rads) SJL/J splenocytes (5 x 10⁵cells/well) were incubated with graded amounts of PLP1 peptide (closed circles) or agg lg-PLP1 (open circles) for 1 hour after which TCC-PLP1-1B10 cells (0.5x 10⁵cells/well) were added and the incubation continued for an additional 24 h. Cytokine production was measured by ELISA from 100µl of culture supernatant as follows. Each point represents the mean of triplicate wells. ELISA was done according to PharMingen's standard protocol. The capture Abs were rat anti-mouse IL-2, JES6-1A12; rat anti-mouse IL-4, 11B11; rat anti-mouse IFNγ, R4-6A2; rat anti-mouse IL-10, JES5-2A5; and rat anti-mouse IL-5, TRFK5. The biotinylated anti-cytokine Abs were rat anti-mouse IL-2, JES6-5H4; rat anti-mouse IL-4, BVD6-24G2; rat anti-mouse IFNγ, XMG1.2; rat anti-mouse IL-10, JES5-16E3; and rat anti-mouse IL-5, TRFK4. ELISA for the detection of active TGF was preformed using the human TGF₁ DuoSet kit (Genzyme, Cambridge, MA) according to the manufacture's instructions. Bound ligand was revealed using the TMB microwell peroxidase substrate system (Kirkegaard & Perry Laboratories, Gaitherburg, MA). Assays were read on a SpectraMAX 340 counter. Graded amounts of recombinant mouse IL-2, IL-4, IFNγ, IL-10, IL-5, and TGF were included in all experiments for construction of standard curves. The cytokine concentration in culture supernatants was estimated by extrapolation from the linear portion of the standard curve.

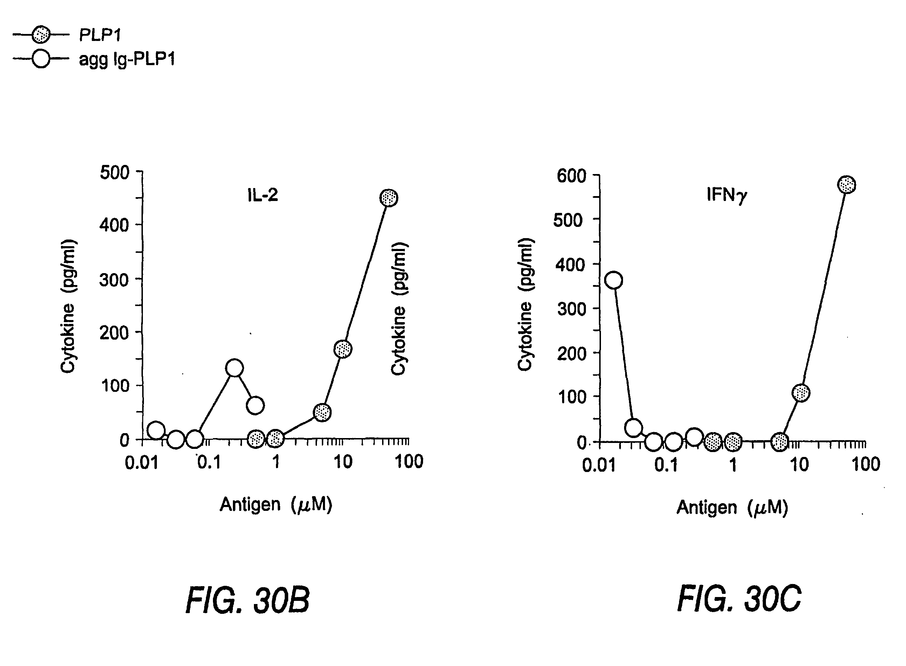

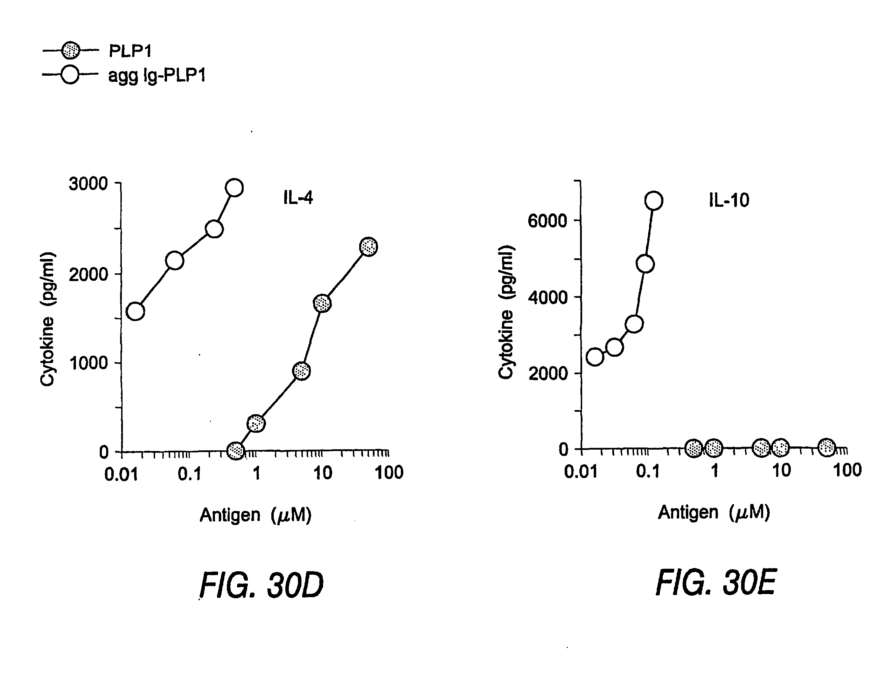

When tested for cytokine production upon incubation with non-fixed splenic APCs and free PLP1 peptide, TCC-PLP1-1B10 produced significant amounts of IL-2, IL-4, and IFNγ (Figure 30b, 30c, and 30d). All three cytokines were also detected when agg Ig-PLP1 was used for stimulation (Figure 30b, 30c, and 30d). However, IL-10 was detectable at significant levels when the stimulator was agg Ig-PLP1 but not free PLP1 (Figure 30e).

Since agg Ig-PLP1 induces IL-10 production by macrophages and dendritic cells, it is likely that the IL-10 seen in the T cell cytokine assessment assay was the product of splenic APCs rather than TCC-PLP1-1B10. To confirm this the following experiments were performed. Fixed and live APCs were used to identify the source of IL-10 in T cell activation by agg Ig-PLP1. In the fixed APCs assay, SJL/J splenocytes (10 x 10⁵cells/well) were pulsed with graded amounts of agg Ig-PLP1 for 4 h, washed extensively, and fixed with paraformaldehyde. In the live APCs assay, irradiated (3000 rads) SJL/J splenocytes (5 x 10⁵cells/well) were mixed with graded amounts of agg Ig-PLP1 and incubated for 1 h. Subsequently, TCC-PLP1-1B10 cells (0.5x 10⁵cells/well) were added to both assays and the incubation continued for an additional 24 h. IL-10 production was measured by ELISA from 100µl of culture supernatant. Each point represents the mean of triplicate wells.

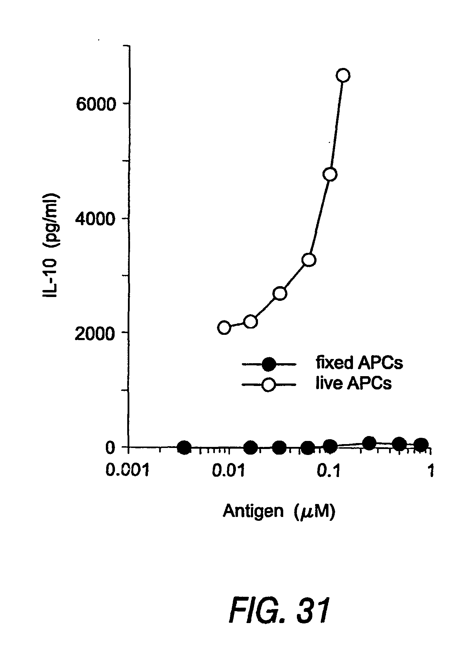

The results are shown in Figure 31, As illustrated in Figure 31, IL-10 was undetectable when APCs, pulsed with agg Ig-PLP1, were washed and fixed with paraformaldehyde prior to incubation with TCC-PLP1-1B10, demonstrating that splenic APCs rather than TCC-PLP1-1B10 were the source of IL-10.

The other striking observation from the T cell cytokine assessment assay was that the production of IFNγ seemed to be decreased as IL-10 production by APCs increased (Figure 30c and 30e). To investigate this issue further, an extended range of Ig-PLP1 concentrations were used for stimulation of bulk and purified APCs, and IL-10 and IFNγ production were assessed simultaneously from the same tissue culture well as follows.

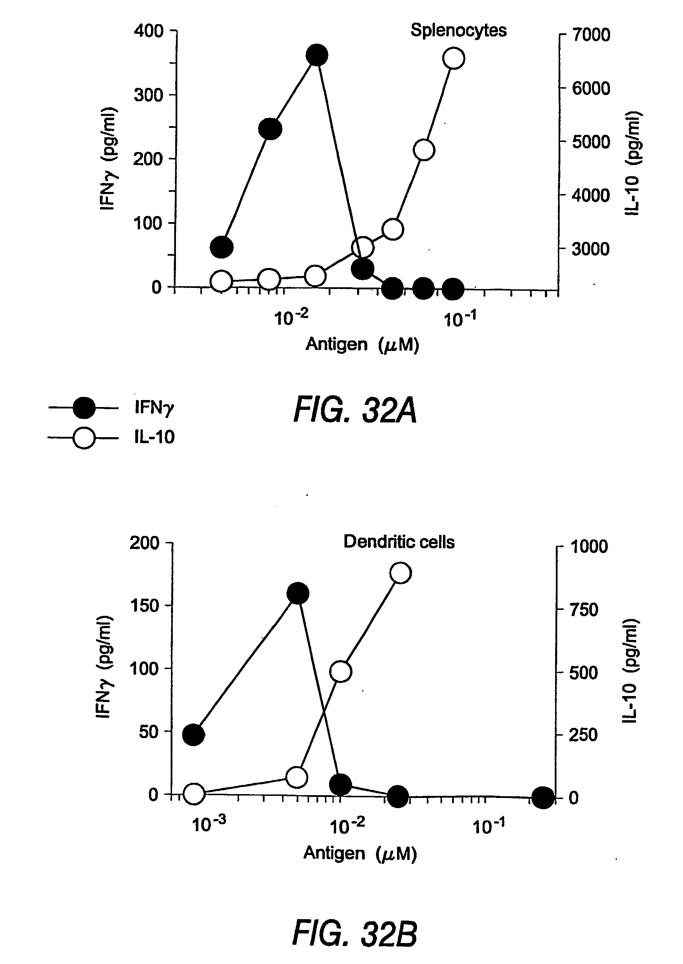

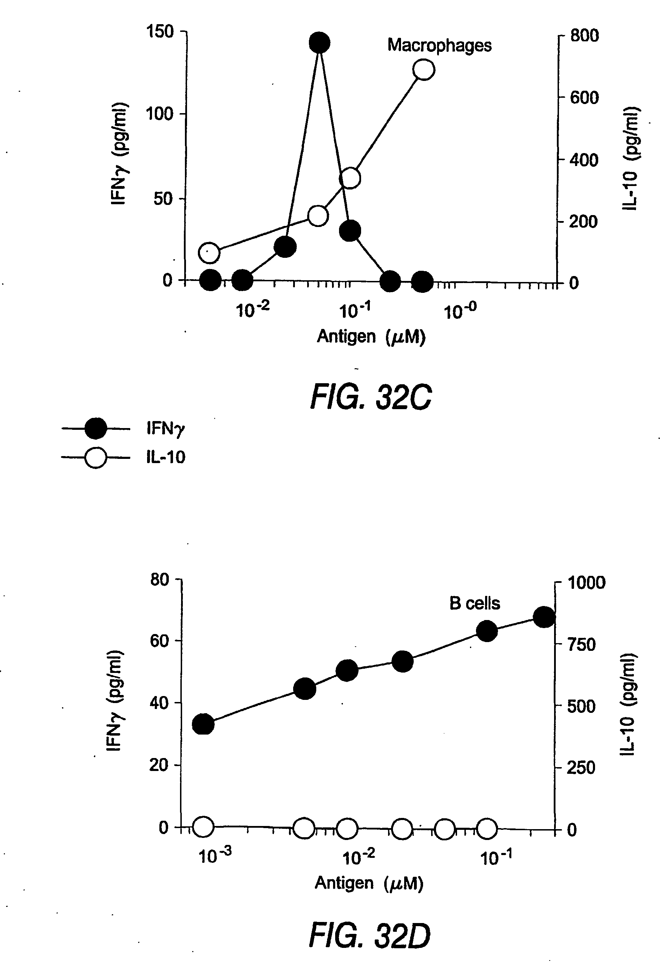

Irradiated (3000 rads) SJL/J splenocytes (5 x 10⁵ cells/well), dendritic cells (0.2 x10⁵ cells/well), macrophages (0.2 x 10⁵ cells/well), or B cells (2 x 10⁵ cells/well) were incubated with graded amounts of agg Ig-PLP1 and after 1 h TCC-PLP1-1B10 cells (0.5x 10⁵cells/well) were added and the incubation was continued for an additional 24 hours. IFNγ and IL-10 production in the same culture well was measured by ELISA. Each point represents the mean of triplicate wells. The results are shown in Figure 32.

As illustrated in Figure 32, the IL-10 secreted by the APCs antagonizes the production of IFNγ by the T cells. Indeed, when the stimulation assay was preformed using splenocytes, purified DCs, or enriched peritoneal macrophages as APCs (all of which produce IL-10 upon incubation with agg Ig-PLP1, Figure 28), IFNγ production by the T cells decreased dramatically and became undetectable as the production of IL-10 by APCs increased (Figure 32a, 32b, and 32c). However, when B cells were used as APCs, which do not produce IL-10 upon incubation with agg Ig-PLP1 (Figure 28b), the secretion of IFNγ by T cells was not affected (Figure 32d). Overall, these results indicate that agg lg-PLP1 triggers IL-10 production by the presenting APCs (dendritic cells and macrophages) and that such IL-10 antagonizes the production of IFNγ by the T cells.

To further confirm that IL-10 production antagonizes production of IFNγ by T cells, the following experiment was performed. Irradiated (3000 rads) SJUJ peritoneal macrophages (0.2 x 10⁵ cells/well) (purified as described above) were incubated with graded amounts of agg Ig-PLP1 for one hour and then TCC-PLP1 1B10 (0.5 x 10⁵ cells/well) was added and the incubation continued for an additional 24 hours. IFN and IL-10 production in the same well were assessed from 100 I of culture supernatant as described above. In addition, anti-IL-10 mAb 2A5 or rat IgG were included in some cultures to determine their effects on IFNγ production.

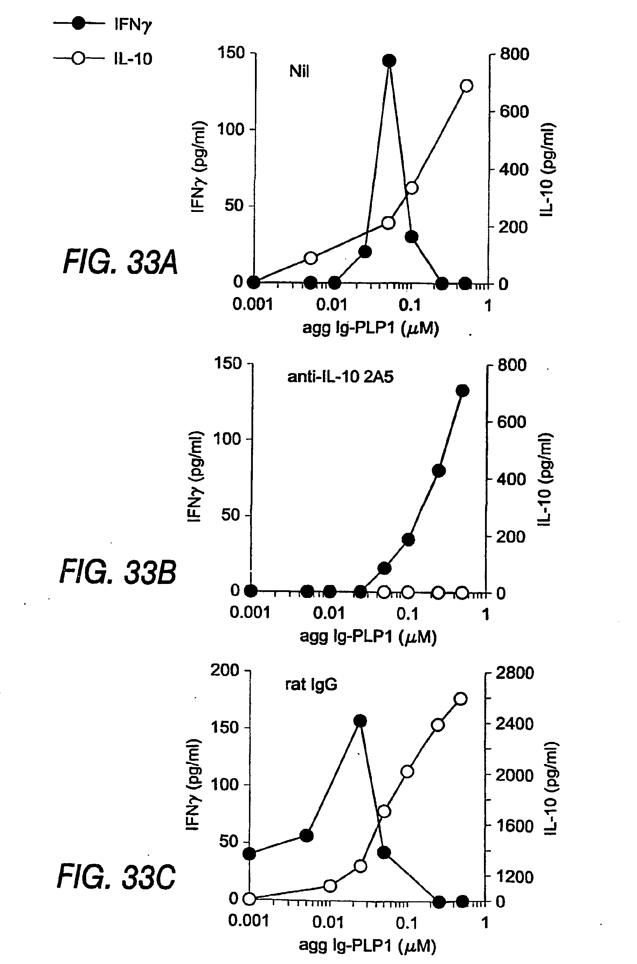

The results are shown in Figure 33. When TCC-PLP1 1B10 was incubated with peritoneal macrophages and agg Ig-PLP1, there was a decrease in IFNγ production proportional to the level of IL-10 secreted by the presenting macrophages (Figure 33a). Furthermore, the inhibition of IFNγ production by the T cells was directly related to APC derived IL-10 as neutralization of such IL-10 by anti-IL-10 mAb, 2A5 restored IFN production (Figure 33b). Incubation with isotype control rat IgG instead of anti-IL-10 had no effect on IL-10's ability to inhibit IFN production by TCC-PLP1 1B10 (Figure 33c).

### Example XXXIII

### Synergy Between Endogenous IL-10 and Peripheral Tolerance for In Vivo Modulation of Aggressive T cells.

Systemic antigen given to animals without adjuvant usually drives tolerance operating through antigen presentation by peripheral APCs expressing minimal or no costimulatory molecules. Incubation of purified macrophages or dendritic cells with sol or agg Ig-PLP1, which allows for efficient loading of peptide onto MHC class II molecules, does not lead to up-regulation of B7-1, B7-2 or CD40 (data not shown). Furthermore, since agg Ig-PLP1 causes the production of IL-10 by APCs (Figure 28), it is likely that IL-10 inhibits up-regulation of costimulatory molecules on APCs.

Since IL-10 has been shown to antagonize Th1 cytokines (Fiorentino et al., *J. Immunol.,* 146:3444-51, 1991 and possibly interfere with inflammatory functions, the effectiveness of agg Ig-PLP1 in T cell modulation and reversal of disease via inadequate peptide presentation by APCs expressing minimal costimulatory molecules and the inhibitory function of IL-10 produced by such APCs were investigated as follows.

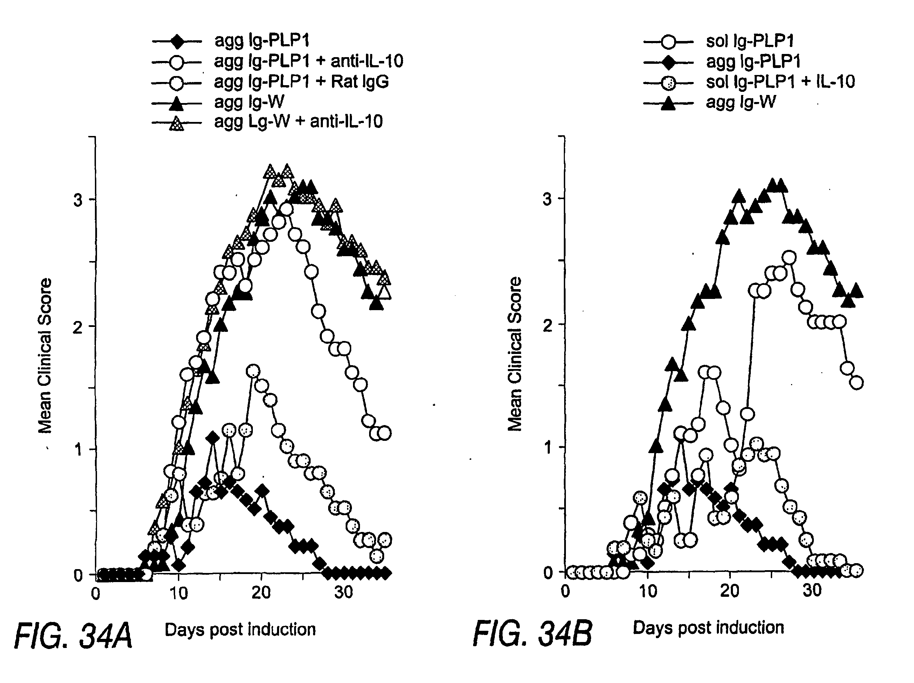

Mice were induced for EAE with PLP1 peptide and when the signs of paralysis became apparent the mice were given agg Ig-PLP1 together with anti-IL-10 antibody and assessed for reduction in disease severity as follows. SJL/J mice (8 per group) were induced for EAE with 100µg PLP1, and on days 9,13 and 17 were given i.p. in PBS 300µg agg lg-PLP1 (agg Ig-PLP1); 300µg agg lg-PLP1 + 500µg Rat anti-mouse IL-10 antibody, 2A5 (agg Ig-PLP1 + anti-IL-10); 300µg agg lg-PLP1 + 500 µg Rat IgG (agg Ig-PLP1 + Rat IgG); 300µg agg Ig-W (agg Ig-W); or 300 g agg lg-W + 500 g Rat anti-mouse IL-10 antibody, 2A5 (agg lg-W + anti-IL-10). All the injections were done i.p. in PBS. The results are shown in Figure 34a.

As shown in Figure 34a, the severity of paralysis was restored when in vivo IL-10 was neutralized by the anti-IL-10 antibody. In fact, mice treated with agg Ig.PLP1 alone had a mean maximal clinical score of 1.1 ± 0.5 while the mice injected with both agg Ig-PLP1 and anti-IL-10 antibody had a score of 3.0 ± 0.3 which is comparable to the 3.3 ± 0.3 (p > 0.23) score seen in mice treated with agg Ig-W. Furthermore, control mice given agg Ig-PLP1 together with rat IgG, instead of anti-IL-10 antibody, did not restore disease severity and had a mean maximal score of 1.6 ± 0.2. Injection of anti-IL-10 antibody together with agg Ig.W neither reduced nor exacerbated the severity of disease. These results demonstrate that agg Ig-PLP1 induced IL-10 plays a significant role in controlling disease severity and that for the effects of IL-10 to occur a specific interaction between APCs and the target T cells is required.

Further support for this mechanism comes from the fact that treatment with sol Ig-PLP1 plus exogenous IL-10 reduces the severity of paralysis to the same extent as agg Ig-PLP1. Groups of mice (8 per group) were induced for EAE with 100µg PLP1 and on days 9, 13, and 17 were given i.p. in PBS 300µg sol lg-PLP1 (sol Ig-PLP1); 300µg agg Ig.PLP1 (agg Ig-PLP1); 300µg sol Ig-PLP1 + 400 U rlL10 (sol Ig-PLP1 + IL-10); or 300µg agg lg-W (agg Ig-W). As shown in Figure 34b, soluble Ig-PLP1, which does not induce detectable levels of IL-10, ameliorates the disease As shown in Figure 34b, soluble Ig-PLP1, which does not induce detectable levels of IL-10, ameliorates the disease slightly with a mean maximal score of 2.5 ± 0.3 while sol Ig-PLP1, together with exogenous IL-10, further reduces the disease to a mean maximal clinical score of 1.1 ± 0.3 which is comparable to the 1.1 ± 0.5 score obtained with mice treated with agg Ig-PLP1.

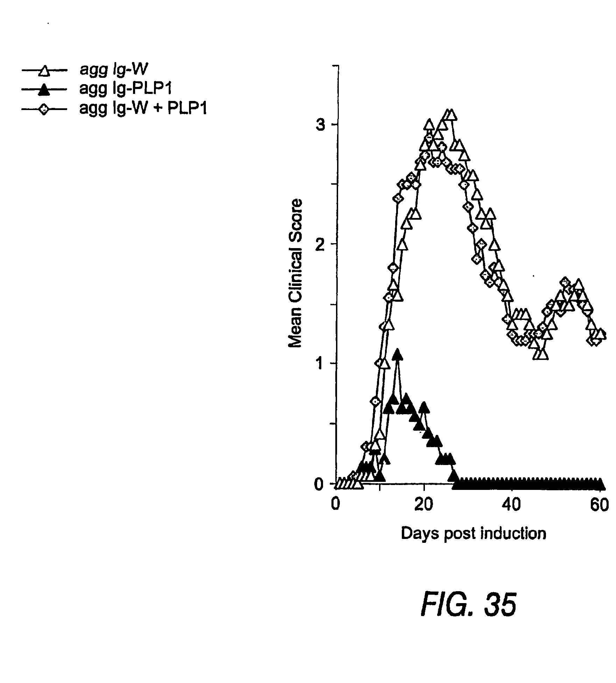

For endogenous 1L-10 to modulate the disease, a physical bridging of the APCs to the T cells seems to be required. To confirm this mechanism, the following experiments were performed. Groups of mice (8 per group) were induced for EAE with 100µg PLP1 and then treated with 300µg of agg lg-PLP1 (agg Ig.PLP1), 3000g agg Ig-W (agg lg-W), or 3000g agg lg-W + 1000g PLP1 (agg Ig-W + PLP1) in PBS on days 9,13, and 17 post disease induction. The results are shown in Figure 35.

As shown in Figure 35, onset of disease was at day 7 in these experimental groups. Treatment of diseased mice with a mixture of agg Ig-W and free PLP1 peptide, instead of agg Ig-PLP1, did not reduce the severity of disease. Overall, effective T cell down-regulation requires physical interaction between IL-10 producing APCs, and the target pathogenic T cell. The likely explanation for this requirement is that IL-10 as a paracrine cytokine needs to be in close proximity to T cells in order to achieve antagonism.

### Example XXXIV

### Agg Ig-PLP1 Provides Expeditious Amelioration of Autoimmune Disease

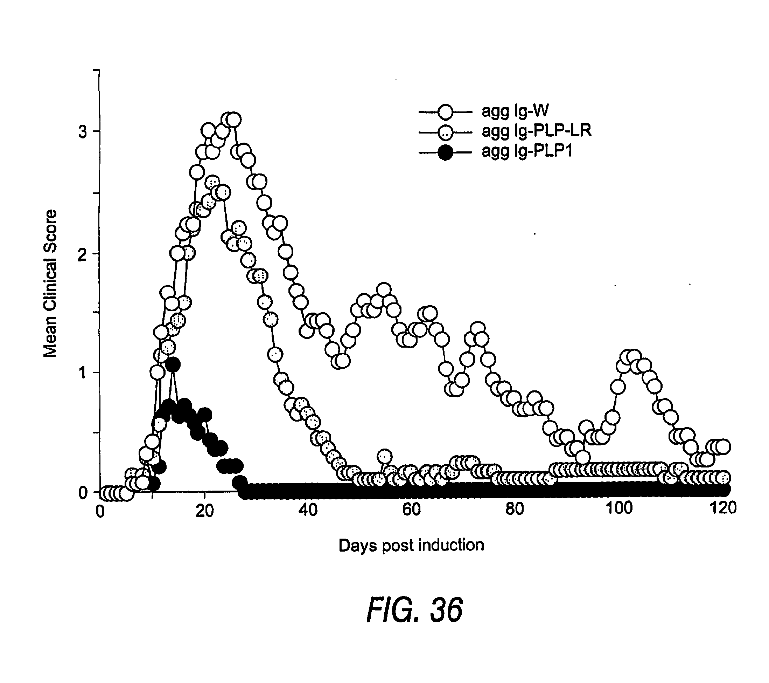

Although both agg Ig-PLP-LR and agg Ig-PLP1 ameliorate the symptoms of autoimmune disease, faster relief is provided by agg Ig-PLP1. Groups of SJL mice were induced for disease with PLP1 peptide and treated i.p. with 300 0g of agg Ig-PLP1, agg Ig-PLP-LR, or agg Ig-W in 3000l PBS on days 9,13, and 17. The results are shown in Figure 36 and Table 5.

As can be seen in Figure 36 and Table 5 treatment with agg Ig-PLP1 dramatically reduced the severity of disease. Although both agg Ig-PLP-LR and agg Ig-PLP1 treatment resulted in recovery from EAE, mice which received agg-ig-PLP1 recovered more quickly than mice which received agg Ig-PLP-LR. The mean of maximal clinical score was reduced from 3.3 ± 0.3 for the agg Ig-W treated group to 1.1± 0.5 in the agg Ig-PLP1 group (see Table 5). Moreover, full recovery from disease after treatment with agg Ig-PLP1 was expeditious (day 24.4 ± 2.2) and relapses did not occur during the 120 day period of clinical assessment.

It is worth noting that agg Ig-PLP1 is more effective in disease modulation than sol Ig-PLP1. While agg Ig-PLP1 reduced the maximal clinical score to 1.1± 0.5, the soluble form of Ig-PLP1 only diminished the severity of paralysis to 2.4 ± 0.3 (compare Table 3 and Table 5). In addition, the recovery was much faster for the group treated with agg Ig-PLP1 than for the mice given sol Ig-PLP1 (see Table 3 and Table 5).

**Table 5. Characteristics of clinical disease following treatment with agg Ig-chimeras**

| Treatment | Incidence | Day of Onset' | Mean Maximum Disease Severity^{**} | Day of Recovery^{***} |
|---|---|---|---|---|
| agg lg-W | 10/10 | 8.9± 1.5 | 3.3± 0.3 | > 120 |
| agg Ig-PLP-LR | 7/7 | 8.0± 1.7 | 2.9± 0.3 | 41.3± 6.2 |
| agg Ig-PLP1 | 10/10 | 9.1± 2.3 | 1.1± 0.5 | 24.4± 2.2 |

| | | | | |
|---|---|---|---|---|
| ^{*} Mean ± SC of the day of disease onset ^{**} Mean ± SD of the maximal clinical scores ^{***} Mice were considered recovered when their clinical score was < 0.5 for at least 5 days | | | | |

Histopathological analyses were also performed. Groups of mice induced for EAE and treated with agg lg-chimeras as in Figure 36 were sacrificed at day 28 post disease induction, and the brain and spinal cord were removed, fixed in formalin, and embedded in paraffin. Serial cross sections, 60m thick, from the cerebrum and lumbar cord were cut and stained with hematoxylin-eosin (H&E). Perivascular clusters containing at least 20 mononuclear cells were counted as an inflammatory focus.

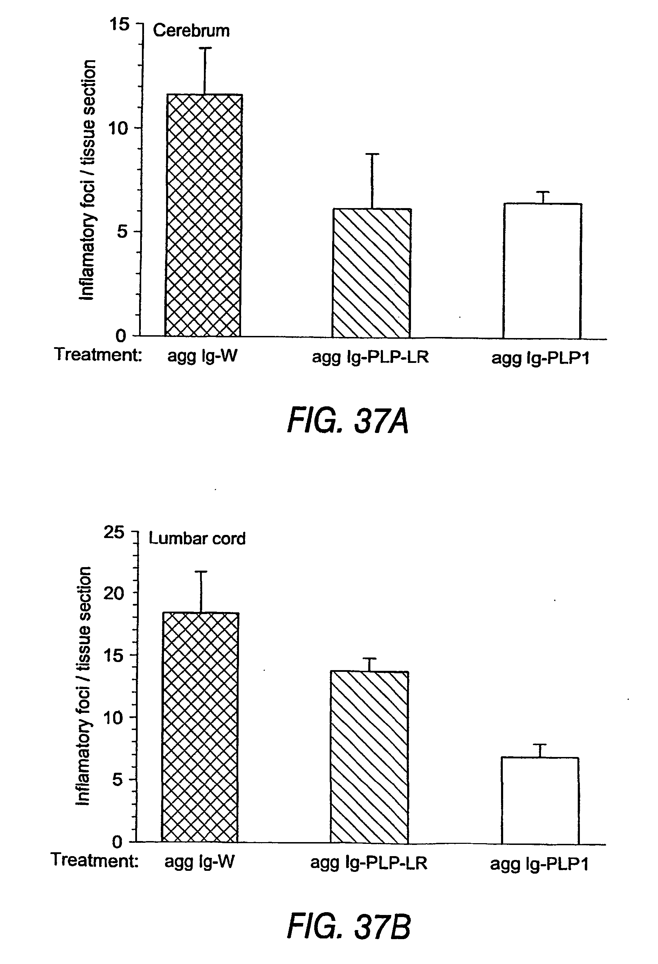

The results are shown in Figure 37. As shown in Figure 37, mice treated with agg Ig-PLP1 had a significantly reduced number of inflammatory foci both in the cerebrum and lumbar spinal cord.

It is worth noting that the efficacy of agg Ig-PLP1 in amelioration of EAE occurs with a much lower dose (300 µg/injection) than soluble Ig-PLP1, which was given at 5000g per injection. This is most likely due to the in vivo production of IL-10 upon treatment with the agg but not the soluble form of Ig-PLP1.

While not wishing to be bound by any particular theory the following mechanisms may explain the faster results obtained with agg lg-PLP1 relative to agg Ig-PLP-LR. Because PLP-LR is a T cell antagonist peptide created by altering PLP1, the affinity of the interaction between T cells presenting this altered peptide and APCs would be expected to be lower than the affinity between T cells presenting unaltered PLP1 and APCs. As a result of this lower affinity, T cells would not interact with APCs presenting PLP-LR for as long as they interact with APCs presenting PLP1, thereby reducing their period of exposure to IL-10 induced by the aggregated immunoglobulins.

Alternatively, the faster results obtained with agg lg-PLP1 may be a consequence of the diversity of the autoreactive T cell repertoire. If the frequency of T cells reactive with PLP1 is greater than the frequency of T cells reactive with PLP-LR, a differential disease modulation by the two chimeras may occur that fits the pattern observed. In this case in the absence of IL-10 the soluble chimeras would affect a common population of T cells but the aggregated forms would favor Ig-PLP1, as high affinity T cells would be subject to effective IL-10 exposure.

### Example XXXV

### IL-10 Produced in Response to Agg Ig-PLP1 Down Regulates Costimulatory Molecules

IL-10 has been reported to down-regulate the expression of costimulatory molecules on APCs. To determine whether IL-10 produced in response to agg Ig-PLP1 down regulates costimulatory molecules on APCs, the following experiment was performed.

Peritoneal macrophages were incubated with agg Ig-PLP chimeras for 24 hours and then assessed for cell surface expression of costimulatory molecules. Macrophages were harvested from peritoneal cells of mice injected with thioglycolate broth as described above. Purified macrophages (1.0 x 10⁸ cells/ml) were subsequently incubated with 0.3 OM agg lg-PLP chimera (black line) or media alone (NIL, grey). After 24 hours the cells were harvested and stained with anti-F4/80, and either anti-B7.1, anti-B7.2, or anti-CD40. Histograms represent F480⁺ gated cells and show the intensity of either B7.1, B7.2, or CD40.

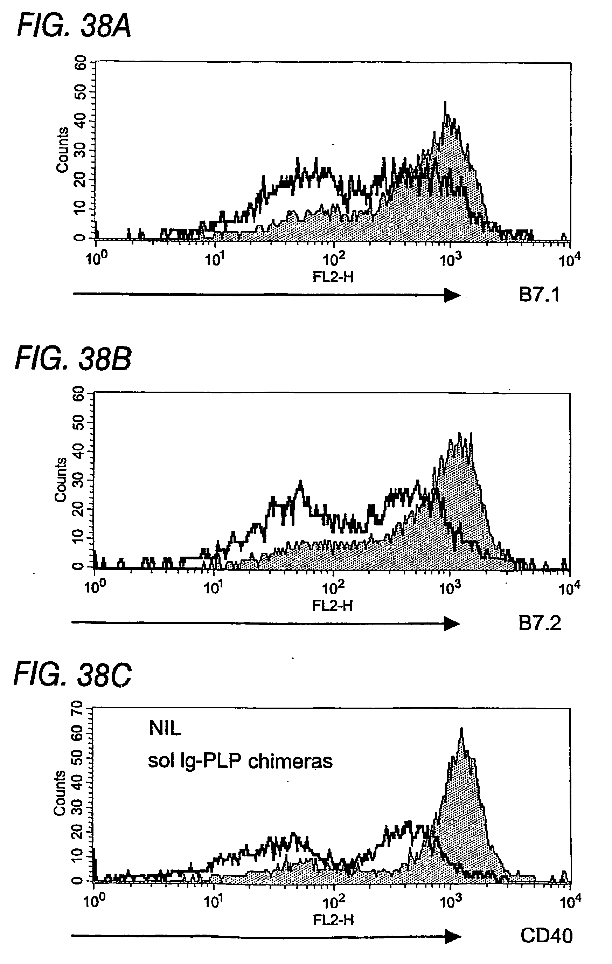

The results are shown in Figure 38. As shown in Figure 38, there was no up-regulation in B7.1, B7.2, or CD40 expression. To the contrary there was a significant down-regulation of these molecules relative to basal level seen in cultures in the absence of agg Ig-PLP chimeras. Thus, IL-10 produced in response to agg Ig-PLP1 down regulates costimulatory molecules.

### Example XXXVI

### Treatment with Aggregated Ig-PLP1 Decreases the Clinical Severity of Active EAE Induced by Multiple Epitopes

IL-10 produced by APCs as a result of agg Ig-PLP1-mediated FcR crosslinking may antagonize specific T cells engaged to the PLP1-MHC ligand on the APCs as well as neighboring T cells with unrelated specificity. This phenomenon known as bystander suppression has proven effective in IL-4 and IL-10 settings.

To determine whether bystander suppression results from IL-10 produced in response to aggregated immunoglobulins comprising an antigen involved in autoimmune disease, EAE was induced with a mixture of epitopes and the ability of agg Ig-PLP1 to modulate unrelated autoreactive T cells and ameliorate the disease was measured. Groups of SJL/J mice (8 per group) were induced for EAE with a mixture of 100µg PLP1 and 100µg PLP2 and on days 9, 13, and 17 treated with 300µg agg Ig-PLP1 or agg Ig-W per injection. All treatments were i.p. in PBS. The onset of disease was at day 7 in these experimental groups. Each point represents the mean clinical score of 8 mice.

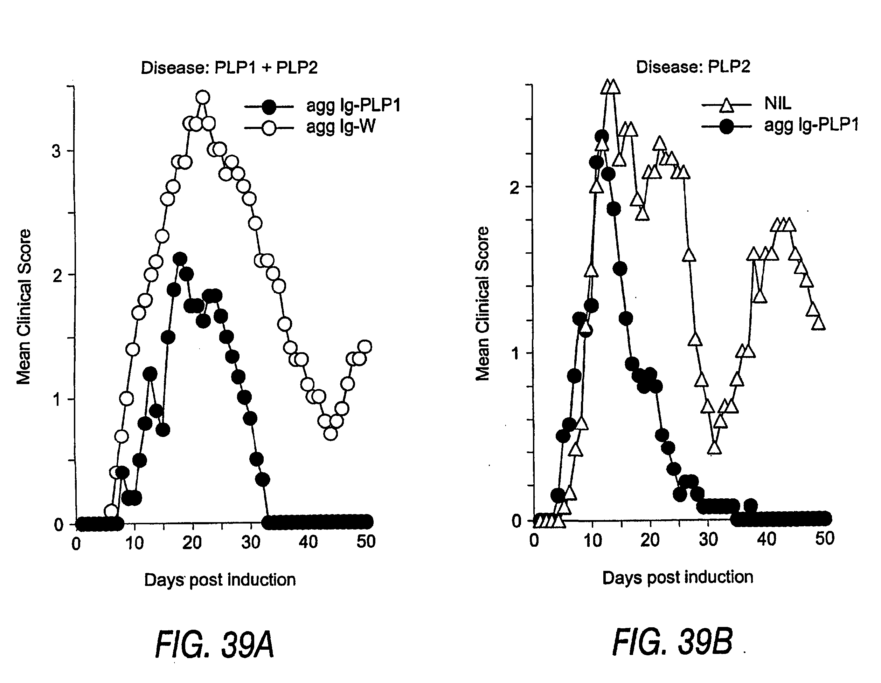

The results are shown in Figure 39a. As shown in Figure 39a, mice with ongoing EAE induced by a mixture of PLP1 and PLP2 peptides manifested reduced severity of paralysis and fully recovered by day 33 post disease induction after treatment with agg lg-PLP1 while animals treated with agg Ig-W had severe paralysis and did not recover from the disease during the 50 day period of clinical assessment. Therefore, endogenous IL-10 appears to have down-regulated effects on PLP2-specific T cells.

Induction of disease with PLP2 peptide should expose whole PLP and drive spreading and activation of PLP1-specific T cells (McRae et al., *J*. *Exp. Med.,* 182:75-85, 1998; Tuohy et al., *Immunol. Rev.,* 164:93-100, 1998. In this case, injection of agg Ig-PLP1 should bridge IL-10 producing APCs to PLP1-specific T cells and promote bystander suppression of these cells as well as neighboring PLP2-specific T cells. To determine whether administering agg lg-PLP1 to mice in which EAE was induced with PLP2 provides bystander suppression of PLP2 specific T cells, the following experiment was performed.

Mice were induced for EAE with PLP2 peptide and when signs of paralysis became apparent they were treated with agg Ig-PLP1 as follows. Groups of SJL/J mice (8 per group) were induced for EAE with 100µg PLP2 and on days 9, 13, and 17 treated i.p. with 300µg agg Ig-PLP1 per injection. A group of untreated mice (NIL) was included for comparison purposes. The results are shown in Figure 39b.

As shown in Figure 39b, although the initial phase of paralysis in mice treated with agg Ig-PLP1 is only slightly milder than untreated mice, the animals quickly recovered by day 26 and, unlike the untreated mice, did not relapse for the remaining period of clinical assessment. These results support bystander suppression and demonstrate that epitope spreading offers an opportunity to modulate disease at a later stage of paralysis.

To further explore the ability of agg lg-PLP1 to provide bystander suppression of T cells specific for antigens other than PLP1, the following experiment was performed. The ability of agg lg-PLP1 to modulate disease induced with CNS homogenate, which incorporates a full range of myelin autoantigens, was measured. CNS homogenate was prepared as follows. Fifty frozen unstripped rat brains (Pelfreez Biologicals, Rodgers, AK) were homogenized in PBS using a Waring blender and adjusted to 300 mg/ml with PBS. CNS homogenate was stored at -20°C. Groups of SJL/J mice (9 per group) were induced for EAE with 6 mg of CNS homogenate and on days 9,13, and 17 treated i.p. with 300µg agg Ig-PLP1 or agg Ig-W per injection. A group of untreated mice (NIL) was included for comparison purposes. The results are shown in Figure 35.

As shown in Figure 35, mice injected with agg Ig-PLP1 had mild signs of paralysis in the initial phase of paralysis and fully recovered by day 24 post disease induction without any relapses for the 60 day period of clinical assessment. Control mice treated with agg Ig-W, instead of agg Ig-PLP1, had a disease pattern similar to that of untreated animals. These results indicate that the down-regulatory function of agg Ig-PLP1 extends both to intra- and intermolecular epitopes and suppresses diverse T cell specificities.

### Example XXXVII

### Agg Ig-PLP1 Provides Bystander Suppression by Inducing APCs to Produce IL-10

Exposure to IL-10 seems to be the likely mechanism underlying down-regulation and suppression of pathogenic myelin-specific T cells. The source of IL-10, as demonstrated in Figures 28 and 34, is APCs such as dendritic cells and macrophages. However, the broadened effectiveness and the endurance of T cell modulation in this setting raised the question of whether the bystander suppression was due to antagonism of the pathogenic T cells by APCs' IL-10 or to down-regulation by regulatory T cells generated under the effect of such IL-10.

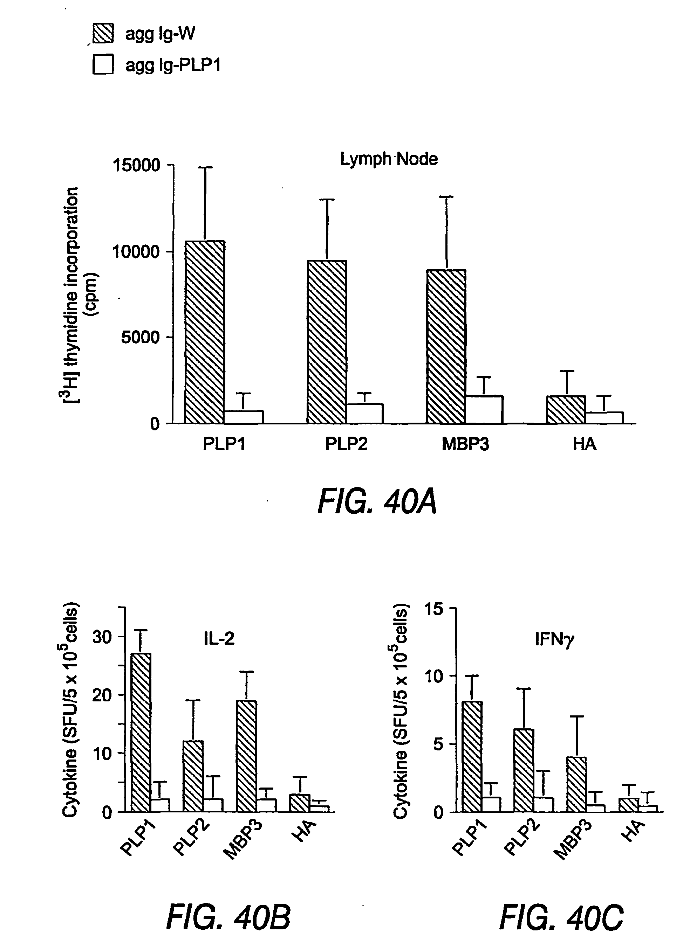

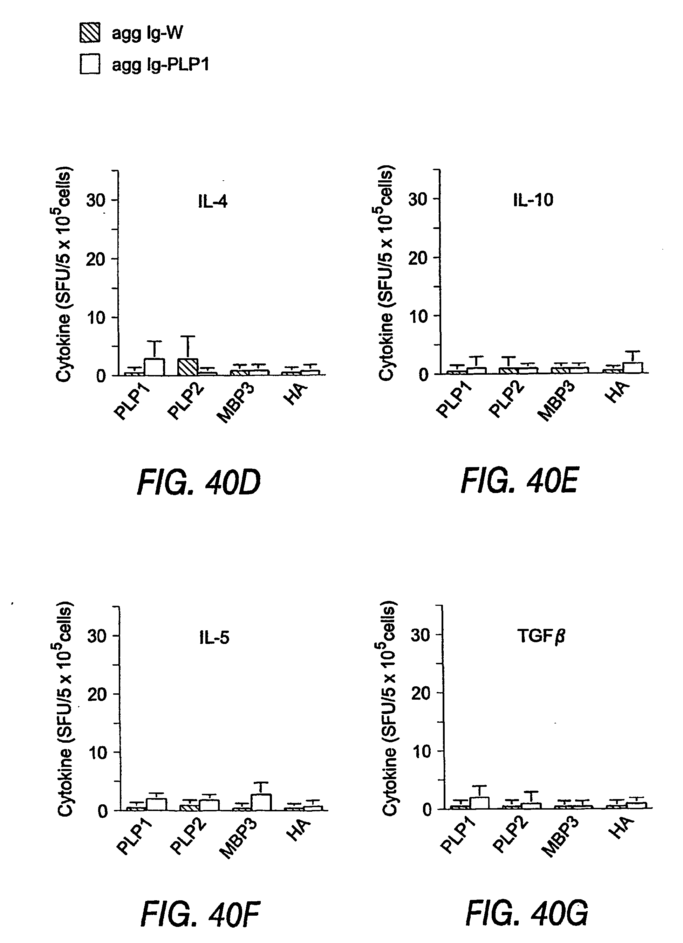

To determine whether agg lg-PLP1 acts via a suppression of T cell proliferation or through regulatory T cells the following experiments were performed. Lymph node T cells from mice, which were recovering from CNS-induced paralysis subsequent to treatment with agg Ig-PLP1, were stimulated with antigen and tested for proliferation and production of cytokines (markers of regulatory T cells). Mice (6 per group) were induced for EAE with CNS homogenate and then treated with agg Ig-W (hatched bars) or agg lg-PLP1 (closed bars) on days 9, 13, and 17 as described above. Two days after completion of the treatment regimen, the lymph nodes (axillary, lateral axillary, and popliteal) were harvested, and the cells (4 x 10⁵ cellsl10ool/well) were stimulated with 1000l/well of antigen (PLP1, PLP2, MBP3. or HA (control)). Cell proliferation was assessed three days later using [³H]thymidine incorporation assay (Figure 40a). In addition, cytokine responses were analyzed after 24 h of incubation with antigen by ELISPOT using 5 x 10⁵ cells per well (Figure 40b.g). ELISPOT assays were used to measure the cytokines produced by lymph node T cells upon stimulation with antigen as described in Min et al., *J. Exp. Med.,* 188:2007-2017, 1998. Briefly, lymph node cells (5 x 10⁵ cells/100*µ*l/well) and the antigen (100 *µ*l/well) were incubated in HA-multiscreen plates (Millipore, Bedford, MA) coated with capture antibody for 24 hours. Bound cytokines were revealed with peroxidase and anti-cytokine antibodies. The anti-cytokine antibody pairs used were those described for the ELISA technique. Spots were counted under a dissecting microscope.

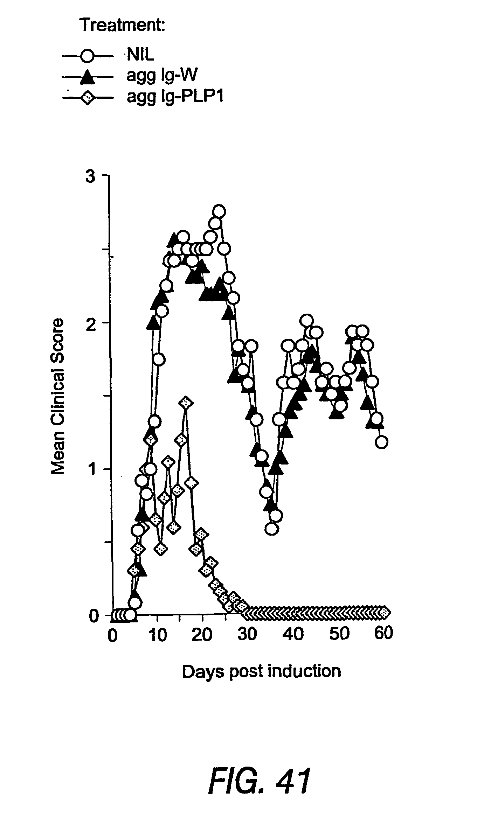

The antigens were used at the defined optimal concentrations of 150g/ml for PLP1, PLP2, and HA and 300g/ml for MBP3. Control wells of media without addition of antigen were included and used as background. Each bar represents the mean ± standard deviation of 6 individually tested mice. The results presented in Figure 41 show that 2 days after the final injection of agg Ig chimeras proliferation to myelin peptides was significant in the mice treated with the control Ig-W but at background levels for those recipient of agg Ig-PLP1. Similarly, while the mice injected with agg Ig-W had significant amounts of IL-2 and IFNγ, those treated with agg Ig-PLP1 had neither Th1 nor Th2 type cytokines and did not produce IL-10, IL-5, or TGFβ. Similar results were obtained when the mice were tested at day 9 after completion of the treatment regimen (data not shown). Furthermore, splenic T cells and cells harvested from the peritoneum showed a similar pattern of responses (data not shown). Overall, these results suggest that the typical proliferative and cytokine responses trademark of regulatory T cells are undetectable in this particular setting of systemic treatment of active autoimmunity. Thus, the bystander suppression resulting from administration of agg Ig-PLP1 was due to antagonism of the pathogenic T cells by IL-10 produced by APCs.

Those skilled in the art will further appreciate that the present invention may be embodied in other specific forms without departing from the spirit or central attributes thereof. In that the foregoing description of the present invention discloses only exemplary embodiments thereof, it is to be understood that other variations are contemplated as being within the scope of the present invention. Accordingly, the present invention is not limited to the particular embodiments which have been described in detail herein. Rather, reference should be made to the appended claims as indicative of the scope and content of the invention.

## Claims

1. A composition comprising an aggregated immunoglobulin or portion thereof linked to an antigen associated with an autoimmune disease, wherein said immunoglobulin or portion thereof is capable of crosslinking Fc receptors present on the cell surfaces of antigen presenting cells.

2. The composition of Claim 1, further comprising a pharmaceutically acceptable carrier.

3. The composition of Claim 2, wherein said composition does not include an adjuvant.

4. The composition of Claim 1, wherein said immunoglobulin is in a polyvalent form.

5. The composition of Claim 1, wherein said immunoglobulin is embedded or absorbed on a matrix.

6. The composition of Claim 1, wherein said immunoglobulin is an IgG molecule.

7. The composition of Claim 1, wherein said antigen is associated with an autoimmune disease selected from the group consisting of multiple sclerosis, lupus, rheumatoid arthritis, scleroderma, insulin-dependent diabetes and ulcerative colitis.

8. The composition of Claim 1 wherein said antigen is an antigen from proteolipid protein.

9. The composition of Claim 1 wherein said antigen is an antigen from myelin basic protein.

10. The composition of Claim 1 wherein said immunoglobulin or portion thereof comprises at least part of a domain of a constant region of an immunoglobulin molecule.

11. The composition of Claim 1 which comprises a fusion protein in which said antigen is covalently joined to said immunoglobulin or portion thereof.

12. The composition of Claim 11 wherein said antigen is positioned within at least one complementarity determining region of said immunoglobulin to partially or fully replace said complementarity determining region.

13. The composition of Claim 12 wherein the antigen is positioned within CDR3.

14. The composition of Claim 1, wherein said immunoglobulin is a human IgG molecule.

15. The composition of Claim 1, wherein said immunoglobulin is chimeric.

16. The composition of claim 1 wherein the composition results in enhanced IL-10 production by antigen presenting cells, thereby providing bystander suppression of autoreactive T cells.

17. The composition of claim 1 wherein the composition results in enhanced IL-10 production of antigen presenting cells thereby antagonizing production of IFNγ by T cells.

18. The composition of claim 1 wherein the composition has the property of being endocytosed by cells bearing said Fc receptors and processed by the antigen presenting cells to present said antigen in association with endogenous MHC Class II molecules, thereby preventing activation of autoreactive T cells in vivo.

## Patentansprüche

1. Zusammensetzung, umfassend ein aggregiertes lmmunglobulin oder einen Teil davon, gebunden an ein Antigen, das mit einer Autoimmunkrankheit assoziiert ist, worin das lmmunglobulin oder der Teil davon in der Lage ist, Fc-Rezeptoren, die an den Zelloberflächen von Antigen-präsentierenden Zellen vorhanden sind, zu vernetzen.

2. Zusammensetzung nach Anspruch 1, weiters umfassend einen pharmazeutisch annehmbaren Träger.

3. Zusammensetzung nach Anspruch 2, worin die Zusammensetzung kein Adjuvans umfasst.

4. Zusammensetzung nach Anspruch 1, worin das Immunglobulin in einer mehrwertigen Form vorliegt.

5. Zusammensetzung nach Anspruch 1, worin das Immunglobulin in eine Matrix eingebettet oder von ihr absorbiert ist.

6. Zusammensetzung nach Anspruch 1, worin das Immunglobulin ein IgG-Molekül ist.

7. Zusammensetzung nach Anspruch 1, worin das Antigen mit einer Autoimmunkrankheit, ausgewählt aus der aus multipler Sklerose, Lupus, rheumatoider Arthritis, Sclerodermia, Insulin-abhängigem Diabetes und Colitis ulcerosa bestehenden Gruppe, assoziiert ist.

8. Zusammensetzung nach Anspruch 1, worin das Antigen ein Antigen aus Proteolipidprotein ist.

9. Zusammensetzung nach Anspruch 1, worin das Antigen ein Antigen aus basischem Myelinprotein ist.

10. Zusammensetzung nach Anspruch 1, worin das Immunglobulin oder der Teil davon zumindest einen Teil einer Domäne einer konstanten Region eines Immunglobulinmoleküls umfasst.

11. Zusammensetzung nach Anspruch 1, umfassend ein Fusionsprotein, in dem das Antigen kovalent an das Immunglobulin oder den Teil davon gebunden ist.

12. Zusammensetzung nach Anspruch 11, worin das Antigen innerhalb von zumindest einer komplementaritätsbestimmenden Region des Immunglobulins angeordnet ist, um die komplementaritätsbestimmende Region teilweise oder vollständig zu ersetzen.

13. Zusammensetzung nach Anspruch 12, worin das Antigen innerhalb von CDR3 angeordnet ist.

14. Zusammensetzung nach Anspruch 1, worin das Immunglobulin ein menschliches IgG-Molekül ist.

15. Zusammensetzung nach Anspruch 1, worin das Immunglobulin chimär ist.

16. Zusammensetzung nach Anspruch 1, worin die Zusammensetzung zu gesteigerter IL-10-Produktion durch Antigen-präsentierende Zellen führt und **dadurch** eine "Bystander"-Suppression von autoreaktiven T-Zellen bereitstellt.

17. Zusammensetzung nach Anspruch 1, worin die Zusammensetzung zu gesteigerter IL-10-Produktion durch Antigen-präsentierende Zellen führt und **dadurch** die Produktion von IFNγ durch T-Zellen antagonisiert.

18. Zusammensetzung nach Anspruch 1, worin die Zusammensetzung die Eigenschaft aufweist, durch Zellen, die Fc-Rezeptoren tragen, Endocytose zu erfahren und durch die Antigen-präsentierenden Zellen verarbeitet zu werden, um so das Antigen in Assoziation mit endogenen MHC-Klasse-II-Molekülen zu präsentieren, wodurch die Aktivierung von autoreaktiven T-Zellen in vivo unterbunden wird.

## Revendications

1. Composition comprenant une immunoglobuline agrégée ou une portion de celle -ci enchaînée à un antigène associé à une maladie auto -immune, où ladite immunoglobuline ou une portion de celle -ci est capable de réticulation des récepteurs Fc présents sur les surfaces des cellules, des cellules présentant l'antigène.

2. Composition de la revendication 1 , comprenant de plus un support pharmaceutiquement acceptable.

3. Composition de la revendication 2 , où ladite proposition ne contient pas d'adjuvent.

4. Composition de la revendication 1 , où ladite immunoglobuline est sous une forme polyvalente.

5. Composition de la revendication 1 , où ladite immunoglobuline est noyée ou absorbée sur une matrice.

6. Composition de la revendication 1 , où ladite immunoglobuline est une molécule de IgG.

7. Composition de la revendication 1 où ledit antigène est associé à une maladie auto-immune sélectionnée dans le groupe consistant en sclérose en plaques, lupus, arthrite rhumatoïde, sclérodermie, diabète dépendant de l'insuline et colite ulcérative.

8. Composition de la revend ication 1, où ledit antigène est un antigène d'une protéine protéolipidique.

9. Composition de la revendication 1, ou ledit antigène est un antigène d'une protéine basique de myéline.

10. Composition de la revendication 1 où ladite immunoglobuline ou sa portion comprend au moins une partie d'un domaine d'une région constante d'une molécule d'immunoglobuline

11. Composition de la revendication 1 qui comprend une protéine de fusion dans laquelle ledit antigène est joint de manière covalente à ladite immunoglobuline ou une portion de celle-ci.

12. Composition de la revendication 11, où ledit antigène est placé dans au moins une région déterminant la complémentarité de ladite immunoglobuline pour remplacer partiellement ou totalement ladite région déterminant la complémentarité.

13. Composition de la revendication 12 où ledit antigène est placé dans CDR3.

14. Composition de la revendication 1 ou ladite immunoglobuline est une molécule de IgG humaine.

15. Composition de la revendication 1 où ladite immunoglobuline est chimérique.

16. Composition de la revendication 1 où la composition a pour résultat la production améliorée de IL-10 par des cellules présentant l'antigène, pour ainsi produire une suppression accessoire de cellules T auto-réactives.

17. Composition de la revendication 1 où la composition a pour résultat une production améliorée de IL-10 des cellules présentant l'antigène pour ainsi antagoniser la production de l'IFNγ par les cellules T.

18. Composition de la revendication 1 où la composition a la propriété d'être endocytosée par des cellules portant lesdits récepteurs Fc et traitée par les cellules présentant l'antigène pour présenter ledit antigène en association avec des molécules MHC classe II endogènes, pour ainsi empêcher l'activation des cellules T auto-réactives in vivo.
